(19) **Europäisches Patentamt** **European Patent Office** **Office européen des brevets**

(11) **EP 3 950 958 B1**

(12) **EUROPEAN PATENT SPECIFICATION**

(45) Date of publication and mention of the grant of the patent:
**04.10.2023 Bulletin 2023/40**

(21) Application number: **21175553.3**

(22) Date of filing: **12.08.2019**

(51) International Patent Classification (IPC):
**C12Q 1/6806** (2018.01)

(52) Cooperative Patent Classification (CPC):
(C-Sets available)
**C12Q 1/6806** (Cont.)

(54) **SEQUENCING ALGORITHM**

**SEQUENZIERUNGSALGORITHMUS**

**ALGORITHME DE SÉQUENÇAGE**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.08.2018 GB 201813171**
**20.05.2019 GB 201907101**

(43) Date of publication of application:
**09.02.2022 Bulletin 2022/06**

(62) Document number(s) of the earlier application(s) in accordance with Art. 76 EPC:
**19755421.5 / 3 710 597**

(73) Proprietor: **Illumina Singapore Pte. Ltd.**
**Singapore 757716 (SG)**

(72) Inventors:
 • **IMELFORT, Michael**
  **Sydney, New South Wales 2000 (AU)**
 • **MONAHAN, Leigh G**
  **Sydney, New South Wales 2000 (AU)**
 • **TO, Joyce**
  **Sydney, New South Wales 2000 (AU)**
 • **BURKE, Catherine M**
  **Sydney, New South Wales 2000 (AU)**
 • **DARLING, Aaron E**
  **Sydney, New South Wales 2000 (AU)**

(74) Representative: **Marks & Clerk LLP**
**15 Fetter Lane**
**London EC4A 1BW (GB)**

(56) References cited:
**WO-A1-02/079502**

 • **Botond Sipos ET AL: "An Improved Protocol for Sequencing of Repetitive Genomic Regions and Structural Variations Using Mutagenesis and Next Generation Sequencing", PLoS ONE, vol. 7, no. 8, 17 August 2012 (2012-08-17) , page e43359, XP055531087, DOI: 10.1371/journal.pone.0043359**
 • **D. Levy ET AL: "Facilitated sequence counting and assembly by template mutagenesis", Proceedings of the National Academy of Sciences, vol. 111, no. 43, 13 October 2014 (2014-10-13), pages E4632-E4637, XP055531168, ISSN: 0027-8424, DOI: 10.1073/pnas.1416204111**

Remarks:
The complete document including Reference Table(s) and the Sequence Listing(s) can be downloaded from the EPO website

(52) Cooperative Patent Classification (CPC): (Cont.)

C-Sets
**C12Q 1/6806, C12Q 2521/101, C12Q 2525/117,
C12Q 2527/146, C12Q 2531/113, C12Q 2535/122**

**Description**

**Field of the Invention**

The invention relates to a method for determining a sequence of at least one target template nucleic acid molecule using non-mutated sequence reads and mutated sequence reads. The invention also relates to a method for determining a sequence of at least one target template nucleic acid molecule in a sample involving controlling or normalising the number of target template nucleic acid molecules in the sample. The invention also relates to a computer programme adapted to perform the method, a computer readable medium comprising the computer programme, and computer implemented methods.

**Background of the Invention**

The ability to sequence nucleic acid molecules is a tool that is very useful in a myriad of different applications. However, it can be difficult to determine accurate sequences for nucleic acid molecules that comprise problematic structures, such as nucleic acid molecules that comprise repeat regions. It can also be difficult to resolve structural variants, such as the haplotype structure of diploid and polyploid organisms.

Many of the more modern techniques (so-called next generation sequencing techniques) are only able to sequence short nucleic acid molecules accurately. The next generation sequencing techniques can be used to sequence longer nucleic acid sequences, but this is often difficult. Next generation sequencing techniques can be used to generate short sequence reads, corresponding to sequences of portions of the nucleic acid molecule, and the full sequence can be assembled from the short sequence reads. Where the nucleic acid molecule comprises repeat regions, it may be unclear to the user whether two sequence reads having similar sequences correspond to sequences of two repeats within a longer sequence, or two replicates of the same sequence. Similarly, the user may want to sequence two similar nucleic acid molecules simultaneously, and it may be difficult to determine whether two sequence reads having similar sequences correspond to sequences of the same original nucleic acid molecule or of two different original nucleic acid molecules. Assembling sequences from short sequence reads can be aided using sequencing aided by mutagenesis (SAM) techniques. In general SAM involves introducing mutations into target template nucleic acid sequences. The mutation patterns that are introduced may assist the user of the method in assembling the sequences of nucleic acid molecules from short sequence reads.

For example, where the template nucleic acid molecules contain repeat regions, the repeats may be distinguished from one another by different mutation patterns, thereby enabling the repeat regions to be resolved and assembled correctly.

WO 02/079502 is directed to the use of secondary sequences that vary from a primary sequence for inferring information about the primary sequence. WO 2014/013218 describes systems and methods of characterising haplotypes in a nucleic acid sample by introducing polymorphisms into fragments of a nucleic acid and sequencing the resulting mutated nucleic acid molecules and assembling a sequence for mutated nucleic acid molecules. WO 2016/057947 describes the introduction of random nucleotide mutations into a target nucleic acid molecule and assembling overlapping sequence reads from mutated nucleic acid molecules.

In general, SAM techniques involve mutating copies of a target template nucleic acid molecule, and then assembling sequences for the mutated copies based on their mutation patterns. The user may then create a consensus sequence from the sequences of the mutated copies. Since the different mutated copies will comprise mutations at different positions, the consensus sequence may be representative of the original template nucleic acid molecule. However, the consensus sequence may comprise artefacts from the mutation process. Furthermore, creating the consensus sequence involves using computer programs that are complicated and processing-intensive.

Accordingly, there remains a need for methods for determining a sequence of at least one target template nucleic acid molecule in which the sequence reads may be assembled, accurately, quickly and efficiently.

**Summary of the Invention**

The present inventors have developed new improved methods for determining a sequence of at least one target template nucleic acid molecule. Thus, in a first aspect of the invention, there is provided a method for determining a sequence of at least one target template nucleic acid molecule comprising:

(a) providing a pair of samples, each sample comprising at least one target template nucleic acid molecule;
(b) sequencing regions of the at least one target template nucleic acid molecule in a first of the pair of samples to provide non-mutated sequence reads;
(c) introducing mutations into the at least one target template nucleic acid molecule in a second of the pair of samples to provide at least one mutated target template nucleic acid molecule;

(d) sequencing regions of the at least one mutated target template nucleic acid molecule to provide mutated sequence reads;

(e) analysing the mutated sequence reads, and using information obtained from analysing the mutated sequence reads to assemble a sequence for at least a portion of at least one target template nucleic acid molecule from the non-mutated sequence reads,

wherein the first of the pair of samples is provided by pooling two or more sub-samples and/or the second of the pair of samples is provided by pooling two or more sub-samples, and wherein the method further comprises controlling the number of target template nucleic acid molecules in the first of the pair of samples and/or in the second of the pair of samples.

Further embodiments are provided as defined by the dependent claims.

**Brief description of the Figures**

Figure 1 shows the level of mutation achieved with three different polymerases in the presence or absence of dPTP. Panel A shows data obtained using Taq (Jena Biosciences), panel B shows data obtained using LongAmp (New England Biolabs) and panel C shows data using Primestar GXL (Takara). The dark grey bars show the results obtained in the absence of dPTP and the pale grey bars show the results obtained in the presence of 0.5 mM dPTP.

Figure 2 describes the mutation rates obtained obtained by dPTP mutagenesis using a *Thermococcus* polymerase (Primestar GXL; Takara) on templates with diverse G+C content. The median observed rate of mutations was ~7% for low GC templates from S. *aureus* (33% GC), while the median for other templates was about 8%.

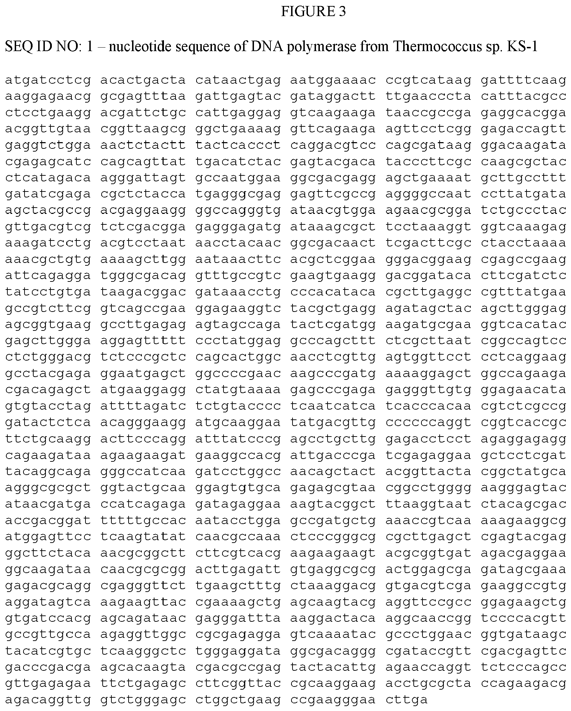

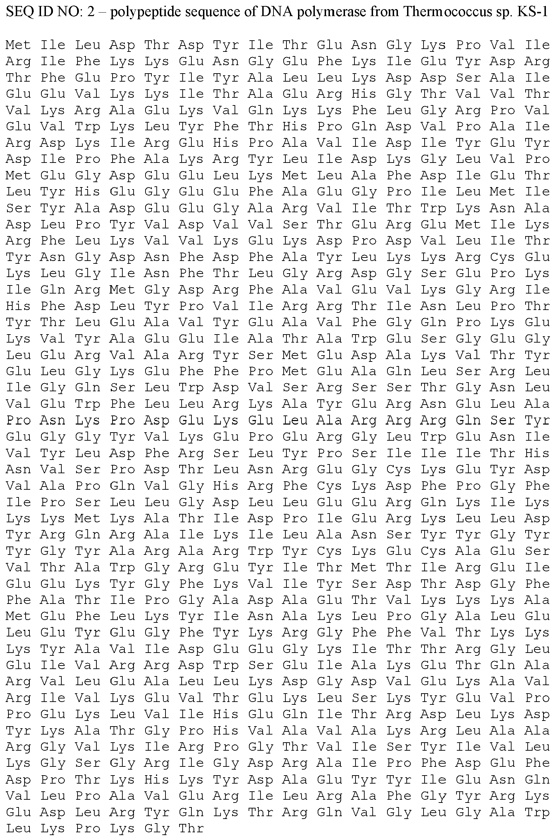

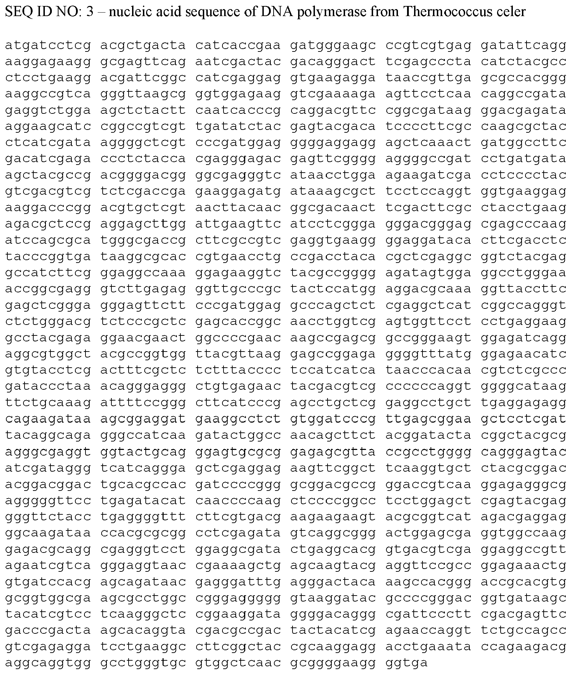

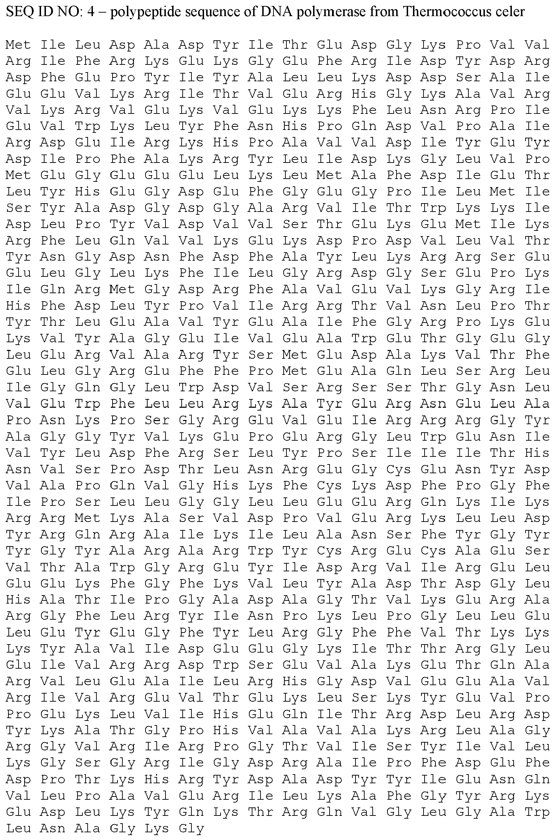

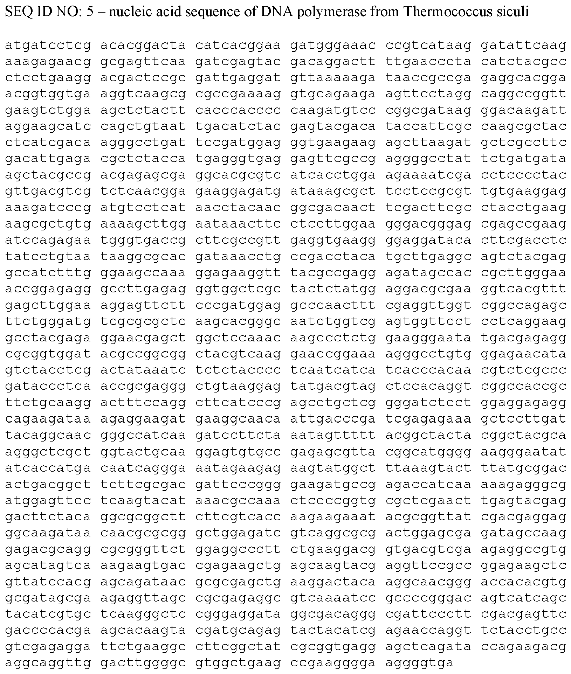

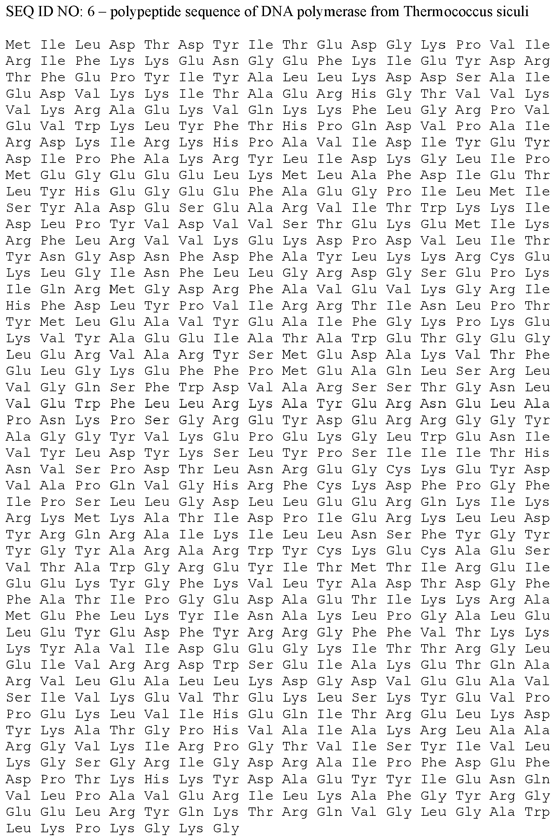

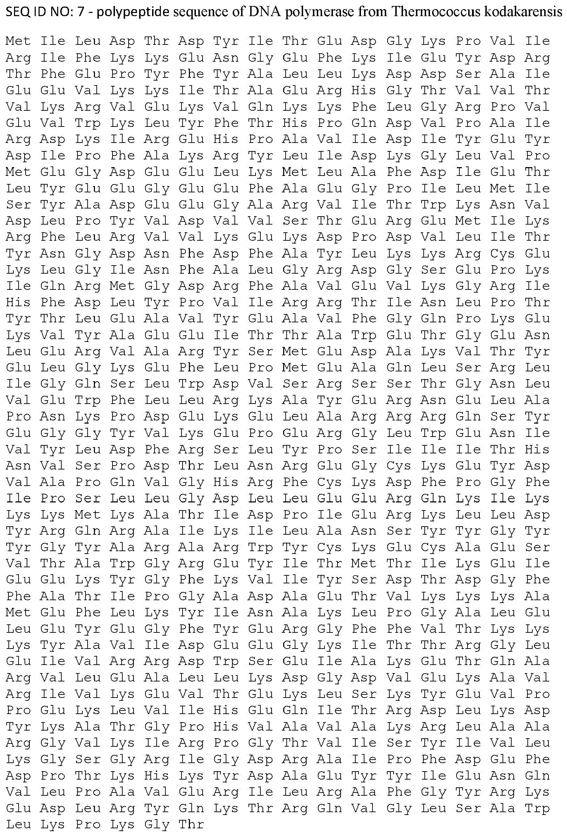

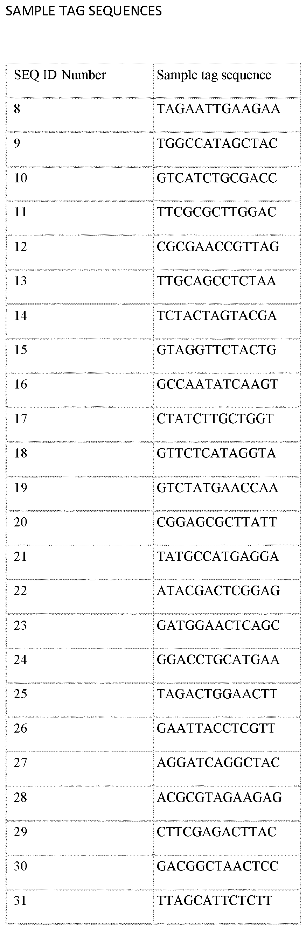

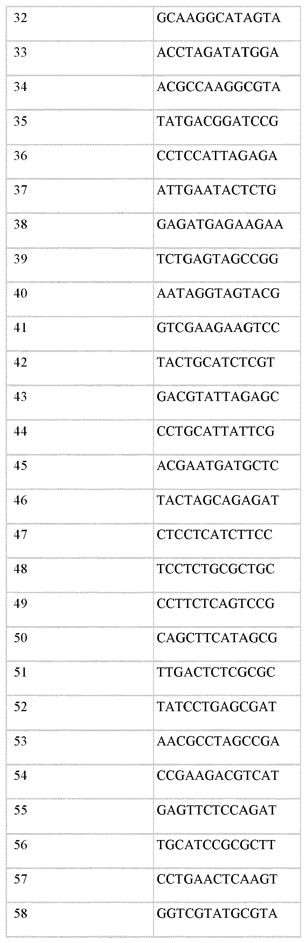

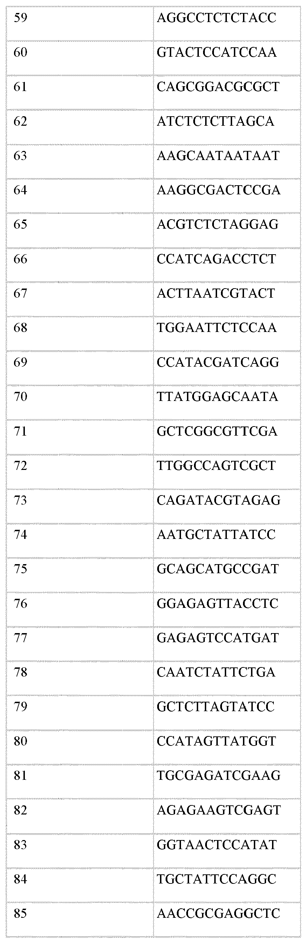

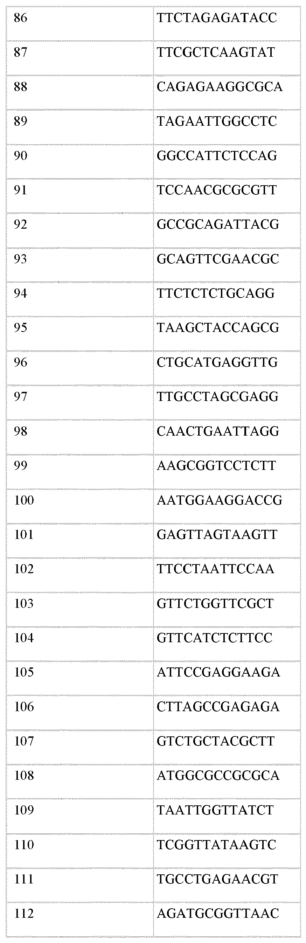

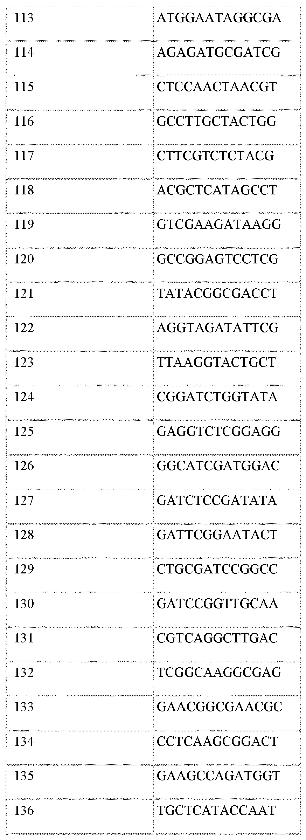

Figure 3 is a sequence listing.

Figure 4 describes the lengths of fragments obtained using the methods described in Example 5.

Figure 5 describes the distribution of values using variational inference on simulated data. Panel A shows the values of M inferred using variational inference on simulated data. True values are 0.895 for identities ([1,1], [2,2], [3,3], [4,4]) and 0.1 for transitions ([1,3],[2,4],[3,1],[4,2]) and 0.005 for transversions (all other entries). Panel B shows the values of $z$ inferred using variational inference on simulated data. True values of $z$ are 1 for same[1:5] and 0 for same[91:95].

Figure 6 is a precision recall plot for simulated data using and cutoff values ranging from 100 to 10,000 in steps of 100. 2,000 tests were performed for each threshold including 1,000 read pairs that did originate from the same template and 1,000 that did not.

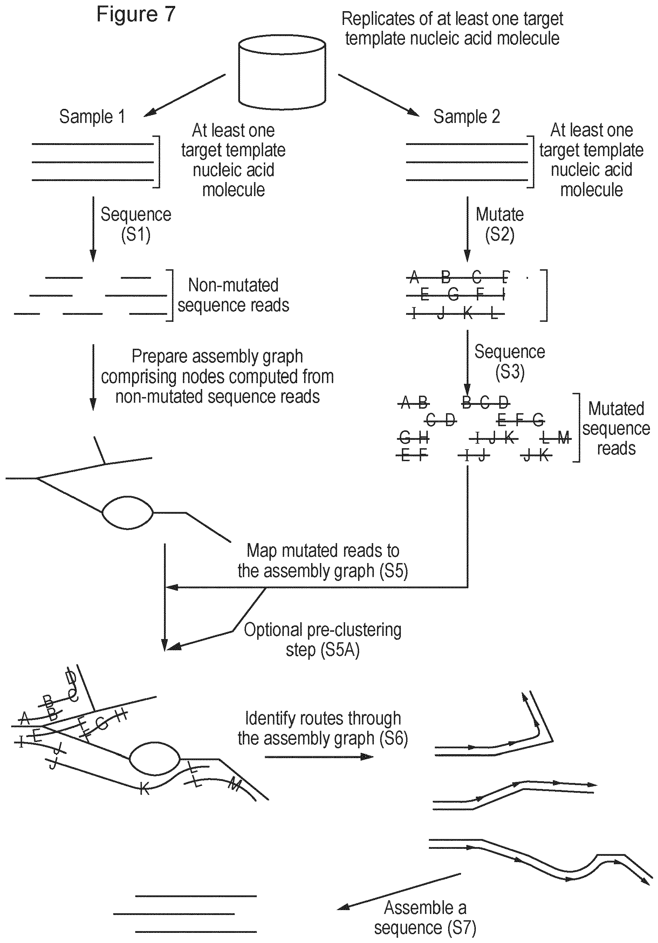

Figure 7 is a flow diagram, illustrating a method for determining a sequence of at least one target template nucleic acid molecule of the invention.

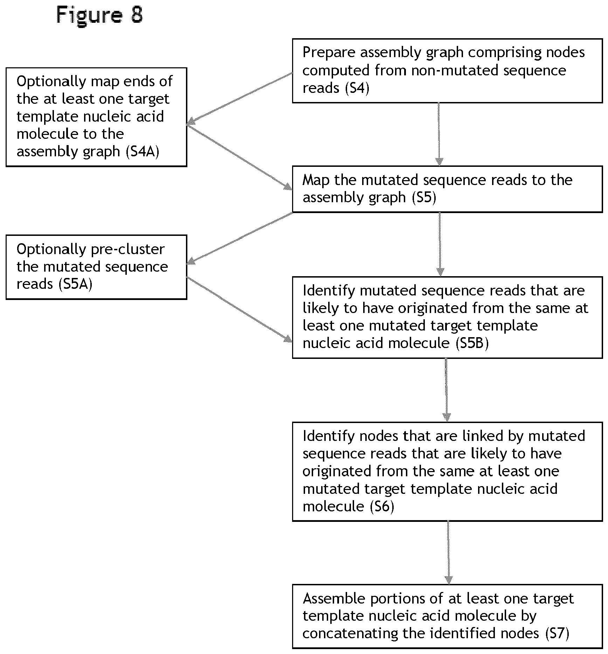

Figure 8 is a flow diagram, illustrating a method for generating a sequence of at least one target template nucleic acid molecule of the invention.

Figure 9 depicts an assembly graph in panel A and mapping mutated sequence reads to the assembly graph in panel B.

Figure 10 depicts the sizes of target nucleic acid molecules amplified using adapters that anneal to one another (right line) or using standard adapters (left line).

Figure 11 is a graph describing a linear relationship between sample dilution factor and observed numbers of unique templates. A starting sample of target template nucleic acid molecules was serially diluted and end sequencing was performed to identify and quantitate the number of unique templates in each dilution.

Figure 12 is a graph showing the normalisation of template counts between individual samples in a pool. (A) shows unique template counts for 66 barcoded bacterial genomes, determined from a pooled sample prior to normalisation.

(B) shows template counts for the same samples after normalisation (expressed per Megabase (Mb) of genome content) showing much less variability.

Figure 13 shows a workflow for the assembly of bacterial genomes according to the present invention.

Figure 14 shows comparison assembly statistics from 65 bacterial genomes for standard read assembly compared to the assembly of the present invention (Morphoseq assemblies).

Figure 15 shows exemplary assembly metrics for the assembly of a bacterial genome for short read assembly compared to the assembly of the present invention.

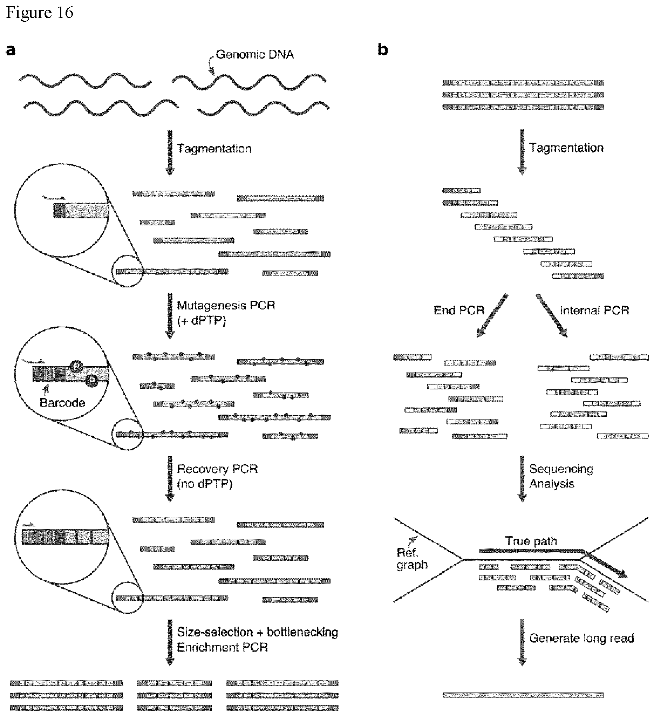

Figure 16 shows an exemplary workflow of the present invention for generating synthetic long reads. (a) Preparation of long mutated templates. Genomic DNA of interest is first tagmented to produce long templates containing end adapters. Templates are then amplified in the presence of the mutageneic nucleotide analogue dPTP, which is randomly incorporated opposite A and G residues on both product strands (mutagenesis PCR). This step also introduces (i) sample tags and (ii) an additional adapter sequence at the template ends to facilitate downstream amplification of products containing the P base. Further amplification is performed in the absence of dPTP (recovery PCR), during which template P residues are replaced with natural nucleotides to generate transition mutations (shown as red lines). The sample is then size-selected (8-10 kb), constrained to a fixed number of unique templates, and selectively enriched to create many copies of each unique molecule. (b) Short-read library preparation, sequencing and analysis. Long mutated templates are processed for short-read sequencing via further tagmentation and library amplification. During this step, fragments derived from the extreme ends of the full-length templates are amplified and barcoded separately from random "internal" fragments using distinct primers targeting the original template end adapters (dark grey) and the internal tagmentation adapters (light grey). Both libraries are sequenced, along with an unmutated reference library generated in parallel, and a custom algorithm is used to reconstruct synthetic long reads. This involves creating an assembly graph from the reference data, to which mutated reads are mapped and linked together via distinct patterns of overlapping mutations. The final synthetic long read corresponds to an identified path through the unmutated assembly graph.

## Detailed Description of the Invention

### General definitions

Unless defined otherwise, technical and scientific terms used herein have the same meaning as commonly understood by a person skilled in the art to which this invention belongs.
In general, the term "*comprising*" is intended to mean including, but not limited to. For example, the phrase "*a method for determining a sequence of at least one target template nucleic acid molecule comprising [certain steps]*" should be interpreted to mean that the method includes the recited steps, but that additional steps may be performed.
In some embodiments of the invention, the word "*comprising*" is replaced with the phrase "*consisting of*". The term "*consisting of*" is intended to be limiting. For example, the phrase "*a method for determining a sequence of at least one target template nucleic acid molecule consisting of [certain steps]*" should be understood to mean that the method includes the recited steps, and that no additional steps are performed.

### A method for determining a sequence of at least one target template nucleic acid molecule

In some aspects, the invention provides a method for determining a sequence of at least one target template nucleic acid molecule or a method for generating a sequence of at least one target template nucleic acid molecule.
For the purposes of the present invention, the terms "*determining*" and "*generating*" may be used interchangeably. However, a method of "*determining*" a sequence generally comprises steps such as sequencing steps, whereas a method of "*generating*" a sequence may be restricted to steps that may be computer-implemented.
The method may be used to determine or generate a complete sequence of the at least one target template nucleic acid molecule. Alternatively, the method may be used to determine or generate a partial sequence, *i.e.* a sequence of a portion of the at least one target template nucleic acid molecule. For example, if it is not possible or not straightforward to determine a complete sequence, the user may decide that the sequence of a portion of the at least one target template nucleic acid molecule is useful or even sufficient for his purpose.
For the purposes of the present invention, a "*nucleic acid molecule*" refers to a polymeric form of nucleotides of any length. The nucleotides may be deoxyribonucleotides, ribonucleotides or analogs thereof. Preferably, the at least one target template nucleic acid molecule is made up of deoxyribonucleotides or ribonucleotides. Even more preferably, the at least one target template nucleic acid molecule is made up of deoxyribonucleotides, *i.e.* the at least one target template nucleic acid molecule is a DNA molecule.

The at least one "*target template nucleic acid molecule*" can be any nucleic acid molecule which the user would like to sequence. The at least one "*target template nucleic acid molecule*" can be single stranded, or can be part of a double stranded complex. If the at least one target template nucleic acid molecule is made up of deoxyribonucleotides, it may form part of a double stranded DNA complex. In which case, one strand (for example the coding strand) will be considered to be the at least one target template nucleic acid molecule, and the other strand is a nucleic acid molecule that is complementary to the at least one target template nucleic acid molecule. The at least one target template nucleic acid molecule may be a DNA molecule corresponding to a gene, may comprise introns, may be an intergenic region, may be an intragenic region, may be a genomic region spanning multiple genes, or may, indeed, be an entire genome of an organism.

The terms "*at least one target template nucleic acid molecule*" and "*at least one target template nucleic acid molecules*" are considered to be synonymous and may be used interchangeably herein.

In the methods of the invention, any number of at least one target template nucleic acid molecules may be sequenced simultaneously. Thus, in an embodiment of the invention, the at least one target template nucleic acid molecule comprises a plurality of target template nucleic acid molecules. Optionally, the at least one target template nucleic acid molecule comprises at least 10, at least 20, at least 50, at least 100, or at least 250 target template nucleic acid molecules. Optionally, the at least one target template nucleic acid molecule comprises between 10 and 1000, between 20 and 500, or between 50 and 100 target template nucleic acid molecules.

The method for determining a sequence of at least one target template nucleic acid molecule may comprise:

(a) providing a pair of samples, each sample comprising at least one target template nucleic acid molecule;
(b) sequencing regions of the at least one target template nucleic acid molecule in a first of the pair of samples to provide non-mutated sequence reads;
(c) introducing mutations into the at least one target template nucleic acid molecule in a second of the pair of samples to provide at least one mutated target template nucleic acid molecule;
(d) sequencing regions of the at least one mutated target template nucleic acid molecule to provide mutated sequence reads;
(e) analysing the mutated sequence reads, and using information obtained from analysing the mutated sequence reads to assemble a sequence for at least a portion of at least one target template nucleic acid molecule from the non-mutated sequence reads.

The method for generating a sequence of at least one target template nucleic acid molecule may comprise:

(a) obtaining data comprising:

(i) non-mutated sequence reads; and
(ii) mutated sequence reads;

(b) analysing the mutated sequence reads, and using information obtained from analysing the mutated sequence reads to assemble a sequence for at least a portion of at least one target template nucleic acid molecule from the non-mutated sequence reads.

*Providing a pair of samples, each sample comprising at least one target template nucleic acid molecule*

The method for determining a sequence of at least one target template nucleic acid molecule may comprise a step of providing a pair of samples, each sample comprising at least one target template nucleic acid molecule.

The methods of the invention use information obtained by analysing mutated sequence reads to assemble a sequence for at least a portion of at least one target template nucleic acid molecule from non-mutated sequence reads. The methods of the invention may comprise introducing mutations into the at least one target template nucleic acid molecule in a second of the pair of samples. Thus, sequencing regions of the at least one mutated target template nucleic acid molecule in the second of the pair of samples can be used to provide mutated sequence reads, and sequencing regions of the at least one non-mutated target template nucleic acid molecule in the first of the pair of samples can be used to provide non-mutated sequence reads.

In order for the user to be able to use information obtained by analysing mutated sequence reads from the second sample to assemble a sequence comprising predominantly non-mutated sequences from the first sample, some of the mutated sequence reads and some of the non-mutated sequence reads will correspond to the same original target template nucleic acid molecule.

For example, if the user wishes to determine the sequence of target template nucleic acid molecules A and B, then the first sample will comprise template nucleic acid molecules A and B and the second sample will comprise template nucleic acid molecules A and B. A and B in the first sample may be sequenced to provide non-mutated sequence reads of A and B, and A and B in the second sample may be mutated and sequenced to provide mutated sequence reads of A and B.

Since the first of the pair of samples and the second of the pair of samples both comprise the at least one target template nucleic acid molecule, the pair of samples may be derived from the same target organism or taken from the same original sample.

For example, if the user intends to sequence the at least one target template nucleic acid molecule in a sample, the user may take a pair of samples from the same original sample. Optionally, the user may replicate the at least one target template nucleic acid molecule in the original sample before the pair of samples is taken from it. The user may intend to sequence various nucleic acid molecules from a particular organism, such as *E.coli.* If this is the case, the first of the pair of samples may be a sample of *E.coli* from one source, and the second of the pair of samples may be a sample of *E.coli* from a second source.

The pair of samples may originate from any source that comprises, or is suspected of comprising, the at least one target template nucleic acid molecule. The pair of samples may comprise a sample of nucleic acid molecules derived from a human, for example a sample extracted from a skin swab of a human patient. Alternatively, the pair of samples may be derived from other sources such as a water supply. Such samples could contain billions of template nucleic acid molecules. It would be possible to sequence each of these billions of target template nucleic acid molecules simultaneously using the methods of the invention, and so there is no upper limit on the number of target template nucleic acid molecules which could be used in the methods of the invention.

In an embodiment, multiple pairs of samples may be provided. For example, 2, 3, 4, 5, 6, 7, 8, 9, 10, 11, 15, 20, 25, 50, 75, or 100 pairs of samples may be provided. Optionally, less than 100, less than 75, less than 50, less than 25, less than 20, less than 15, less than 11, less than 10, less than 9, less than 8, less than 7, less than 6, less than 5, or less than 4 samples are provided. Optionally, between 2 and 100, 2 and 75, 2 and 50, between 2 and 25, between 5 and 15, or between 7 and 15 pairs of samples are provided.

Where multiple pairs of samples are provided, the at least one target template nucleic acid molecules in different pairs of samples may be labelled with different sample tags. For example, if the user intends to provide 2 pairs of samples, all or substantially all of the at least one target template nucleic acid molecules in the first pair of samples may be labelled with sample tag A, and all or substantially all of the at least one target template nucleic acid molecules in the second pair of samples may be labelled with sample tag B. Sample tags are discussed in more detail under the heading "*Sample tags and barcodes*".

*Controlling the number of target template nucleic acid molecules in a sample*

As described above, the sequencing methods of the present invention comprise assembling a sequence for at least a portion of at least one target template nucleic acid molecule from non-mutated reads using information obtained from analysing corresponding mutated sequence reads. Typically, target template nucleic acid molecules in a sample may be assembled to generate the sequence of a larger nucleic acid molecule or molecules present in a sample. By way of a representative embodiment, target template nucleic acid molecules may be assembled to generate the sequence of a genome. Performing a sequencing run generates a certain finite amount of data, in the form of the sequencing reads which are obtained. In order to assemble the sequence of a target template nucleic acid molecule from the sequencing reads obtained therefrom (and thus to assemble the target template nucleic acid molecules to generate the sequence of a larger target template nucleic acid molecule or molecules), it is preferable to ensure that the coverage of the target template nucleic acid molecules amongst the sequencing reads is adequate (i.e. sufficient to assemble the sequence) without an excessive degree of redundant (i.e. duplicative) sequencing reads being generated for each target template nucleic acid molecule. For example, if a sample contains too many target template nucleic acid molecules for a sufficient number of sequencing reads to be generated from each target template nucleic acid molecule, it may not be possible to assemble the sequence of each target template nucleic acid molecule (i.e. there may not be sufficient data for each template). On the other hand, if a sample contains too few target template nucleic acid molecules, whilst it may be possible to assemble each target template nucleic acid molecule, it may not be possible to assemble the target template nucleic acid molecules to generate the sequence of a larger nucleic acid molecule *e.g.* it may not be possible to generate the sequence of a genome (*i.e.* there may be an excess of data for each template, and thus insufficient data for the sample as a whole).

With these considerations in mind, it is advantageous for the user to be able to control the number of unique target template nucleic acid molecules which are present in the first of the pair of samples and/or the second of the pair of samples. The user can then select the optimal number of unique target template nucleic acid molecules that are present in the first of the pair of samples and/or the second of the pair of samples. The optimal number of unique target template nucleic acid molecules may depend on a number of different factors, which the user will appreciate. For example, if the target template nucleic acid molecules are longer, they will be more difficult to sequence and the user may wish to select a smaller number of unique target template nucleic acid molecules.

Accordingly, the methods of the invention may comprise a step of providing a pair of samples, each sample comprising at least one target template nucleic acid molecule which step comprises controlling the number of target template nucleic acid molecules in a first and/or a second of the pair of samples.

It may be useful to control the number of target template nucleic acid molecules in the first of the pair of samples. However, it is particularly preferred that the number of target template nucleic acid molecules in the second of the pair of samples is controlled for the second of the pair of samples (*i.e.* the sample comprising at least one target template nucleic acid molecule into which mutations will be introduced). In the methods of the invention, the at least one target template nucleic acid molecule in the second of the pair of samples is mutated, and used to reconstruct the sequence of a target template nucleic acid molecule. In this context, the number of target template nucleic acid molecules in the second of the pair of samples can be crucial. Thus, it may be particularly advantageous to control the number of target template nucleic acid molecules in the second of the pair of samples.

Similarly, in one aspect of the invention, there is provided a method for determining a sequence of at least one target template nucleic acid molecule comprising:

(a) providing at least one sample comprising the at least one target template nucleic acid molecule;
(b) sequencing regions of the at least one target template nucleic acid molecule; and
(c) assembling a sequence of the at least one target template nucleic acid molecule from the sequences of the regions of the at least one target template nucleic acid molecule, wherein the step of providing at least one sample comprising the at least one target template nucleic acid molecule comprises controlling the number of target template nucleic acid molecules in the at least one sample.

Similarly, in one aspect of the invention, there is provided a method for determining a sequence of at least one target template nucleic acid molecule comprising:

(a) providing at least one sample comprising the at least one target template nucleic acid molecule;
(b) sequencing regions of at least a portion of the at least one target template nucleic acid molecule; and
(c) assembling a sequence of the at least one target template nucleic acid molecule from the sequences of the regions of the at least one target template nucleic acid molecule, wherein the step of providing at least one sample comprising the at least one target template nucleic acid molecule comprises controlling the number of target template nucleic acid molecules in the at least one sample.

For the purposes of the present application, the phrase "*controlling the number of target template nucleic acid molecules*" in a sample refers to providing a number of target template nucleic acid molecules that is desired in the sample. According to certain particular embodiments, this may comprise manipulating or adjusting the sample such that it contains the desired number of target template nucleic acid molecules (for example by diluting the sample or pooling the sample with another sample that also comprises target template nucleic acid molecules).

It will be appreciated that "*controlling the number of target template nucleic acid molecules*" may not be entirely precise as, for example, it is difficult to achieve a precise number of template nucleic acid molecules by diluting a sample using conventional techniques. However, if the user finds that the sample comprises around twice as many target template nucleic acid molecules as desired, the user may dilute the sample and achieve a diluted sample comprising approximately half of the number of target template nucleic acid molecules present in the original sample (for example between 45% and 55% of the number of target template nucleic acid molecules present in the original sample).

Controlling the number of target template nucleic acid molecules may comprise measuring the number of target template nucleic acid molecules in the sample (for example the user may measure the number of target template nucleic acid molecules in the first of the pair of samples, the second of the pair of samples or the at least one sample). The term "*measuring*" may be substituted herein by the term "*estimating*". In general, measuring the number of target template nucleic acid molecules in the sample is used as part of a step of controlling the number of target template nucleic acid molecules in a sample, and the step of controlling the number of target template nucleic acid molecules in a sample can be used to help the user to ensure that the sample comprises a number of target template nucleic acid molecules which is appropriate (*i.e.* within a desired range) for use in a particular sequencing method. However, there is no requirement for such a step of controlling the number of target template nucleic acid molecules to be completely accurate. A method for approximately controlling the number of target template nucleic acid molecules in the sample would be helpful to improve a method of sequencing a target template nucleic acid molecule. In an embodiment, "*measuring the number of target template nucleic acid molecules*" refers to determining the number of target template nucleic acid molecules in a sample to within at least the correct order of magnitude, *i.e.* within a factor of 10, or more preferably within a factor of 5, 4, 3 or 2 compared to the true number. More preferably, the number of target template nucleic acid molecules in a sample may be determined within at least 50%, or at least 40%, or at least 30%, or at least 25%, or at least 20%, or at least 15%, or at least 10% of the true number. Any method may be used to measure the number of target template nucleic acid molecules in the sample.

A sample (*e.g.* the first of the pair of samples, the second of the pair of samples, or the at least one sample) may be diluted prior to or in the course of measuring the number of target template nucleic acid molecules in the sample. For example, if the user believes that the sample comprises a large number of target template nucleic acid molecules, he may wish to dilute the sample in order to obtain a sample having an appropriate number of target template nucleic acid molecules to measure accurately by, for example, sequencing. Thus, a diluted sample may be provided. Accordingly, the number of target template nucleic acid molecules may be measured in a diluted sample, thereby to determine the number of target template nucleic acid molecules in a sample.

According to certain embodiments it may be advantageous for more than one diluted sample to be prepared, each at a different dilution factor. For example, if the user does not have a good idea of how many target template nucleic acid molecules are present in the sample, he may wish to prepare a dilution series and measure the number of target template nucleic acid molecules in each dilution (*i.e.* in each diluted sample). Thus, measuring the number of target template nucleic acid molecules may comprise preparing a dilution series on the first of the pair of samples, the second of the pair of samples, or the at least one sample to provide a dilution series comprising diluted samples. A dilution series may comprise between 1 and 50, between 1 and 25, between 1 and 20, between 1 and 15, between 1 and 10, between 1 and 5 diluted samples, between 5 and 25, between 5 and 20, between 5 and 15, or between 5 and 10 diluted samples.

Such a dilution series may be prepared by performing a serial dilution. Optionally, the samples may be diluted between 2-fold and 20-fold, between 5-fold and 15-fold, or around 10-fold. For example, in order to obtain a dilution series of 10 samples each diluted 10-fold, the user will prepare a 10-fold dilution of the sample, then isolate a portion of the diluted sample and dilute that a further 10-fold and so on until 10 diluted samples are obtained.

The user may prepare 10 diluted samples, but only determine the number of target template nucleic molecules in fewer than 10 of the diluted samples. For example, if the user determines the number of target template nucleic acid molecules in 5 of the diluted samples, and determines the number of target template nucleic acid molecules accurately in the fifth diluted sample, there is no need to further determine the number of target template nucleic acid molecules in any of the other diluted samples. In yet further embodiments, the user may correlate results from multiple diluted samples in order to be more confident in the result. Advantageously, this may also provide the user with information regarding the dynamic range over which the number of target template nucleic acid molecules in the sample may be accurately determined under a given set of conditions. The user may, however, only perform a single dilution in order to accurately determine the number of target template nucleic acid molecules in a sample.

According to certain particular embodiments, the number of target template nucleic acid molecules in a sample (or a diluted sample) may be measured by determining the molar concentration of the target template nucleic acid molecules in the sample. This may be done, for example, by electrophoresis. According to a particular embodiment, the number of target template nucleic acid molecules in a sample may be determined by high resolution microfluidic electrophoresis, whereby a sample may be loaded into a microchannel and target template nucleic acid molecules may be electrophoretically separated, and detected by their fluorescence. Suitable systems for measuring the number of target template nucleic acid molecules in this way include the Agilent 2100 Bioanalyzer and the Agilent 4200 Tapestation.

In alternative embodiments, the number of target template nucleic acid molecules may be measured by sequencing the target template nucleic acid molecules in the first of the pair of samples, the second of the pair of samples, the at least one sample or one or more of the diluted samples.

According to a particular embodiment, the method may comprise measuring the number of target template nucleic acid molecules by sequencing the target template nucleic acid molecules in one or more of the diluted samples.

The target template nucleic acids may be sequenced using any method of sequencing. Examples of possible sequencing methods include Maxam Gilbert Sequencing, Sanger Sequencing, sequencing comprising bridge amplification (such as bridge PCR), or any high throughput sequencing (HTS) method as described in Maxam AM, Gilbert W (February 1977), "A new method for sequencing DNA ", Proc. Natl. Acad. Sci. U. S. A. 74 (2): 560-4, Sanger F, Coulson AR (May 1975), "A rapid method for determining sequences in DNA by primed synthesis with DNA polymerase ",J. Mol. Biol. 94 (3): 441-8; and Bentley DR, Balasubramanian S, et al. (2008), "Accurate whole human genome sequencing using reversible terminator chemistry", Nature, 456 (7218): 53-59. Measuring the number of target template nucleic acid molecules may comprise amplifying and then sequencing the target template nucleic acid molecules (or viewed another way, the amplified target template nucleic acid molecules) in the first of the pair of samples, the second of the pair of samples, the at least one sample, or one or more of the diluted samples. Amplifying the target template nucleic acid molecules provides the user with multiple copies of the target template nucleic acid molecules, enabling the user to sequence the target template nucleic acid molecule more accurately (as sequencing technology is not completely accurate, sequencing multiple copies of the target template nucleic acid sequence and then calculating a consensus sequence from the sequences of the copies improves accuracy). Making multiple copies of a fixed number of unique target template nucleic acid molecules in a sample and sequencing a fraction of the total (amplified) sample allows sequence information from all of the target template nucleic acid molecules to be obtained.

Suitable methods for amplifying the at least one target template nucleic acid molecule are known in the art. For example, PCR is commonly used. PCR is described in more detail below under the heading *"introducing mutations into the at least one target template nucleic acid molecule* ".

In a typical embodiment the sequencing step may involve bridge amplification. Optionally, the bridge amplification step is carried out using an extension time of greater than 5, greater than 10, greater than 15, or greater than 20 seconds. An example of the use of bridge amplification is in Illumina Genome Analyzer Sequencers. Preferably paired-end sequencing is used.

Measuring the number of target template nucleic acid molecules may comprise fragmenting the target template nucleic acid molecules in the first of the pair of samples, the second of the pair of samples, the at least one sample or one or more of the diluted samples. This may be particularly advantageous, for example, where a sequencing platform precludes the use of a long nucleic acid molecule as a template. The fragmenting may be carried out using any suitable technique. For example, fragmentation can be carried out using restriction digestion or using PCR with primers complementary to at least one internal region of the at least one mutated target nucleic acid molecule. Preferably, fragmentation is carried out using a technique that produces arbitrary fragments. The term *"arbitrary fragment"* refers to a randomly generated fragment, for example a fragment generated by tagmentation. Fragments generated using restriction enzymes are not *"arbitrary"* as restriction digestion occurs at specific DNA sequences defined by the restriction enzyme that is used. Even more preferably, fragmentation is carried out by tagmentation. If fragmentation is carried out by tagmentation, the tagmentation reaction optionally introduces an adapter region into the target template nucleic acid molecules. This adapter region is a short DNA sequence which may encode, for example, adapters to allow the at least one target nucleic acid molecule to be sequenced using Illumina technology.

In particular embodiments, measuring the number of target template nucleic acid molecules comprises amplifying and fragmenting the target template nucleic acid molecules, and then sequencing the target template nucleic acid molecules (or viewed another way, the amplified and fragmented target template nucleic acid molecules) in the first of the pair of samples, the second of the pair of samples, the at least one sample or one or more of the diluted samples. Amplification and fragmentation may be performed in any order prior to sequencing. In an embodiment, measuring the number of target template nucleic acid molecules may comprise amplifying, then fragmenting and then sequencing the target template nucleic acid molecules in the first of the pair of samples, the second of the pair of samples, the at least one sample or one or more of the diluted samples. Alternatively, measuring the number of target template nucleic acid molecules may comprise fragmenting, then amplifying, and then sequencing the target template nucleic acid molecules in the first of the pair of samples, the second of the pair of samples, the at least one sample or one or more of the diluted samples. Amplification and fragmentation may alternatively be performed simultaneously, *i.e.* in a single step. It can be useful for the method to comprise fragmenting and then amplifying the target template nucleic acid molecules when the target template nucleic acid molecules are very long (for example too long to be sequenced using conventional technology).

Measuring the number of target template nucleic acid molecules may comprise identifying the total number of target template nucleic acid molecules in a sample. Preferably, however, measuring the number of target template nucleic acid molecules comprises identifying the number of unique target template nucleic acid molecule sequences in the first of the pair of samples, the second of the pair of samples, the at least one sample or one or more of the diluted samples. As discussed above, determining a sequence of at least one target template nucleic acid sequence is more difficult when the at least one target template nucleic acid sequence is part of a sample comprising many different target template nucleic acid sequences. Thus, reducing the number of unique target template nucleic acid molecules makes a method of determining a sequence of at least one target template nucleic acid molecule simpler.

As discussed elsewhere herein, introducing mutations into a target template nucleic acid sequence may facilitate the assembly of at least a portion of the sequence of a target template nucleic acid. Mutating target template nucleic acid molecules may be particularly beneficial, for example, in identifying whether sequence reads are likely to have originated from the same target template nucleic acid molecule, or whether the sequence reads are likely to have originated from different target template nucleic acid molecules. According to certain embodiments of the present aspect of the invention, it may, therefore, be beneficial to introduce mutations into target template nucleic acid molecules where the number of target template nucleic acid molecules is to be measured by sequencing. Thus, in particular such embodiments, measuring the number of target template nucleic acid molecules may comprise mutating the target template nucleic acid molecules.

Mutating the target template nucleic acid molecules may be performed by any convenient means. In particular, mutating the target template nucleic acid molecules may be performed as described elsewhere herein. According to a particularly preferred embodiment, mutations may be introduced by using a low bias DNA polymerase. In additional or alternative embodiments, mutating the target template nucleic acid molecules may comprise amplifying the target template nucleic acid molecules in the presence of a nucleotide analog, for example dPTP.

According to preferred embodiments, measuring the number of target template nucleic acid molecules may comprise:

(i) mutating the target template nucleic acid molecules to provide mutated target template nucleic acid molecules;
(ii) sequencing regions of the mutated target template nucleic acid molecules; and
(iii) identifying the number of unique mutated target template nucleic acid molecules based on the number of unique mutated target template nucleic acid molecule sequences.

In order to quantitate the number of target template nucleic acid molecules in the sample, the user does not require a complete sequence for each target template nucleic acid molecule. Rather, all that is required is sufficient information about the sequence of the different target template nucleic acid molecules in the sample (or where applicable, amplified and fragmented target template nucleic acid molecules) to allow the user to estimate the total number of target template nucleic acid molecules and/or the number of unique target template nucleic acid molecules. For this reason, the user may opt to sequence only a region of each target template nucleic acid molecule. For example, in certain embodiments, the user may opt to sequence an end region of each unique target template nucleic acid molecule or fragmented target template nucleic acid molecules as part of the step of measuring the number of unique target template nucleic acid molecules. The user may, therefore, sequence the 3' end region and/or the 5' end region of the target template nucleic acid molecules or fragmented target template nucleic acid molecules as part of the step of measuring the number of target template nucleic acid molecules. An end region of a target template nucleic acid molecule encompasses the terminal (*e.g.* the 5' or 3' terminal) nucleotide in a target template nucleic acid molecule (*i.e.* the 5'-most or 3'-most nucleotide in a target template nucleic acid molecule) and the contiguous stretch of nucleotides adjacent thereto of the desired length.

According to certain representative embodiments, measuring the number of target template nucleic acid molecules may comprise introducing barcodes (also referred to as unique molecular tags or unique molecular identifiers herein, as described below) or a pair of barcodes into the target template nucleic acid molecules (or put another way, labelling the target template nucleic acid molecules with barcodes or a pair of barcodes) to provide barcoded target template nucleic acid molecules. As described elsewhere herein, barcodes are suitably degenerate that substantially each target template nucleic acid molecule may comprise a unique or substantially unique sequence, such that each (or substantially each) target template nucleic acid molecule is labelled with a different barcode sequence. The introduction of barcodes into target template nucleic acid molecules may be performed as described elsewhere herein. In particular embodiments, the barcode sequences may be introduced at the ends of the target template nucleic acid molecules, *i.e.* as additional sequences 5' to the 5' terminal (or 5'-most) or 3' to the 3' terminal (or 3'-most) nucleotide in a target template nucleic acid molecule.

In a preferred embodiment, target template nucleic acid molecules labelled with barcode sequences may be sequenced in order to measure the number of target template nucleic acid molecules in a sample. More particularly, regions of the target template nucleic acid molecules which comprise the barcode sequences may be sequenced in order to measure the number of target template nucleic acid molecules in a sample. Barcode sequences are substantially unique and labelling target template nucleic acid molecules with barcode sequences thus introduces substantially unique (and therefore countable) sequences into the target template nucleic acid molecules. Thus, the number of unique barcodes which are identified by sequencing according to such an embodiment may allow the determination of the number of unique target template nucleic acid molecules in the sample.

Thus, according to certain embodiments, measuring the number of target template nucleic acid molecules may comprise:

(i) sequencing regions of the barcoded target template nucleic acid molecules comprising the barcodes or the pairs of barcodes; and
(ii) identifying the number of unique barcoded target template nucleic acid molecules based on the number of unique barcodes or pairs of barcodes.

According to yet further embodiments, it may not be necessary to use a barcode or barcodes in order to determine the number of target template nucleic acid molecule present in a sample. In a particular representative embodiment, the number of target template nucleic acid molecules may be determined by sequencing end regions of the target template nucleic acid molecules. Optionally, the user then identifies the number of unique end sequences present, and/or the user then maps the sequences of the end regions against a reference sequence, for example a reference genome. Without wishing to be bound by theory, it is believed that such an approach may allow the number of target template nucleic acid molecules to be determined as the sequence for each target template nucleic acid molecule may start at a different site in the reference sequence.

Furthermore, the sequencing step according to this aspect of the invention may be a *"rough"* sequencing step, in that the user may not need precise sequence information in order to be able to measure the number of target template nucleic acid molecules in a sample. By way of a representative example, the sequencing step may be performed on a poorly amplified set of molecules, which may allow this step to be performed more quickly and/or at lower cost.

Optionally, measuring the number of unique target template nucleic acid molecules in a sample may comprise sequencing end regions of barcoded target template nucleic acid molecules comprising barcodes or pairs of barcodes. Thus, reference to sequencing the end regions of target template nucleic acid molecules may encompass sequencing end regions of barcoded target template nucleic acid molecules which may comprise a barcode or a pair of barcodes.

Once the number of unique target template nucleic acid molecules in a sample is measured, the sample may be adjusted in order to control the number of target template nucleic acid molecules in the sample, such that the sample comprises a desired number of unique target template nucleic acid molecules. According to certain embodiments, this may comprise a step of diluting the sample. Thus, controlling the number of target template nucleic acid molecule in a sample may comprise measuring the number of target template nucleic acid molecules in the sample, and diluting the sample such that the sample comprises a desired number of target template nucleic acid molecules.

As noted above, the sample according to this aspect of the invention may be any sample, and in particular may be a first or a second sample according to methods of the present invention. Thus, according to particular embodiments, controlling the number of target template nucleic acid molecules in a first of a pair of samples and/or a second of a pair of samples a comprise measuring the number of target template nucleic acid molecules and diluting the first of the pair of samples and/or the second of the pair of samples such that the first of the pair of samples and/or the second of the pair of samples comprises a desired number of target template nucleic acid molecules.

*Pooling sub-samples to provide a sample*

A sample may be provided by pooling several sub-samples. This may allow target template nucleic acid molecules from multiple samples (*e.g.* from multiple sources) to be sequenced simultaneously, which in turn may allow greater sample throughput to be achieved, reducing the cost and time required for determining the sequences of target template nucleic acid molecules.

The methods of the present invention may therefore be performed on samples provided by pooling two or more sub-samples. According to certain embodiments, the first of the pair of samples may be provided by pooling two or more sub-samples. In further embodiments, the second of the pair of samples may be provided by pooling two or more sub-samples. Thus, the first and/or the second sample may be provided by pooling two or more sub-samples. First and second samples may alternatively be taken from a pooled sample, and subjected to the methods of the present invention.

This aspect of the present invention therefore allows the sequence of at least one target template nucleic acid molecule from each of the two or more smaller samples which are pooled to provide the sample to be determined.

One problem associated with pooling samples for sequencing is that each sample may contain a different number of target nucleic acid molecules. It may therefore be beneficial for a pooled sample to contain target template nucleic acid molecules from each of its constituent sub-samples in a desired amount, and more particularly, in a desired ratio. Put another way, it may be beneficial for a pooled sample to comprise a number of unique target template nucleic acid molecules from each of its sub-samples which is appropriate (*i.e.* within a desired range), such that a particular sequencing method may be used for sequencing the target template nucleic acid molecules from each of the sub-samples in the pooled sample.

By way of representative example, two separate sub-samples, sample Y and sample Z, may be provided. If the total number of target template nucleic acid molecules in sample Y is 100x greater than the total number of target template nucleic acid molecules in sample Z, pooling samples Y and Z in equal amounts and subjecting the pooled sample to a sequencing method, would be expected to result in the number of sequencing reads arising from target template nucleic acid molecules in sample Y to be 100x greater than the number of sequencing reads arising from target template nucleic acid molecules in sample Z. Pooling samples in this way may, therefore, not only result in insufficient sequencing reads arising from sample Z to allow a sequence assembly step to be performed using sequence reads obtained from sample Z, it may also complicate performing a sequence assembly step on sequencing reads obtained from sample Y.

Accordingly, the methods of the invention may comprise a step of normalising the number of target template nucleic acid molecules in each of the sub-samples that are pooled to provide the first of the pair of samples and/or the second of the pair of samples.

More generally, however, the present invention provides a method for determining a sequence of at least one target template nucleic acid molecule comprising:

(a) providing at least one sample comprising the at least one target template nucleic acid molecule;
(b) sequencing regions of the at least one target template nucleic acid molecule; and
(c) assembling a sequence of the at least one target template nucleic acid molecule from the sequences of the regions of the at least one target template nucleic acid molecule, wherein the at least one sample is provided by pooling two or more sub-samples and the number of target template nucleic acid molecules in each of the sub-samples is normalised.

For the purposes of the present application the phrases *"the number of target template nucleic acid molecules in each of the sub-samples is normalised'* and *"normalising the number of target template nucleic acid molecules in each of the sub-samples that are pooled"* refer to pooling sub-samples in such a way that the total number of target template nucleic acid molecules in the pooled sample which derive from each of the sub-samples is provided at a desired amount. In some embodiments, the number of unique target template nucleic acid molecules is normalised. *"Unique target template nucleic acid* molecules" are target template nucleic acid molecules comprising different nucleic acid sequences. Optionally, each of the at least one target template nucleic acid molecule is a unique target template nucleic acid molecule. Unique target template nucleic acid molecules may differ by as little as a single nucleotide in sequence, or may be substantially different to one another.

A normalising step may advantageously allow the number of target template nucleic acid molecules from each of the sub-samples to be provided in a desired ratio. According to certain embodiments, this may comprise manipulating or adjusting each of the sub-samples such that, when pooled, the pooled sample contains the desired number of target template nucleic acid molecules from each of the sub-samples. Viewed another way, this step may be seen to allow the number of target template nucleic acid molecules in a pooled sample which are from each of the two or more sub-samples to be controlled, or controlling the number of target template nucleic acid molecules in the at least one sample from each of the two or more sub-samples.

Alternatively viewed, the present invention thus provides a method for determining the sequence of at least one target template nucleic acid molecule comprising:

(a) providing at least one sample comprising the at least one target template nucleic acid molecule;
(b) sequencing regions of the at least one target template nucleic acid molecule; and
(c) assembling a sequence of the at least one target template nucleic acid molecule from the sequences of the regions of the at least one target template nucleic acid molecule, wherein the step of providing at least one sample comprising the at least one target template nucleic acid molecule comprises pooling two or more sub-samples and controlling the number of target template nucleic acid molecules in the at least one sample from each of the two or more sub-samples.

According to certain embodiments, normalising the number of target template nucleic acid molecules in each of the sub-samples may comprise providing a similar number of target template nucleic acid molecules in the pooled sample from each of the sub-samples (*i.e.* in approximately a 1:1 ratio). Such an embodiment may be particularly useful, for example, where each sub-sample is derived from a sample containing genome(s) of similar size. In alternative embodiments, however, the number of target template nucleic acid molecules may be provided in a different amount, *i.e.* the number of target template nucleic acid molecules from a first sub-sample may be provided at a higher abundance than the number of target template nucleic acid molecules from a second sub-sample. Such an embodiment may be desirable, for example, if a first sub-sample is derived from a larger genome and a second sub-sample is derived from a sample containing a smaller genome.

It will be understood that *"normalising the number of target template nucleic acid molecules in each of the sub-samples that are pooled'* may not be entirely precise, as, for example, it may be difficult to measure the number of target template nucleic acid molecules in each of the sub-samples. However, if the user finds that a sub-sample contains around twice as many target template nucleic acid molecules as desired, the user may normalise the number of target template nucleic acid molecules in the sub-sample such that the number of target template nucleic acid molecules in the pooled sample is approximately half the number of target template nucleic acid molecules present in the sub-sample (for example, between 45% and 55% of the number of target template nucleic acid molecules present in the sub-sample).

At its broadest, normalising the number of target template nucleic acid molecules in each of the sub-samples may be viewed as corresponding to controlling the number of target template nucleic acid molecules from each of the sub-samples that is provided in a pooled sample. Thus, normalising the number of target template nucleic acid molecules may comprise measuring the number of target template nucleic acid molecules in each of the sub-samples.

According to certain embodiments, the number of target template nucleic acid molecules in a sub-sample may be measured as described elsewhere herein, particularly in the context of methods for controlling the number of target template nucleic acid molecules in a sample.

In preferred embodiments, normalising the number of target template nucleic acid molecules in each of the sub-samples may comprise labelling target template nucleic acid molecules from different sub-samples with different sample tags. A sample tag is a tag which is used to label a substantial portion or all of the at least one target template nucleic acid molecules in a sample. Labelling target template nucleic acid molecules in different sub-samples with different sample tags may allow template target nucleic acid molecules derived from different sub-samples to be distinguished. Sample tags may therefore be of particular utility in this aspect of the present invention, as their use may allow the number of target template nucleic acid molecules in each of two or more sub-samples to be measured simultaneously. In particular, sample tags may allow the number of target template nucleic acid molecules in each of two or more sub-samples to be measured in a single sample. Preferably, target template nucleic acid molecules may be labelled with a sample tag prior to pooling sub-samples. In a particular embodiment, the present aspect of the invention may therefore comprise preparing a preliminary pool of the sub-samples, each comprising target template nucleic acid molecules labelled with sample tags, and measuring the number of target template nucleic acid molecules labelled with each sample tag in the preliminary pool.

Viewed another way, the present invention provides a method for measuring the number of target template nucleic acid molecules in two or more sub-samples, comprising:

(a) labelling target template nucleic acid molecules from two or more different sub-samples with different sample tags;
(b) pooling the two or more sub-samples to provide a preliminary pool of the sub-samples; and
(c) measuring the number of target template nucleic acid molecules in the preliminary pool which are labelled with each sample tag.

Optionally, two or more preliminary pools may be prepared, for example each comprising sub-samples provided in different amounts or ratios, and/or comprised of different sub-samples (*e.g.* a different combination of sub-samples).

According to certain embodiments, the number of target template nucleic acid molecules labelled with each sample tag in the preliminary pool may be measured using techniques described elsewhere herein for measuring the number of target template nucleic acid molecules in a sample (in particular, in the context of controlling the number of target template nucleic acid molecules in a sample). In this regard, a skilled person will understand that target template nucleic acid molecules from each sample are distinguishable on the basis of the sample tag which they comprise, and thus measuring the number of target template nucleic acid molecules in a preliminary pool which are labelled with any given sample tag may be performed by adapting methods for measuring the total number of target template nucleic acid molecules which are present in a particular sample.

In this regard, according to certain embodiments, a preliminary pool may be diluted prior to or in the course of measuring the number of target template nucleic acid molecules labelled with each sample tag. The dilution may be performed as described elsewhere herein. For example, in certain embodiments, a serial dilution on a preliminary pool may be performed, to provide a serial dilution comprising diluted preliminary pools.

As mentioned elsewhere, two or more different preliminary pools may be prepared. Each preliminary pool may be diluted to a different extent, e.g. according to a different serial dilution.

According to a particularly preferred embodiment, the number of target template nucleic acid molecules labelled with each sample tag in a preliminary pool may be measured by sequencing the labelled (sample tagged) target template nucleic acid molecules in a preliminary pool or in a diluted preliminary pool. Sequencing may be performed according to any convenient method of sequencing, for example those described elsewhere herein. Preferably, sequencing a labelled target template nucleic acid molecules may comprise sequencing the sample tag of a labelled target template nucleic acid molecule.

In particular embodiments, measuring the number of target template nucleic acid molecules labelled with each sample tag in a preliminary pool may comprise an amplification step. Suitable methods for amplifying the labelled target template nucleic acid molecules are known in the art, and amplification may be performed, for example, as described elsewhere herein. In certain embodiments, measuring the number of target template nucleic acid molecules labelled with each sample tag in the preliminary pool may comprise amplifying and then sequencing the target template nucleic acid molecules.

In certain embodiments, the target template nucleic acid molecules in a sub-sample may be amplified, *i.e.* prior to pooling two or more sub-samples to provide a preliminary pooled sample. Amplification may be performed prior to labelling target template nucleic acid molecules in a sub-sample with a sample tag, or in certain preferred embodiments, may be performed simultaneously with labelling target template nucleic acid molecules in a sub-sample with a sample tag (*e.g.* using PCR primers comprising a sample barcode). In further embodiments, target template nucleic acid molecules labelled with a sample tag may be amplified prior to providing a preliminary pooled sample.

According to yet further embodiments, measuring the number of target template nucleic acid molecules labelled with each sample tag in a preliminary pool may comprise amplifying target template nucleic acid molecules labelled with sample tags in the preliminary pool, *i.e.* following pooling two or more sub-samples.

Optionally, two or more amplification steps may be performed, for example a first amplification before or simultaneously with labelling target template nucleic acid molecules in a sub-sample with a sample tag, and a second amplification to amplify the target template nucleic acid molecules labelled with a sample tag (this second amplification may be performed on the sub-sample or on a preliminary pooled sample, as outlined above).

Following amplification, measuring the number of target template nucleic acid molecules labelled with each sample tag in the preliminary pool may comprise sequencing the target template nucleic acid molecules in a preliminary pool or a diluted preliminary pool which are labelled with each sample tag (*i.e.* the sample tag labelled target template nucleic acid molecules). In preferred embodiments, measuring the number of target template nucleic acid molecules labelled with each sample tag in a preliminary pool may, therefore, comprise amplifying and then sequencing the target template nucleic acid molecules in the preliminary pool or a diluted preliminary pool labelled with each sample tag.

Measuring the number of target template nucleic acid molecules labelled with each sample tag in the preliminary pools may comprise a fragmentation step. Preferably, target template nucleic acid molecules in the pooled sample are fragmented, *i.e.* a after the pooled sample is prepared. Fragmentation may be carried out using any suitable technique, including any of the techniques described elsewhere herein.

In particular embodiments, measuring the number of target template nucleic acid molecules labelled with each sample tag may comprise both amplification and fragmentation steps, prior to sequencing the target template nucleic acid molecules in a preliminary pool or diluted preliminary pool. According to preferred embodiments, target nucleic acid molecules in a sub-sample may, therefore, be amplified, fragmented and labelled with a sample tag, prior to pooling two or more sub-samples to provide a preliminary pooled sample and sequencing the target template nucleic acid molecules. Amplification and fragmentation may be performed in any order. In an embodiment, target template nucleic acid molecules in a sub-sample may be amplified and then fragmented, or fragmented and then amplified, prior to labelling with a sample tag. In further embodiments, target template nucleic acid molecules may be amplified, fragmented and labelled simultaneously, *i.e.* in a single step. A particularly preferred method for amplifying, fragmenting and labelling target template nucleic acid molecules in a single step may be carried out using tagmentation and PCR, particularly using PCR primers which comprise a sample tag. Amplified and fragmented target nucleic acid molecules following such a step will thus be labelled with a sample tag, and may be identifiable as deriving from a particular sub-sample once pooled in a preliminary pooled sample e.g. when sequenced.

Measuring the number of target template nucleic acid molecules labelled with each sample tag in the preliminary pools may comprise identifying the number of target template nucleic acid molecules (optionally unique target template nucleic acid molecules) in a preliminary pool (or diluted preliminary pool) with each sample tag (*i.e.* labelled with each sample tag). Preferably, however, measuring the number of target template nucleic acid molecules with each sample tag comprises identifying the number of unique target template nucleic acid sequences in a preliminary pool (or diluted preliminary pool) with each sample tag.

As discussed elsewhere, mutating target template nucleic acid molecules may be particularly beneficial, for example, in identifying whether sequence reads are likely to have originated from the same target template nucleic acid molecule or different target template nucleic acid molecules. Accordingly, this may be beneficial in determining the number of target template nucleic acid molecules in a preliminary pool which originate from a particular sub-sample.

Thus, according to certain embodiments, measuring the number of target template nucleic acid molecules labelled with each sample tag in the preliminary pool (or diluted preliminary pool) may comprise mutating the target template nucleic acid molecules. In certain embodiments, target template nucleic acid molecules in a preliminary pooled sample may be mutated. However, mutating target template nucleic acid molecules may preferably take place in a sub-sample, *i.e.* before two or more samples are pooled to provide a pooled sample. In particularly preferred embodiments, target template nucleic acid molecules may be mutated prior to or simultaneously with, labelling target template nucleic acid molecules with a sample tag. It may be preferred not to mutate sample tag sequences which are used to label target template nucleic acid molecules. Mutating target template nucleic acid molecules may be performed by any convenient means, including any means described elsewhere herein. Thus, in one embodiment mutations may be introduced by using a low bias DNA polymerase. In further embodiments, mutating the target template nucleic acid molecules may comprise amplifying the target template nucleic acid molecules in the presence of a nucleotide analog, for example dPTP.

According to preferred embodiments, measuring the number of target template nucleic acid molecules labelled with each sample tag in the preliminary pools may comprise:

(i) mutating the target template nucleic acid molecules to provide mutated target template nucleic acid molecules;
(ii) sequencing regions of the mutated target template nucleic acid molecules; and
(iii) identifying the number of unique mutated target template nucleic acid molecules with each sample tag based on the number of unique mutated target template nucleic acid molecules labelled with each sample tag.

As outlined in greater detail above, it may not be necessary for a complete sequence for each target template nucleic acid molecule to be obtained in order to quantitate target template nucleic acid molecules, and it may be sufficient simply to sequence an end region of each labelled target template nucleic acid molecule as part of the step of measuring the number of target template nucleic acid molecules in a preliminary pool which are labelled with each sample tag. The user may, therefore, opt to sequence only an end region of each target template nucleic acid molecule. As outlined above, the sample tag will preferably be sequenced.

According to certain representative embodiments, measuring the number of target template nucleic acid molecules may comprise introducing barcodes or a pair of barcodes into the target template nucleic acid molecules to provide barcoded, sample tagged target template nucleic acid molecules. Barcodes suitable for use in such a step, and methods for their introduction into target template nucleic acid molecules are described in greater detail elsewhere herein.

Preferably, barcodes may be introduced into target template nucleic acid molecules prior to pooling the sub-samples, *i.e.* prior to pooling the sub-samples to provide a provisional pooled sample. Barcodes and sample tags may be introduced to target template nucleic acid molecules in any order. For example, in one embodiment, barcodes may be introduced into target template nucleic acid molecules, followed by sample tags. In another embodiment, sample tags may be introduced into target template nucleic acid molecules, followed by barcodes. In yet further embodiments, sample tags and barcode tags may be introduced simultaneously. In any event, in certain embodiments, target template nucleic acid molecules from a sub-sample may be labelled with both sample tags and barcodes. In this regard, it is noted that sample tags are particularly beneficial in identifying a particular target template nucleic acid molecule in a preliminary sample as originating from a particular sub-sample, whilst barcodes may be particularly beneficial in allowing the number of unique target template nucleic acid molecules from each sub-sample to be measured.

Thus, according to particularly preferred embodiments, measuring the number of target template nucleic acid molecules labelled with each sample tag may comprise:

> (i) sequencing regions of the barcoded, sample tagged, target template nucleic acid molecules; and
> (ii) identifying the number of unique barcoded target template nucleic acid molecules with each sample tag based on the number of unique barcode or barcode pair sequences associated with each sample tag.

A sequencing step in measuring the number of target template nucleic acid molecules may be a *"rough"* sequencing step, as discussed elsewhere herein, in that the user may not need precise sequence information in order to be able to measure the number of target template nucleic acid molecules in a sample. Instead, it may be sufficient for sequencing to allow a sample tag, barcode and/or target template nucleic acid molecule to be identified.

In certain representative embodiments, once the number of target template nucleic acid molecules comprising the different sample tags has been measured, the ratio of the number of target template nucleic acid molecules comprising the different sample tags may be calculated. In further representative embodiments, once the number of target template nucleic acid molecules comprising different sample tags has been measured, it may be possible to determine the number of target template nucleic acid molecules (in a preliminary pooled sample) which arise from each sub-sample, and thereby calculate the number of target template nucleic acid molecules which are present in each sub-sample.

Information on the ratio of target template nucleic acid molecules comprising the different sample tags, and/or of the number of target template nucleic acid molecules which arise from each sub-sample, may be used to prepare a pooled sample for use in the methods of the present invention. In particular, such information may be used in a normalisation step, to normalise the number of target template nucleic acid molecules which are provided from each of two or more sub-samples in a pooled sample, thereby to provide target template nucleic acid molecules from each of the sub-samples in a desired ratio in the pooled sample.

It will be seen, therefore, that the present invention provides a method for determining a sequence of at least one target template nucleic acid molecule comprising:

> (a) providing at least one sample comprising the at least one target template nucleic acid molecule;
> (b) sequencing regions of the at least one target template nucleic acid molecule; and
> (c) assembling a sequence of the at least one target template nucleic acid molecule from the sequences of the regions of the at least one target template nucleic acid molecule, wherein the at least one sample is provided by:

>> (i) providing a preliminary pooled sample by pooling two or more of the sub-samples;
>> (ii) measuring the number of target template nucleic acid molecules in the preliminary pooled sample which arise from each of the two or more sub-samples; and
>> (iii) pooling two or more sub-samples;

> wherein the number of target template nucleic acid molecules in the sample from each of the sub-samples is normalised.

As discussed above, normalising the number of target template nucleic acid molecules in a sample provided by pooling two or more sub-samples may comprise providing target template nucleic acid molecules from each of the sub-samples in a desired ratio. According to certain embodiments, the sample formed by pooling two or more sub-samples may be seen to be a re-pooled sample in which the target template nucleic acid molecules in each of the sub-samples are provided in a desired ratio *(i.e.* after providing a preliminary pool and measuring the number of target template nucleic acid molecule in said preliminary pool which arise from each of the two or more sub-samples). Measuring the number of target template nucleic acid molecules in the sub-sample therefore allows the number of target template nucleic acid molecules in the sample from each of the sub-samples to be normalised when re-pooling the sub-samples.

A sample may be provided by pooling two or more sub-samples according to the present aspect of the invention. Thus, 2 or more, preferably 3, 4, 5, 6, 7, 8, 9, 10, 15, 20, 25, 30, 40, 50, 60, 70, 80, 90, 100, 150, 200, 250, 300, 400, 500, 600, 700, 800, 900, 1000, 1500, 2000, 2500, 3000, 4000, 5000 or more sub-samples may be pooled in order to provide a sample (*i.e.* a pooled sample) for use in the methods of the invention. According to certain embodiments, between 2 and 5000, 10 and 1000, or 25 and 150 sub-samples may be pooled.

The term *"pooling two or more sub-samples"* does not require the entirety of a sub-sample to be combined with another sub-sample in order to provide a sample, and preferably instead refers to obtaining an aliquot of each of the sub-samples and combining the aliquots in order to provide a sample. Similarly, reference to introducing barcodes or tags into target template nucleic acid molecules in a sub-sample, or mutating target template nucleic acid molecules in a sub-sample may be understood to mean performing such steps on an aliquot or a portion of a sub-sample.

According to certain particular embodiments, "*pooling two or more sub-samples*" may comprise diluting a sub-sample and combining the diluted sub-samples in order to provide a sample. In further embodiments, this term may comprise obtaining an aliquot of a sample and diluting said aliquot, and combining the diluted aliquots of the sub-samples in order to provide a sample. Diluting a sub-sample (or aliquot) may include a separate dilution step performed prior to pooling the sub-samples (or aliquots) to provide a sample. However, it will be seen that pooling two or more sub-samples (or aliquots) to provide a sample may in effect reduce the concentration of target template nucleic acid molecules from each of the sub-samples which is provided in the sample, and may, therefore, represent a dilution step. The skilled person will be able to determine the extent to which dilution of each sub-sample may be required, including any dilution which may occur as a result of pooling two or more sub-samples (or aliquots).

*Sequencing regions of the at least one target template nucleic acid molecule or the at least one mutated target template nucleic acid molecule*

The method for determining a sequence of at least one target template nucleic acid molecule may comprise a step of sequencing regions of the at least one target template nucleic acid molecule in a first of the pair of samples to provide non-mutated sequence reads and/or a step of sequencing regions of the at least one mutated target template nucleic acid molecule to provide mutated sequence reads.

The sequencing steps may be carried out using any method of sequencing. Examples of possible sequencing methods include Maxam Gilbert Sequencing, Sanger Sequencing, sequencing comprising bridge amplification (such as bridge PCR), or any high throughput sequencing (HTS) method as described in Maxam AM, Gilbert W (February 1977), "A new method for sequencing DNA ", Proc. Natl. Acad. Sci. U. S. A. 74 (2): 560-4, Sanger F, Coulson AR (May 1975), "A rapid method for determining sequences in DNA by primed synthesis with DNA polymerase ", J. Mol. Biol. 94 (3): 441-8; and

Bentley DR, Balasubramanian S, et al. (2008), "Accurate whole human genome sequencing using reversible terminator chemistry", Nature, 456 (7218): 53-59. In a typical embodiment at least one, or preferably both, of the sequencing steps involve bridge amplification. Optionally, the bridge amplification step is carried out using an extension time of greater than 5, greater than 10, greater than 15, or greater than 20 seconds. An example of the use of bridge amplification is in Illumina Genome Analyzer Sequencers.

Optionally, steps (i) of sequencing regions of the at least one target template nucleic acid molecule in a first of the pair of samples to provide non-mutated sequence reads and (ii) of sequencing regions of the at least one mutated target template nucleic acid molecule to provide mutated sequence reads are carried out using the same sequencing method. Optionally steps (i) of sequencing regions of the at least one target template nucleic acid molecule in a first of the pair of samples to provide non-mutated sequence reads and (ii) of sequencing regions of the at least one mutated target template nucleic acid molecule to provide mutated sequence reads are carried out using different sequencing methods.

Optionally, steps (i) of sequencing regions of the at least one target template nucleic acid molecule in a first of the pair of samples to provide non-mutated sequence reads and (ii) of sequencing regions of the at least one mutated target template nucleic acid molecule to provide mutated sequence reads may be carried out using more than one sequencing method. For example, a fraction of the at least one target template nucleic acid molecules in the first of the pair of samples may be sequenced using a first sequencing method, and a fraction of the at least one target template nucleic acid molecules in the first of the pair of samples may be sequenced using a second sequencing method. Similarly, a fraction of the at least one mutated target template nucleic acid molecules may be sequenced using a first sequencing method, and a fraction of the at least one mutated target template nucleic acid molecules may be sequenced using a second sequencing method.

Optionally, steps (i) of sequencing regions of the at least one target template nucleic acid molecule in a first of the pair of samples to provide non-mutated sequence reads and (ii) of sequencing regions of the at least one mutated target template nucleic acid molecule to provide mutated sequence reads are carried out at different times. Alternatively, steps (i) and (ii) may be carried out fairly contemporaneously, such as within 1 year of one another. The first of the pair of samples and the second of the pair of samples need not be taken at the same time as one another. Where the two samples are derived from the same organism, they may be provided at substantially different times, even years apart, and so the two sequencing steps may also be separated by a number of years. Furthermore, even if the first of the pair of samples and the second of the pair of samples were derived from the same original sample, biological samples can be stored for some time and so there is no need for the sequencing steps to take place at the same time.

The mutated sequence reads and/or the non-mutated sequence reads may be single ended or paired-ended sequence reads.

Optionally, the mutated sequence reads and/or the non-mutated sequence reads are greater than 50 bp, greater than 100 bp, greater than 500 bp, less than 200,000 bp, less than 15,000 bp, less than 1,000 bp, between 50 and 200,000 bp, between 50 and 15,000 bp, or between 50 and 1,000 bp. The longer the read length, the easier it will be to use information obtained from analysing the mutated sequence reads to assemble a sequence for at least a portion of at least one target template nucleic acid molecule from the non-mutated sequence reads. For example, if an assembly graph is used, using longer sequence reads will make it easier to identify valid routes through the assembly graph. For example, as described in more detail below, identifying valid routes through the assembly graph may comprise identifying signature k-mers, and greater read length may allow for longer k-mers.

Optionally, the sequencing steps are carried out using a sequencing depth of between 0.1 and 500 reads, between 0.2 and 300 reads, or between 0.5 and 150 reads per nucleotide per at least one target template nucleic acid molecule. The greater the sequencing depth, the greater the accuracy of the sequence that is determined/generated will be, but assembly may be more difficult.

*Introducing mutations into the at least one target template nucleic acid molecule*

The method may comprise a step of introducing mutations into the at least one target template nucleic acid molecule in a second of the pair of samples to provide at least one mutated target template nucleic acid molecule.

The mutations may be substitution mutations, insertion mutations, or deletion mutations. For the purposes of the present invention, the term *"substitution mutation"* should be interpreted to mean that a nucleotide is replaced with a different nucleotide. For example, the conversion of the sequence ATCC to the sequence AGCC introduces a single substitution mutation. For the purposes of the present invention, the term *"insertion mutation"* should be interpreted to mean that at least one nucleotide is added to a sequence. For example, conversion of the sequence ATCC to the sequence ATTCC is an example of an insertion mutation (with an additional T nucleotide being inserted). For the purposes of the present invention, the term *"deletion mutation"* should be interpreted to mean that at least one nucleotide is removed from a sequence. For example, conversion of the sequence ATTCC to ATCC is an example of a deletion mutation (with a T nucleotide being removed). Preferably, the mutations are substitution mutations.

The phrase *"introducing mutations into the at least one target template nucleic acid molecule"* refers to exposing the at least one target template nucleic acid molecule in the second of the pair of samples to conditions in which the at least one target template nucleic acid molecule is mutated. This may be achieved using any suitable method. For example, mutations may be introduced by chemical mutagenesis and/or enzymatic mutagenesis.

Optionally, the step of introducing mutations into the at least one target template nucleic acid molecule mutates between 1% and 50%, between 3% and 25%, between 5% and 20%, or around 8% of the nucleotides of the at least one target template nucleic acid molecule. Optionally, the at least one mutated target template nucleic acid molecule comprises between 1% and 50%, between 3% and 25%, between 5% and 20%, or around 8% mutations.

The user can determine how many mutations are comprised within the at least one mutated target template nucleic acid molecule, and/or the extent to which the step of introducing mutations into the at least one target template nucleic acid molecule mutates the at least one target template nucleic acid molecule by performing the step of introducing mutations on a nucleic acid molecule of known sequence, sequencing the resultant nucleic acid molecule and determining the percentage of the total number of nucleotides that have changed compared to the original sequence.

Optionally, the step of introducing mutations into the at least one target template nucleic acid molecule mutates the at least one target template nucleic acid molecule in a substantially random manner. Optionally, the at least one mutated target template nucleic acid molecule comprises a substantially random mutation pattern.

The at least one mutated target template nucleic acid molecule comprises a substantially random mutation pattern if it contains mutations throughout its length at substantially similar levels. For example, the user can determine whether the at least one mutated target template nucleic acid molecule comprises a substantially random mutation pattern by mutating a test nucleic acid molecule of known sequence to provide a mutated test nucleic acid molecule. The sequence of the mutated test nucleic acid molecule may be compared to the test nucleic acid molecule to determine the positions of each of the mutations. The user may then determine whether the mutations occur throughout the length of the mutated test nucleic acid molecule at substantially similar levels by:

> (i) calculating the distance between each of the mutations;
> (ii) calculating the mean of the distances;
> (iii) sub-sampling the distances without replacement to a smaller number such as 500 or 1000;
> (iv) constructing a simulated set of 500 or 1000 distances from the geometric distribution, with a mean given by the method of moments to match that previously computed on the observed distances; and
> (v) computing a Kolmolgorov-Smirnov on the two distributions.

The at least one mutated target template nucleic acid molecule may be considered to comprise a substantially random mutation pattern if $D < 0.15$, $D < 0.2$, $D < 0.25$, or $D < 0.3$, depending on the length of the non-mutated reads.

Similarly, the step of introducing mutations into the at least one target template nucleic acid molecule mutates the at least one target template nucleic acid molecule in a substantially random manner, if the resultant at least one mutated target template nucleic acid molecule comprises a substantially random mutation pattern. Whether a step of introducing mutations into the at least one target template nucleic acid molecule does mutate the at least one target template nucleic acid molecule in a substantially random manner may be determined by carrying out the step of introducing mutations into the at least one target template nucleic acid molecule on a test nucleic acid molecule of known sequence to provide a mutated test nucleic acid molecule. The user may then sequence the mutated test nucleic acid molecule to identify which mutations have been introduced and determine whether the mutated test nucleic acid molecule comprises a substantially random mutation pattern.

Optionally, the at least one mutated target template nucleic acid molecule comprises an unbiased mutation pattern. Optionally, the step of introducing mutations into the at least one target template nucleic acid molecule introduces mutations in an unbiased manner. The at least one mutated target template nucleic acid molecule comprises an unbiased mutation pattern, if the types of mutations that are introduced are random. If the mutations that are introduced are substitution mutations, then the mutations that are introduced are random if a similar proportion of A (adenosine), T (thymine), C (cytosine) and G (guanine) nucleotides are introduced. By the phrase "*a similar proportion of A (adenosine), T (thymine), C (cytosine) and G (guanine) nucleotides are introduced",* we mean that the number of adenosine, the number of thymine, the number of cytosine and the number of guanine nucleotides that are introduced are within 20% of one another (for example 20 A nucleotides, 18 T nucleotides, 24 C nucleotides and 22 G nucleotides could be introduced).

Whether a step of introducing mutations into the at least one target template nucleic acid molecule does mutate the at least one target template nucleic acid molecule in a unbiased manner may be determined by carrying out the step of introducing mutations into the at least one target template nucleic acid molecule on a test nucleic acid molecule of known sequence to provide a mutated test nucleic acid molecule. The user may then sequence the mutated test nucleic acid molecule to identify which mutations have been introduced and determine whether the mutated test nucleic acid molecule comprises an unbiased mutation pattern.

Usefully, the methods of generating a sequence of at least one target template nucleic acid molecule may be used even when the step of introducing mutations into the at least one target template nucleic acid molecule introduces unevenly distributed mutations. Thus, in one embodiment the at least one mutated target template nucleic acid molecule comprises unevenly distributed mutations. Optionally, the step of introducing mutations into the at least one mutated target template nucleic acid molecule introduces mutations that are unevenly distributed. Mutations are considered to be *"unevenly distributed"* if the mutations are introduced in a biased manner, *i.e.* the number of adenosine, the number of thymine, the number of cytosine, and the number of guanine nucleotides that are introduced are not within 20% of one another. Whether the at least one mutated target template nucleic acid molecule comprises unevenly distributed mutations, or the step of introducing mutations into the at least one target template nucleic acid molecule introduces mutations that are unevenly distributed may be determined in a similar way to that described above for determining whether the step of introducing mutations into the at least one target template nucleic acid molecule introduces mutations in an unbiased manner.

Similarly, the methods of generating a sequence of at least one target template nucleic acid molecule may be used even when the mutated sequence reads and/or the non-mutated sequence reads comprise unevenly distributed sequencing errors. Thus, in one embodiment, the mutated sequence reads and/or the non-mutated sequence reads comprise sequencing errors that are unevenly distributed. Similarly, in one embodiment, the step of sequencing regions of the at least one target template nucleic acid molecule and/or the sequencing regions of the at least one mutated target template nucleic acid molecule introduces sequence errors that are unevenly distributed.

Whether a particular step of sequencing regions of the at least one target template nucleic acid molecule and/or sequencing regions of the at least one mutated target template nucleic acid molecule introduces sequence errors that are unevenly distributed will likely depend on the accuracy of the sequencing instrument and will likely be known to the user. However, the user may investigate whether a step of sequencing regions of the at least one target template nucleic acid molecule and/or the sequencing regions of the at least one mutated target template nucleic acid molecule introduces sequence errors that are unevenly distributed by performing the sequencing method on a nucleic acid molecule of known sequence and comparing the sequence reads produced with those of the original nucleic acid molecule of known sequence. The user may then apply the probability function discussed in Example 6, and determine values for M and E. If the values of the E and the matrix model are unequal or substantially unequal (within 10% of one another), then the step of sequencing regions of the at least one target template nucleic acid molecule introduces sequence errors that are unevenly distributed.

Introducing mutations into the at least one target template nucleic acid molecule via chemical mutagenesis may be achieved by exposing the at least one target template nucleic acid to a chemical mutagen. Suitable chemical mutagens include Mitomycin C (MMC), N-methyl-N-nitrosourea (MNU), nitrous acid (NA), diepoxybutane (DEB), 1, 2, 7, 8,-diepoxyoctane (DEO), ethyl methane sulfonate (EMS), methyl methane sulfonate (MMS), N-methyl-N'-nitro-N-nitrosoguanidine (MNNG), 4-nitroquinoline 1-oxide (4-NQO), 2-methyloxy-6-chloro-9(3-[ethyl-2-chloroethyl]-aminopropylamino)-acridinedihydrochloride( ICR-170), 2-amino purine (2A), bisulphite, and hydroxylamine (HA). For example, when nucleic acid molecules are exposed to bisulphite, the bisulphite deaminates cytosine to form uracil, effectively introducing a C-T substitution mutation.

As noted above, the step of introducing mutations into the at least one target template nucleic acid molecule may be carried out by enzymatic mutagenesis. Optionally, the enzymatic mutagenesis is carried out using a DNA polymerase. For example, some DNA polymerases are error-prone (are low fidelity polymerases) and replicating the at least one target template nucleic acid molecule using an error-prone DNA polymerase will introduce mutations. Taq polymerase is an example of a low fidelity polymerase, and the step of introducing mutations into the at least one target template nucleic acid molecule may be carried out by replicating the at least one target template nucleic acid molecule using Taq polymerase, for example by PCR.

The DNA polymerase may be a low bias DNA polymerase, which are discussed in more detail below.

If the step of introducing mutations into the at least one target template nucleic acid molecule is carried out using a DNA polymerase, the at least one target template nucleic acid molecule may be incubated with the DNA polymerase and suitable primers under conditions suitable for the DNA polymerase to catalyse the generation of at least one mutated target template nucleic acid molecule.

Suitable primers comprise short nucleic acid molecules complementary to regions flanking the at least one target template nucleic acid molecule or to regions flanking nucleic acid molecules that are complementary to the at least one target template nucleic acid molecule. For example, if the at least one target template nucleic acid molecule is part of a chromosome, the primers will be complementary to regions of the chromosome immediately 3' to the 3' end of the at least one target template nucleic acid molecule and immediately 5' to the 5' end of the at least one target template nucleic acid molecule, or the primers will be complementary to regions of the chromosome immediately 3' to the 3' end of a nucleic acid molecule complementary to the at least one target template nucleic acid molecule and immediately 5' to the 5' end of a nucleic acid molecule complementary to the at least one target template nucleic acid molecule.

Suitable conditions include a temperature at which the DNA polymerase can replicate the at least one target template nucleic acid molecule. For example, a temperature of between 40°C and 90°C, between 50°C and 80°C, between 60°C and 70°C, or around 68°C.

The step of introducing mutations into the at least one template nucleic acid molecule may comprise multiple rounds of replication. For example, the step of introducing mutations into the at least one target template nucleic acid molecule preferably comprises:

    i) a round of replicating the at least one target template nucleic acid molecule to provide at least one nucleic acid molecule that is complementary to the at least one target template nucleic acid molecule; and
    ii) a round of replicating the at least one target template nucleic acid molecule to provide replicates of the at least one target template nucleic acid molecule.

Optionally, the step of introducing mutations into the at least one target template nucleic acid molecule comprises at least 2, at least 4, at least 6, at least 8, at least 10, less than 10, less than 8, around 6, between 2 and 8, or between 1 and 7 rounds of replicating the at least one target template nucleic acid molecule. The user may choose to use a low number of rounds of replication to reduce the possibility of introducing amplification bias.

Optionally, the step of introducing mutations into the at least one target template nucleic acid molecule comprises at least 2, at least 4, at least 6, at least 8, at least 10, less than 10, less than 8, around 6, between 2 and 8, or between 1 and 7 rounds of replication at a temperature between 60°C and 80°C.

Optionally, the step of introducing mutations into the at least one target template nucleic acid molecule is carried out using the polymerase chain reaction (PCR). PCR is a process that involves multiple rounds of the following steps for replicating a nucleic acid molecule:

a) melting;
b) annealing; and
c) extension and elongation.

The nucleic acid molecule (such as the at least one target template nucleic acid molecule) is mixed with suitable primers and a polymerase. In the melting step, the nucleic acid molecule is heated to a temperature above 90°C such that a double-stranded nucleic acid molecule will denature (separate into two strands). In the annealing step, the nucleic acid molecule is cooled to a temperature below 75°C, for example between 55°C and 70°C, around 55°C, or around 68°C, to allow the primers to anneal to the nucleic acid molecule. In the extension and elongation steps, the nucleic acid molecule is heated to a temperature greater than 60°C to allow the DNA polymerase to catalyse primer extension, the addition of nucleotides complementary to the template strand.

Optionally, the step of introducing mutations into the at least one target template nucleic acid molecule comprises replicating the at least one target template nucleic acid molecule using Taq polymerase, in error-prone reactions conditions. For example, the step of introducing mutations into the at least one target template nucleic acid molecule may comprise PCR using Taq polymerase in the presence of $Mn^{2+}$, $Mg^{2+}$ or unequal dNTP concentrations (for example an excess of cytosine, guanine, adenine or thymine).

*Obtaining data comprising non-mutated sequence reads and mutated sequence reads*

The methods of the invention may comprise a step of obtaining data comprising non-mutated sequence reads and mutated sequence reads. The non-mutated sequence reads and the mutated sequence reads may be obtained from any source.

Optionally, the non-mutated sequence reads are obtained by sequencing regions of at least one target template nucleic acid molecule in a first of a pair of samples. Optionally, the mutated sequence reads are obtained by introducing mutations into the at least one target template nucleic acid molecule in a second of the pair of samples to provide at least one mutated target template nucleic acid molecule, and sequencing regions of the at least one mutated target template nucleic acid molecule.

Optionally, the non-mutated sequence reads comprise sequences of regions of at least one target template nucleic acid molecule in a first of a pair of samples, the mutated sequence reads comprise sequences of regions of at least one mutated target template nucleic acid molecule in a second of a pair of samples, and the pair of samples were taken from the same original sample or are derived from the same organism.

*Analysing the mutated sequence reads, and using information obtained by analysing the mutated sequence reads to assemble a sequence*

As discussed above, the first sample and the second sample comprise the at least one target template nucleic acid molecule. Thus, the mutation patterns present in the mutated sequence reads may help the user to assemble a sequence for at least a portion of the at least one target template nucleic acid molecule.

As discussed above, assembling a sequence may be difficult if, for example, regions of a sequence are similar to one another or the sequence comprises repeat portions. However, the user may be able to assemble a sequence from non-mutated sequence reads more effectively using information obtained from mutated sequence reads that correspond to the non-mutated sequence reads. For example, mutated sequence reads may be used to identify nodes computed from non-mutated sequence reads that form part of a valid route through the sequence assembly graph.

According to certain embodiments, a sequence may be assembled using information from multiple mutated reads. As described in greater detail below, mutated sequence reads which are likely to have originated from the same mutated target template nucleic acid molecule may be identified. According to certain embodiments, mutated sequence reads may be assembled, and/or a consensus sequence may be generated from multiple mutated sequence reads. In a particular embodiment, a long mutated read may be reconstructed (i.e. a synthetic long mutated read) from multiple partially overlapping mutated reads originating from the same mutated target template nucleic acid molecule to provide information to assemble a sequence. Such a synthetic long read may correspond to an identified path through an unmutated assembly graph as discussed elsewhere herein.

*Preparing an assembly graph*

The step of analysing the mutated sequence reads, and using information obtained from analysing the mutated sequence reads to assemble a sequence for at least a portion of at least one target template nucleic acid molecule from the non-mutated sequence reads may comprise preparing an assembly graph.

For the purpose of the present invention "*an assembly graph*" is a graph comprising nodes computed from non-mutated sequence reads, and routes which may (in the case of valid routes) correspond to portions of at least one target template nucleic acid molecules. For example, the nodes may represent consensus sequences computed from assembled non-mutated sequence reads.

The nodes may be computed from non-mutated sequence reads. However, if some of the at least one target template nucleic acid molecule have not been sequenced correctly, it is possible that insufficient non-mutated sequence reads are available to assemble a complete sequence for an at least one target template nucleic acid molecule. If that is the case, then the nodes may be computed from a combination of non-mutated sequence reads and mutated sequence reads with the mutated sequence reads being used to supplement regions of the assembly graph representing missing non-mutated sequence reads. Optionally, the nodes are computed from non-mutated sequence reads and mutated sequence reads. Using nodes computed from non-mutated sequence reads alone is beneficial, as the non-mutated sequence reads correspond exactly to the original target template nucleic acid molecule. Thus, using an assembly graph that consists of nodes computed from non-mutated sequence reads may avoid artefacts introduced by the mutation steps.

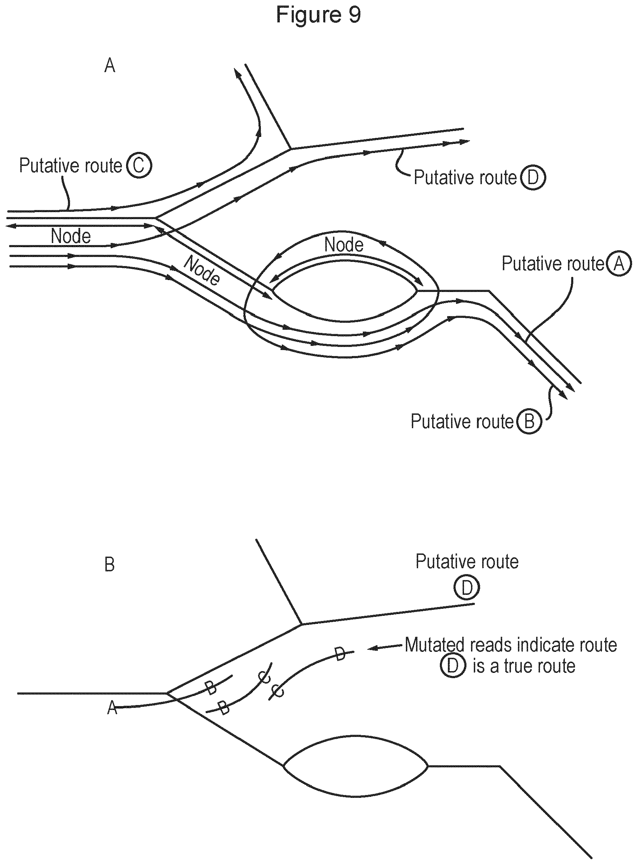

A pictorial representation of a suitable assembly graph is provided in Figure 9, panel A.

Optionally, the nodes of the assembly graph are unitigs. For the purpose of the present invention, the term *"unitig"* is intended to refer to a portion of at least one target template nucleic acid molecule whose sequence can be defined with a high level of confidence. For example, the nodes of the assembly graph may comprise unitigs corresponding to consensus sequences of all or portions of one or more non-mutated sequence reads and/or all or portions of one or more mutated sequence reads. Preferably, the nodes of the assembly graph comprise unitigs corresponding to consensus sequences of all or portions of one or more non-mutated sequence reads.

The assembly graph may be a contig graph, a unitig graph or a weighted graph. For example, the assembly graph may be a de Bruijn graph.

*Identifying nodes that form part of a valid route through the assembly graph*

Using information obtained from analysing the mutated sequence reads to assemble a sequence for at least a portion of at least one target template nucleic acid molecule from the non-mutated sequence reads may comprise identifying nodes computed from non-mutated sequence reads that form part of a valid route through the assembly graph using information obtained by analysing the mutated sequence reads. Each valid route through the assembly graph may represent the sequence of a portion of at least one target template nucleic acid molecule. If the assembly graph comprises numerous putative routes from node to node, information obtained by analysing the mutated sequence reads can be used to obtain the order of the nodes. In further embodiments, information obtained by analysing the mutated sequence reads can be used to determine the number of copies of a given sequence in a genome.

Optionally, analysing the mutated sequence reads comprises identifying mutated sequence reads that are likely to have originated from the same at least one mutated target template nucleic acid molecule. The methods of the invention may result in the provision of multiple mutated sequence reads that comprise a mutated sequence corresponding to the same region, *i.e.* groups of mutated sequence reads that correspond to the same region. Some of the mutated sequence reads in the group may overlap and some of the mutated sequence reads in the group may be repeats. When the group of mutated sequence reads is mapped to the assembly graph, they may be used to identify valid routes through the assembly graph, as depicted in Figure 9B, as they may link nodes computed from non-mutated sequence reads.

Thus, optionally, analysing the mutated sequence reads comprises identifying mutated sequence reads that are likely to have originated from the same at least one mutated target template nucleic acid molecule. Optionally, identifying nodes that form part of a valid route through the assembly graph using information obtained by analysing the mutated sequence reads may comprise:

(i) computing nodes from non-mutated sequence reads;
(ii) mapping the mutated sequence reads to the assembly graph;
(iii) identifying mutated sequence reads that are likely to have originated from the same at least one mutated target template nucleic acid molecule; and
(iv) identifying nodes that are linked by mutated sequence reads that are likely to have originated from the same at least one mutated target template nucleic acid molecule,

wherein nodes that are linked by mutated sequence reads are likely to have originated from the same at least one mutated target template nucleic acid molecule and form part of a valid route through the assembly graph.

Optionally, mutated sequence reads that are likely to have originated from the same mutated target template nucleic acid molecule are assigned into groups.

*Identifying mutated sequence reads that are likely to have originated from the same mutated target template nucleic acid molecule*

As discussed, analysing the mutated sequence reads may comprise identifying mutated sequence reads that are likely to have originated from the same at least one mutated target template nucleic acid molecule.

Optionally, mutated sequence reads are likely to have originated from the same mutated target template nucleic acid molecule if they share common mutation patterns. Optionally, mutated sequence reads that share common mutation patterns comprise common signature k-mers or common signature mutations. Preferably, mutated sequence reads that share common mutation patterns comprise at least 1, at least 2, at least 3, at least 4, at least 5, or at least k common signature k-mers and/or common signature mutations.

Identifying mutated sequence reads that are likely to have originated from the same at least one mutated target template nucleic acid molecule may be of particular utility when a sample is provided by pooling two or more sub-samples. In certain embodiments, such a step may be used when determining the sequence of at least one target template nucleic acid molecule in samples which are provided by pooling two or more sub-samples. More particularly, such a step may be used when determining the sequence of at least one target template nucleic acid molecule from each of the two or more sub-samples which are pooled to provide the sample. Such a step may also be of particular utility when measuring the number of target template nucleic acid molecules in the sample which are from each of two or more sub-samples when target template nucleic acid molecules in the sub-samples have mutated.

*Signature k-mers or signature mutations*

Mutated sequence reads that share common mutation patterns may comprise common signature k-mers and/or common signature mutations. Preferably, mutated sequence reads that share common mutation patterns comprise at least 1, at least 2, at least 3, at least 4, at least 5, or at least k common signature k-mers and/or common signature mutations.

In the context of the invention, a *"k-mer"* represents a nucleic acid sequence of length $k$, that is contained within a sequence read. A *"signature k-mer"* may be a k-mer that does not appear in the non-mutated sequence reads, but appears at least twice in the mutated sequence reads. In an embodiment, a signature k-mer is a k-mer that appears at least n times more frequently in the mutated sequence reads that in the non-mutated sequence reads, wherein n is any integer for example 2, 3, 4 or 5. Optionally a signature k-mer is a k-mer that appears at least two times, at least three times, at least four times, at least five times, or at least ten times in the mutated sequence reads. Thus, the user may determine whether mutated sequence reads comprise common signature k-mers by partitioning the mutated sequence reads into k-mers and partitioning the non-mutated sequence reads into k-mers. The user may then compare the mutated sequence read k-mers and the non-mutated sequence read k-mers, and determine which k-mers appear in the mutated sequence read k-mers and not in the non-mutated sequence read k-mers (or which k-mers appear more frequently in the mutated sequence read k-mers than in the non-mutated read k-mers). The user may then assess the k-mers which appear in the mutated sequence read k-mers and not (or less frequently) in the non-mutated sequence read k-mers and count them. Any k-mers which appear at least twice, at least three times, at least four times, at least five times, or at least ten times in the mutated sequence read k-mers and not in the non-mutated sequence read k-mers are signature k-mers. Any k-mers that appear less than $k,$ less than 5, less than 4, less than 3, or once in the mutated sequence read k-mers and not (or less frequently) in the non-mutated sequence read k-mers may be a result of a sequencing error and so should be disregarded.

The value of $k$ can be selected by the user, and can be any value. Optionally, the value of k is at least 5, at least 10, at least 15, less than 100, less than 50, less than 25, between 5 and 100, between 10 and 50, or between 15 and 25. Generally, the user will select a value of k which is as long as possible, whilst ensuring that the fraction of k-mers in a read that contain one or more sequencing errors low. Preferably, the proportion of k-mers in a read that contains sequencing errors is less than 50%, less than 40%, less than 30%, between 0% and 50%, between 0% and 40%, or between 0% and 30%.

A *"signature mutation"* may be a nucleotide that appears at least twice in the mutated sequence reads and does not appear in a corresponding position in the non-mutated sequence reads. In an embodiment, a signature mutation is a mutation that appears at least n times more frequently in the mutated sequence reads that in the non-mutated sequence reads, wherein n is any integer for example 2, 3, 4 or 5. Optionally, the signature mutation is a mutation that appears at least two times, at least three times, at least four times, at least five times or at least ten times in the mutated reads and does not appear (or appears less frequently) in a corresponding position in a non-mutated read.

Optionally, the signature mutations are co-occurring mutations. *"Co-occurring mutations"* are two or more signature mutations that occur in the same mutated sequence read. For example, if a mutated sequence read contains three signature mutations then it contains three co-occurring mutation pairs or one co-occurring mutation 3-tuple. If it contains four signature mutations then it contains six co-occurring mutation pairs, four co-occurring mutation 3-tuples and one co-occurring mutation 4-tuple.

Optionally, signature mutations may be disregarded if they do not meet certain criteria suggesting that the signature mutations identified are spurious or do not help to assemble a sequence for at least a portion of at least one target template nucleic acid molecule.

Optionally, signature mutations are disregarded if at least 1, at least 2, at least 3, or at least 5 nucleotides at corresponding positions in mutated sequence reads that share the signature mutations differ from one another. For example, if two mutated sequence reads overlap, and share common signature mutations in the overlap, the nucleotides within the overlap should be identical. If they have a low level of identity, then an error has likely occurred and so the mutated sequence reads should be disregarded. One nucleotide difference, for example, may be tolerated as this may be a simple sequencing error.

Optionally, signature mutations are disregarded if they are mutations that are unexpected. By the phrase *"mutations that are unexpected"*, we mean mutations that are unlikely to occur using a particular step of introducing mutations into the at least one target template nucleic acid molecule. For example, if the step of introducing mutations into the at least one target template nucleic acid molecule is carried out using a chemical mutagen which only introduces substitutions of guanine for adenine, any substitutions of cytosine are unexpected and mutated sequence reads containing such mutations should be disregarded.

Optionally, the step of identifying mutated sequence reads that are likely to have originated from the same at least one mutated target template nucleic acid molecule comprises identifying mutated sequence reads corresponding to a specific region of the at least one target template nucleic acid molecule. For example, the user may only be interested in identifying mutated sequence reads that comprise signature mutations in regions of overlap with other mutated sequence reads, and signature mutations that occur in other regions may be disregarded.

In general, mutated sequence reads whose sets of signature mutations have a larger intersection and smaller symmetric differences are more likely to have originated from the same at least one mutated target template nucleic acid molecule. For two mutated sequence reads A and B with signature mutations SM(A) and SM(B) then A and B can be assumed to originate from the same at least one mutated target template nucleic acid molecule if:

$$\text{intersection } ( \text{SM(A), SM(B)} ) >= C$$

and

$$\text{symmetric\_difference } ( \text{SM(A), SM(B)} ) < \text{intersection } ( \text{SM(A), SM(B)} )$$

where C is greater than 4, greater than 5, less than 20, or less than 10 and SM(X) is a set of signature mutations for mutated sequence read X which may be a subset of the signature mutations for X.

Optionally, sets of co-occurring mutations may be used in place of signature mutations in the following equation.

$$\text{intersection } ( \text{SM(A), SM(B)} ) >= C$$

and

$$\text{symmetric\_difference } ( \text{SM(A), SM(B)} ) < C2* \text{ intersection } ( \text{SM(A), SM(B)} )$$

where C2 is less than 3, less than 2, or less than or equal to 1.5 and SM(X) is a set of coocurring mutations for mutated sequence read X which may be a subset of the signature mutations for X.

Mutated sequence reads that share common signature k-mers or common signature mutations may be grouped together. Preferably mutated sequence reads are grouped together if they share at least 1, at least 2, at least 3, at least 4, at least 5, or at least k common signature k-mers and/or common signature mutations. In such embodiments "*k*" is the length of the k-mer used.

*Determining the probability that two mutated sequence reads originated from the same mutated target template nucleic acid molecule*

Mutated sequence reads that are likely to have originated from the same mutated target template nucleic acid molecule may be identified by calculating the following odds ratio:

probability that the mutated sequence reads originated from the same mutated target template nucleic acid molecule: probability that the mutated sequence reads did not originate from the same mutated target template nucleic acid molecule.

If the odds ratio exceeds a threshold, then the mutated sequence reads are likely to have originated from the same at least one mutated target template nucleic acid molecule. Similarly, if the odds ratio is higher for a first mutated sequence read and a second mutated sequence read compared to the first mutated sequence read and other mutated sequence reads that map to the same region of the assembly graph, then the first mutated sequence read is likely to have originated from the same at least one target template nucleic acid molecule as the second mutated sequence read.

The threshold applied may be at any level. Indeed, the user will determine the threshold for any given sequencing method depending on their requirements.

For example, the user may determine what level of stringency is required. If the user is using the method to determine or generate a sequence for at least one target template nucleic acid for which accuracy is not important, then the threshold that is chosen may be considerably lower than if the user is using the method to generate or determine a sequence for at least one target template nucleic acid for which accuracy is important. If the user is using the method to determine or generate sequences for target template nucleic acids in a sample, in order to, for example, determine whether the sample comprises multiple bacterial strains or just one, a lower level of accuracy may be required than if the user is using the method to determine or generate a sequence of a specific variant gene in order to determine how it differs from the native gene. Thus, the threshold may be varied (determined) based on the stringency required.

Similarly, the user may alter the threshold according to the mutation rate used in the step of introducing mutations into the at least one target template nucleic acid molecule. If the mutation rate is higher, then it is easier to determine whether two mutated sequence reads originate from the same mutated target template nucleic acid molecule, and so a higher probability threshold may be used.

Similarly, the user may alter the threshold according to the size of the at least one target template nucleic acid molecule. The larger the size of the at least one target template nucleic acid molecule, the more difficult it is to sequence the entire length without any sequencing errors, and so a user may wish to use a higher threshold for a longer at least one target template nucleic acid molecule.

Similarly, the user may alter the threshold according to time constraints and resource constraints. If these constraints are higher, the user may be satisfied with a lower threshold providing a less accurate sequence.

In addition, the user may alter the threshold according to the error rate of the step of sequencing regions of the at least one mutated target template to provide mutated sequence reads. If the error rate is high, then the user may set a higher threshold than if the error rate is low. That is because, if the error rate is high, the data may be less informative about whether two mutated sequence reads originate from the same mutated target template nucleic acid molecule, especially if the errors are biased in a manner that is similar to the introduced mutations.

Optionally, identifying mutated sequence reads that are likely to have originated from the same mutated target template nucleic acid molecule comprises using a probability function based on the following parameters:

    a. a matrix (N) of nucleotides in each position of the mutated sequence reads and the assembly graph;
    b. a probability (M) that a given nucleotide (i) was mutated to read nucleotide (j);
    c. a probability (E) that a given nucleotide (i) was read erroneously to read nucleotide (j) conditioned on the nucleotide having been read erroneously; and
    d. a probability (Q) that a nucleotide in position Y was read erroneously.

The probability function may be used to determine the odds ratio:
probability that the mutated sequence reads originated from the same mutated target template nucleic acid molecule: probability that the mutated sequence reads did not originate from the same mutated target template nucleic acid molecule.

Optionally, the value of Q is obtained by performing a statistical analysis on the mutated and non-mutated sequence reads, or is obtained based on prior knowledge of the accuracy of the sequencing method. For example, Q is dependent on the accurate of the sequencing method that is used. Thus, the user can determine a value for Q by sequencing a nucleic acid molecule of known sequence, and determining the number of nucleotides that are read erroneously on average. Alternatively, the user could select a sub-group of the mutated and non-mutated sequence reads and compare these. The differences between the mutated and the non-mutated sequence reads will either be due to sequencing error or the introduction of mutations. The user could use statistical analysis to approximate the number of differences that are due to sequencing error.

Optionally, the value of M and E are estimated based on a statistical analysis carried out on a subset of the mutated sequence reads and non-mutated sequence reads, wherein the subset includes mutated sequence reads and non-mutated sequence reads that are selected as they map to the same region of the reference assembly graph. An example of how to determine M and E is provided in Example 6. In short, the user may perform a statistical analysis on the subset of the mutated sequence reads and non-mutated sequence reads to obtain the best fit values for M and E (by unsupervised learning). Since unsupervised learning can be a computationally expensive process, it is advantageous to carry out this step on a subset of the mutated sequence reads and non-mutated sequence reads, and then apply the values of M and E to the complete set of mutated sequence reads and non-mutated sequence reads afterwards.

Optionally, the statistical analysis is carried out using Bayesian inference, a Monte Carlo method such as Hamiltonian Monte Carlo, variational inference, or a maximum likelihood analog of Bayesian inference.

Optionally, identifying mutated sequence reads that are likely to have originated from the same mutated target template nucleic acid molecule comprises using machine learning or neural nets; for example as described in detail in Russell & Norvig *"Artificial Intelligence, a modern approach"*.

*Pre-clustering*

Optionally, the method comprises a pre-clustering step. For example, the user may make an initial calculation to assign mutated sequence reads into groups, wherein each member of the same group has a reasonable likelihood of having originated from the same at least one mutated target template nucleic acid molecule. The mutated sequence reads in each groups may map to a common location on the assembly graph and/or share a common mutation pattern. Two mutated sequence reads in the group map to a common location on the assembly graph if they map to the same region, or if they overlap in the assembly graph. The likelihood threshold applied in the pre-clustering step may be lower than that applied in a step of identifying mutated sequence reads that are likely to have originated from the same at least one mutated target template nucleic acid molecule, *i.e.* the pre-clustering step may be a lower stringency step than the step of identifying mutated sequence reads that are likely to have originated from the same at least one mutated target template nucleic acid molecule.

Optionally, identifying mutated sequence reads that are likely to have originated from the same mutated target template nucleic acid molecule is constrained by the results of a pre-clustering step. For example, the user may apply a lower stringency pre-clustering step to group mutated sequence reads that map to a common region of the assembly graph and that have a reasonable likelihood of having originated from the same at least one mutated target template nucleic acid molecule. The user may then apply a higher stringency step of identifying mutated sequence reads that are likely to have originated from the same at least one mutated target template nucleic acid molecule to each of the members of a group to see which of those are, indeed, likely to have originated from the same at least one mutated target template nucleic acid molecule. The advantage of using a pre-clustering step is that the higher stringency step will use a larger amount of processing power than the lower stringency step, and in this example the higher stringency step need only be applied to mutated sequence reads assigned to the same group by the lower stringency step, thereby reducing the overall processing power required.

Optionally, the pre-clustering step comprises Markov clustering or Louvain clustering(https://micans.org/mcl/ and https://arxiv.org/abs/0803.0476).

Optionally, the pre-clustering step is carried out by assigning mutated sequence reads into the same group that share at least 1, at least 2, at least 3, at least 5, or at least k signature k-mers or at least 1, at least 2, at least 3, or at least 5 signature mutations, as described above. Optionally, mutated sequence reads are reasonably likely to have originated from the same at least one mutated target template nucleic acid molecule if they share common mutation patterns and mutated sequence reads that share common mutation patterns are mutated sequence reads that comprise at least 1, at least 2, at least 3, at least 5, or at least k common signature k-mers or common signature mutations.

Optionally, as described under the heading *"signature k-mers or signature mutations"* signature k-mers are k-mers that do not appear (or appear less frequently) in the non-mutated sequence reads, but appear at least twice (optionally at least three times, at least four times, at least five times, or at least ten times) in the mutated sequence reads. Optionally, signature mutations are nucleotides that appear at least twice (optionally at least three times, at least four times, at least five times, or at least ten times) in the mutated sequence reads and do not appear (or appear less frequently) in a corresponding position in the non-mutated sequence reads.

*Disregarding putative routes through the assembly graph*

In some embodiments of the invention, the step of identifying nodes that form part of a valid route through the assembly graph comprises disregarding putative routes through the assembly graph.

For example, putative routes through the assembly graph may be disregarded if:

(i) they have ends that do not match those present in a library of sequences of ends;
(ii) they are a result of template collision;
(iii) they are longer or shorter than expected; and/or
(iv) they have atypical depth of coverage.

The term *"template collision"* refers to the situation where two putative routes through the assembly graph are identified that correspond to one or more of the same mutated sequence reads or of mutated sequence reads that have the same mutation patterns (the two putative routes have collided).

*Disregarding putative routes through the assembly graph that have ends that do not match*

The method may comprise preparing a library of sequences of pairs of ends of the at least one mutated target template nucleic acid molecules. For example, the library may specify that a first at least one target template nucleic acid molecule has end sequences of A and B, and a second at least one target template nucleic acid molecule has end sequences of C and D. A library could be prepared by carrying out paired end sequencing of the at least one target template nucleic acid molecule. Optionally, the method comprises sequencing the ends of the at least one target template nucleic acid molecule using mate-pair sequencing.

In such embodiments, identifying nodes that form part of a valid route through the assembly graph comprises disregarding putative routes having mismatched ends, *i.e.* the sequences of the ends of the putative routes do not correspond to one of the pairs in the library. For example, if the library specifies that a first at least one target template nucleic acid molecule has end sequences of A and B, and a second at least one target template nucleic acid molecule has end sequences of C and D, then a putative route that pairs end A with end D will be a false route and should be disregarded.

In order to disregard putative routes having mismatched ends, the user may map the sequences of the ends of the at least one target template nucleic acid molecule to an assembly graph. Optionally, the user may also wish to map the sequences of the ends of the at least one target template nucleic acid molecule to an assembly graph to identify where each at least one target template nucleic acid molecules starts and ends on the assembly graph, in order to assist the user in assembling a sequence for at least a portion of at least one target template nucleic acid molecule from the non-mutated sequence reads.

Optionally, the at least one target template nucleic acid molecule comprises at least one barcode. Optionally, the at least one target template nucleic acid molecule comprises a barcode at each end. By the term *"at each end"* is meant a barcode is present substantially close to both ends of the at least one target template nucleic acid molecule, for example within 50 base pairs, within 25 base pairs, or within 10 base pairs of the end of the at least one target template nucleic acid molecule. If the at least one target template nucleic acid molecule comprises at least one barcode, then it is easier for the user to determine whether a putative route has mismatched ends. That is because the end sequences are more distinctive, and it is easier to determine whether sequences of two ends that look mismatched are indeed mismatched, or whether a sequencing error has been introduced into the sequence of one of the ends.

*Barcodes and sample tags*

For the purposes of the present invention, a barcode (also referred to as a *"unique molecular tag"* or a *"unique molecular identifier"* herein) is a degenerate or randomly generated sequence of nucleotides. The target template nucleic acid molecules may comprise 1, 2 or 3 barcodes. According to certain embodiments, each barcode may have a different sequence from every other barcode that is generated. In other embodiments, however, two or more barcode sequences may be the same, *i.e.* a barcode sequence may occur more than once. For example, at least 90% of the barcode sequences may be different to the sequences of every other barcode sequence. It is simply required that the barcodes are suitably degenerate that each target template nucleic acid molecule comprises a barcode of a unique or substantially unique sequence compared to each other target template nucleic acid molecule in the pair of samples. Labelling (or tagging) target template nucleic acid molecules with barcodes therefore allows target template nucleic acid molecules to be differentiated from one another, thereby to facilitate the methods discussed elsewhere herein. A barcode may, therefore, be considered to be a unique molecular tag (UMT). The barcodes may be 5, 6, 7, 8, between 5 and 25, between 6 and 20, or more nucleotides in length.

Optionally, as discussed above, the at least one target template nucleic acid molecules in different pairs of samples may be labelled with different sample tags.

For the purposes of the present invention, a sample tag is a tag which is used to label a substantial portion of the at least one target template nucleic acid molecules in a sample. Different sample tags may be used in further samples, in order to distinguish which at least one target template nucleic acid molecule was derived from which sample. The sample tag is a known sequence of nucleotides. The sample tag may be 5, 6, 7, 8, between 5 and 25, between 6 and 20, or more nucleotides in length.

Optionally, the methods of the invention comprise a step of introducing at least one barcode or a sample tag into the at least one target template nucleic acid molecule. The at least one barcode or sample tag may be introduced using any suitable method including PCR, tagmentation and physical shearing or restriction digestion of target nucleic acids combined with subsequent adapter ligation (optionally sticky-end ligation). For example, PCR can be carried out on the at least one target template nucleic acid molecule using a first set of primers capable of hybridising to the at least one target nucleic acid molecule. The at least one barcode or sample tag may be introduced into each of the at least one target template nucleic acid molecule by PCR using primers comprising a portion (a 5' end portion) comprising a barcode, a sample tag and/or an adapter, and a portion (a 3' end portion) having a sequence that is capable of hybridising to (optionally complementary to) the at least one target nucleic acid molecule. Such primers will hybridise to an at least one target template nucleic acid molecule, PCR primer extension will then provide at least one target template acid molecule which comprises a barcode, and/or a sample tag. A further cycle of PCR with these primers can be used to add a further barcode or sample tag, optionally to the other end of the at least one target template nucleic acid molecule. The primers may be degenerate, *i.e.* the 3' end portion of the primers may be similar but not identical to one another.

The at least one barcode or sample tag may be introduced using tagmentation. The at least one barcode or sample tag can be introduced using direct tagmentation, or by introducing a defined sequence by tagmentation followed by two cycles of PCR using primers that comprise a portion capable of hybridising to the defined sequence, and a portion comprising a barcode, a sample tag and/or an adapter. The at least one barcode or sample tag can be introduced by restriction digestion of the original at least one target template nucleic acid molecule followed by ligation of nucleic acids comprising the barcode and/or sample tag. The restriction digestion of the original at least one nucleic acid molecule should be performed such that the digestion results in a nucleic acid molecule comprising the region to be sequenced (the at least one target template nucleic acid molecule). The at least one barcode or sample tag may be introduced by shearing the at least one target template nucleic acid molecule, followed by end repair, A-tailing and then ligation of nucleic acids comprising the barcode and/or the sample tag.

*Disregarding putative routes that are a result of template collision*

The method may comprise disregarding putative routes that are a result of template collision. As discussed, above, the term *"template collision"* refers to the situation where two putative routes through the assembly graph are identified that correspond to one or more of the same mutated sequence reads or of mutated sequence reads that have the same mutation patterns (the two putative routes have collided). Since each valid route should comprise a unique set of mutated sequence reads, it is likely that at least one of the two putative routes that have collided is false. For these reasons, disregarding putative routes that are a result of template collision may reduce the number of false routes that are identified.

Similarly, it is possible that two different at least one mutated target template nucleic acid molecules may have similar or the same mutation patterns as they either did not receive many mutations during the step of introducing mutations into the at least one target template nucleic acid molecule, or the mutations that they received were the same by chance. If this is the case, again template collision will be seen. In such circumstances, it is virtually impossible to use information obtained by analysing these poorly mutated at least one mutated target template nucleic acid molecules to assemble a sequence for at least a portion of at least one target template nucleic acid molecule from the non-mutated sequence reads, and putative routes that correspond to nodes computed from non-mutated sequence reads that originated from such poorly mutated at least one mutated target template nucleic acid molecules should be disregarded.

*Disregarding putative routes that are longer or shorter than expected*

The at least one target template nucleic acid molecule may be a known or predictable length.

The length may be defined by analysing the length of the at least one target template nucleic acid molecule in a laboratory setting. For example, the user could use gel electrophesis to isolate a sample of at least one target template nucleic acid molecule, and use that sample in the methods of the invention. In such cases, all of the at least one target template nucleic acid molecule whose sequence is to be determined or generated will be within a known size range. For example, the user could extract a band from a gel that has been exposed to gel electrophoresis corresponding to an at least one target template nucleic molecule of 6,000-14,000 or 18,000-12,000 bp in length. Alternatively, or in addition, the size of the at least one target template nucleic acid molecule may be quantitated using a variety of methods for determining the size of a nucleic acid molecule, including gel electrophoresis. For example, the user may use an instrument such as an Agilent Bioanalzyer or a FemtoPulse machine.

When the size of the at least one target template nucleic acid molecule is known or predictable, putative routes that are longer and shorter than the defined length are likely to be incorrect and should be disregarded.

*Disregarding putative routes that have atypical depth of coverage*

The methods of the invention may comprise a step of amplifying the at least one mutated target template nucleic acid molecule, i.e. replicating the at least one mutated target nucleic acid molecule to provide copies of the at least one mutated target template nucleic acid molecule. For example, the method may comprise amplifying the at least one mutated target template nucleic acid molecule using PCR. Amplification will likely result in some of the at least mutated target template nucleic acid molecules being replicated a greater number of times than others. If some of the at least one mutated target template nucleic acid molecules are amplified to a greater extent (have higher depth of coverage) than other at least one mutated target template nucleic acid molecules, then a greater number of mutated sequence reads will be associated with the putative route that corresponds to those at least one mutated target template nucleic acid molecule compared to others. Similarly, one would expect that the depth of coverage would be consistent across the length of the at least one template nucleic acid molecule. Thus, one would expect that different portions of a valid route would have similar numbers of mutated sequence reads associated with them (similar depth of coverage). If a putative route comprises a portion that has low depth of coverage and a portion that has high depth of coverage, those two portions likely do not correspond to the same valid route, the putative route is false and should be disregarded.

*Assembly of a sequence for at least a portion of at least one target template nucleic acid molecule*

Optionally, a sequence is assembled for at least a portion of at least one target template nucleic acid molecule from non-mutated sequence reads that form part of a valid route through the assembly graph.

Optionally, the method does not comprise generating a consensus sequence from mutated sequence reads. Optionally, the method does not comprise a step of assembling a sequence of the at least one mutated target template nucleic acid molecule, or a large portion of the at least one mutated target template nucleic acid molecule.

A *"consensus sequence"* is intended to refer to a sequence that comprises probable nucleotides at each position defined by analysing a group of sequence reads that align to one another, for example the most frequently occurring nucleotides at each position in a group of sequence reads that align to one another.

The methods comprise a step of assembling a sequence for at least a portion of at least one target template nucleic acid molecule from nodes that form a valid route through the assembly graph. Optionally, the step of assembling a sequence for at least a portion of at least one target template nucleic acid molecule comprises assembling a sequence for at least a portion of at least one target template nucleic acid molecule from nodes that form part of a valid route through the assembly graph.

Optionally, assembling a sequence for at least a portion of at least one target template nucleic acid molecule comprises identifying *"end walls"*. End walls are locations on the assembly graph that correspond to multiple *"end + int reads"* (end reads correspond to one of the ends of at least one target template nucleic acid molecule and int reads correspond to an internal sequence (*i.e.* a sequence which is not at the end of the at least one target template nucleic acid molecule)). End reads may be generated using, for example, paired-end sequencing methods. Optionally, an end wall is identified as a location on the assembly graph to which at least 5 end reads map. Optionally, an end wall is identified as a location on the assembly graph to which between 2 and 4 end reads map and to which at least 5 end or int reads map. Optionally, assembling a sequence for at least a portion of at least one target template nucleic acid molecule comprises assembling a sequence for at least a portion of at least one target template nucleic acid molecule from nodes that form part of a valid route through the assembly graph, and the assembling step starts at an end wall.

As discussed above, valid routes through the assembly graph may comprise linked nodes. When a series of linked nodes form a single path through the assembly graph (e.g. wherein the nodes of said graph may be unitigs), consisting of one or more nodes, the sequence covered by the linked nodes represents at least a portion of at least one target template nucleic acid molecule. These portions can then be assembled by concatenating the nodes using standard techniques such as canu (https://github.com/marbl/canu) or miniasm (https://github.com/lh3/miniasm). For example, the user may prepare a consensus sequence from the node that form a valid route.

Optionally, the assembled sequence comprises nodes computed from predominantly non-mutated sequence reads. An assembled sequence will comprise nodes computed from predominantly non-mutated sequence reads, if the sequence was assembled from nodes computed from more than 50% non-mutated sequence reads. It is advantageous to assemble the sequence from nodes computed from predominantly non-mutated sequence reads, as the assembled sequence is more likely to exactly correspond to the original at least one target template nucleic acid molecule sequence. However, if it is not possible to map non-mutated sequence reads to a portion of a putative route through the assembly graph, the sequence of the missing portion could be assembled from nodes computed from mutated sequence reads. Preferably, the assembled sequence comprises nodes computed from greater than 50%, greater than 60%, greater than 70%, greater than 80%, greater than 90%, greater than 98%, between 50% and 100%, between 60% and 100%, between 70% and 100%, or between 80% and 100% non-mutated sequence reads.

*Amplifying the at least one target template nucleic acid molecule*

The methods may comprise a step of amplifying the at least one target template nucleic acid molecule in the first of the pair of samples prior to the step of sequencing regions of the at least one target template nucleic acid molecule. The methods may comprise a step of amplifying the at least one target template nucleic acid molecule in the second of the pair of samples prior to the step of sequencing regions of the at least one mutated target template nucleic acid molecule.

Suitable methods for amplifying the at least one target template nucleic acid molecule are known in the art. For example, PCR is commonly used. PCR is described in more detail above under the heading *"introducing mutations into the at least one target template nucleic acid molecule "*.

*Fragmenting the at least one target template nucleic acid molecule*

The methods may comprise a step of fragmenting the at least one target template nucleic acid molecule in a first of the pair of samples prior to the step of sequencing regions of the at least one target template nucleic acid molecule. Optionally, the methods comprise a step of fragmenting the at least one target template nucleic acid molecule in a second of the pair of samples prior to the step of sequencing regions of the at least one mutated target template nucleic acid molecule.

The at least one target template nucleic acid molecule may be fragmented using any suitable technique. For example, fragmentation can be carried out using restriction digestion or using PCR with primers complementary to at least one internal region of the at least one mutated target nucleic acid molecule. Preferably, fragmentation is carried out using a technique that produces arbitrary fragments. The term *"arbitrary fragment"* refers to a randomly generated fragment, for example a fragment generated by tagmentation. Fragments generated using restriction enzymes are not *"arbitrary"* as restriction digestion occurs at specific DNA sequences defined by the restriction enzyme that is used. Even more preferably, fragmentation is carried out by tagmentation. If fragmentation is carried out by tagmentation, the tagmentation reaction optionally introduces an adapter region into the at least one mutated target nucleic acid molecule. This adapter region is a short DNA sequence which may encode, for example, adapters to allow the at least one mutated target nucleic acid molecule to be sequenced using Illumina technology.

*Low bias DNA polymerase*

As discussed above, mutations may be introduced using a low bias DNA polymerase. A low bias DNA polymerase may introduce mutations uniformly at random, and this can be beneficial in the methods of the invention as, if the mutations are introduced in a manner that is uniformly random, then the likelihood that any give portion of a template nucleic acid molecule would have a unique mutation pattern is higher. As set out above, unique mutation patterns can be useful in identifying valid routes through the assembly graph.

In addition, methods using DNA polymerases having high template amplification bias may be limited. DNA polymerases having high template amplification bias will replicate and/or mutate some target template nucleic acid molecules better than others, and so a sequencing method that uses such a high bias DNA polymerase may not be able to sequence some target template nucleic acid molecules well.

The low bias DNA polymerase may have low template amplification bias and/or low mutation bias.

*Low mutation bias*

A low bias DNA polymerase that exhibits low mutation bias is a DNA polymerase that is able to mutate adenine and thymine, adenine and guanine, adenine and cytosine, thymine and guanine, thymine and cytosine, or guanine and cytosine at similar rates. In an embodiment, the low bias DNA polymerase is able to mutate adenine, thymine, guanine, and cytosine at similar rates.

Optionally, the low bias DNA polymerase is able to mutate adenine and thymine, adenine and guanine, adenine and cytosine, thymine and guanine, thymine and cytosine, or guanine and cytosine at a rate ratio of 0.5-1.5:0.5-1.5, 0.6-1.4:0.6-1.4, 0.7-1.3:0.7-1.3, 0.8-1.2:0.8-1.2, or around 1:1 respectively. Preferably, the low bias DNA polymerase is able to mutate guanine and adenine at a rate ratio of 0.5-1.5:0.5-1.5, 0.6-1.4:0.6-1.4, 0.7-1.3:0.7-1.3, 0.8-1.2:0.8-1.2, or around 1:1 respectively. Preferably, the low bias DNA polymerase is able to mutate thymine and cytosine at a rate ratio of 0.5-1.5:0.5-1.5, 0.6-1.4:0.6-1.4, 0.7-1.3:0.7-1.3, 0.8-1.2:0.8-1.2, or around 1:1 respectively.

In such embodiments, in a step of introducing mutations into the plurality of target template nucleic acid molecules, the low bias DNA polymerase mutates adenine and thymine, adenine and guanine, adenine and cytosine, thymine and guanine, thymine and cytosine, or guanine and cytosine nucleotides in the at least one target template nucleic acid molecule at a rate ratio of 0.5-1.5:0.5-1.5, 0.6-1.4:0.6-1.4, 0.7-1.3:0.7-1.3, 0.8-1.2:0.8-1.2, or around 1:1 respectively. Preferably, the low bias DNA polymerase mutates guanine and adenine nucleotides in the at least one target template nucleic acid molecule at a rate ratio of 0.5-1.5:0.5-1.5, 0.6-1.4:0.6-1.4, 0.7-1.3:0.7-1.3, 0.8-1.2:0.8-1.2, or around 1:1 respectively. Preferably, the low bias DNA polymerase mutates thymine and cytosine nucleotides in the at least one target template nucleic acid molecule at a rate ratio of 0.5-1.5:0.5-1.5, 0.6-1.4:0.6-1.4, 0.7-1.3:0.7-1.3, 0.8-1.2:0.8-1.2, or around 1:1 respectively.

Optionally, the low bias DNA polymerase is able to mutate adenine, thymine, guanine, and cytosine at a rate ratio of 0.5-1.5:0.5-1.5:0.5-1.5:0.5-1.5, 0.6-1.4:0.6-1.4:0.6-1.4:0.6-1.4, 0.7-1.3:0.7-1.3:0.7-1.3:0.7-1.3, 0.8-1.2:0.8-1.2:0.8-1.2:0.8-1.2, or around 1:1:1:1 respectively. Preferably, the low bias DNA polymerase is able to mutate adenine, thymine, guanine and cytosine at a rate ratio of 0.7-1.3:0.7-1.3:0.7-1.3:0.7-1.3.

In such embodiments, in a step of introducing mutations into the at least one target template nucleic acid molecule in a second of the pair of samples, the low bias DNA polymerase may mutate adenine, thymine, guanine, and cytosine nucleotides in the at least one target template nucleic acid molecule at a rate ratio of 0.5-1.5:0.5-1.5:0.5-1.5:0.5-1.5, 0.6-1.4:0.6-1.4:0.6-1.4:0.6-1.4, 0.7-1.3:0.7-1.3:0.7-1.3:0.7-1.3, 0.8-1.2:0.8-1.2:0.8-1.2:0.8-1.2, or around 1:1:1:1 respectively. Preferably, the low bias DNA polymerase mutates adenine, thymine, guanine, and cytosine nucleotides in the at least one target template nucleic acid molecule at a rate ratio of 0.7-1.3:0.7-1.3:0.7-1.3:0.7-1.3.

The adenine, thymine, cytosine, and/or guanine may be substituted with another nucleotide. For example, if the low bias DNA polymerase is able to mutate adenine, enzymatic mutagenesis using the low bias DNA polymerase may substitute at least one adenine nucleotide in the nucleic acid molecule with thymine, guanine, or cytosine. Similarly, if the low bias DNA polymerase is able to mutate thymine, enzymatic mutagenesis using the low bias DNA polymerase may substitute at least one thymine nucleotide with adenine, guanine, or cytosine. If the low bias DNA polymerase is able to mutate guanine, enzymatic mutagenesis using the low bias DNA polymerase may substitute at least one adenine nucleotide with thymine, guanine, or cytosine. If the low bias DNA polymerase is able to mutate cytosine, enzymatic mutagenesis using the low bias DNA polymerase may substitute at least one cytosine nucleotide with thymine, guanine, or adenine.

The low bias DNA polymerase may not be able to substitute a nucleotide directly, but it may still be able to mutate that nucleotide by replacing the corresponding nucleotide on the complementary strand. For example, if the target template nucleic acid molecule comprises thymine, there will be an adenine nucleotide present in the corresponding position of the at least one nucleic acid molecule that is complementary to the at least one target template nucleic acid molecule. The low bias DNA polymerase may be able to replace the adenine nucleotide of the at least one nucleic acid molecule that is complementary to the at least one target template nucleic acid molecule with a guanine and so, when the at least one nucleic acid molecule that is complementary to the at least one target template nucleic acid molecule is replicated, this will result in a cytosine being present in the corresponding replicated at least one target template nucleic acid molecule where there was originally a thymine (a thymine to cytosine substitution).

In an embodiment, the low bias DNA polymerase mutates between 1% and 15%, between 2% and 10%, or around 8% of the nucleotides in the at least one target template nucleic acid. In such embodiments, the enzymatic mutagenesis using the low bias DNA polymerase is carried out in such a way that between 1% and 15%, between 2% and 10%, or around 8% of the nucleotides in the at least one target template nucleic acid are mutated. For example, if the user wishes to mutate around 8% of the nucleotides in the target template nucleic acid molecule, and the low bias DNA polymerase mutates around 1% of the nucleotides per round of replication, the step of introducing mutations into the plurality of target template nucleic acid molecules by enzymatic mutagenesis may comprise 8 rounds of replication in the presence of a low bias DNA polymerase.

In an embodiment, the low bias DNA polymerase is able to mutate between 0% and 3%, between 0% and 2%, between 0.1% and 5%, between 0.2% and 3%, or around 1.5% of the nucleotides in the at least one target template nucleic acid molecule per round of replication. In an embodiment, the low bias DNA polymerase mutates between 0% and 3%, between 0% and 2%, between 0.1% and 5%, between 0.2% and 3%, or around 1.5% of the nucleotides in the at least one target template nucleic acid molecule per round of replication. The actual amount of mutation that takes place each round may vary, but may average to between 0% and 3%, between 0% and 2%, between 0.1% and 5%, between 0.2% and 3%, or around 1.5%.

*Whether a DNA polymerase is able to mutate a nucleotide and, if so, at what rate*

Whether the low bias DNA polymerase is able to mutate a certain percentage of the nucleotides in the at least one target template nucleic acid molecule per round of replication can be determined by amplifying a nucleic acid molecule of known sequence in the presence of the low bias DNA polymerase for a set number of rounds of replication. The resulting amplified nucleic acid molecule can then be sequenced, and the percentage of nucleotides that are mutated per round of replication calculated. For example, the nucleic acid molecule of known sequence can be amplified using 10 rounds of PCR in the presence of the low bias DNA polymerase. The resulting nucleic acid molecule can then be sequenced. If the resulting nucleic acid molecule comprises 10% nucleotides that are different in corresponding nucleotides in the original known sequence, then the user would understand that the low bias DNA polymerase is able to mutate 1% of the nucleotides in the at least one target template nucleic acid molecule on average per round of replication. Similarly, to see whether the low bias DNA polymerase mutates a certain percentage of the nucleotides in the at least one target template nucleic acid molecule in a given method, the user could perform the method on a nucleic acid molecule of known sequence and use sequencing to determine the percentage of nucleotides that are mutated once the method is completed.

The low bias DNA polymerase is able to mutate a nucleotide such as adenine, if, when used to amplify a nucleic acid molecule, it provides a nucleic acid molecule in which some instances of that nucleotide are substituted or deleted. Preferably, the term *"mutate"* refers to introduction of substitution mutations, and in some embodiments the term *"mutate"* can be replaced with *"introduces substitutions of"*.

The low bias DNA polymerase mutates a nucleotide such as adenine in at least one target template nucleic acid molecule if, when a step of introducing mutations into the plurality of target template nucleic acid molecules using a low bias DNA polymerase is carried out, this step results in a mutated at least one target template nucleic acid molecule in which some instances of that nucleotide are mutated. For example, if the low bias DNA polymerase mutates adenine in the at least one target template nucleic acid molecule, when a step of introducing mutations into the plurality of target template nucleic acid molecules using a low bias DNA polymerase is carried out, this step results in a mutated at least one target template nucleic acid molecule in which at least one adenine has been substituted or deleted.

To determine whether a DNA polymerase is able to introduce certain mutations, the skilled person merely needs to test the DNA polymerase using a nucleic acid molecule of known sequence. A suitable nucleic acid molecule of known sequence is a fragment from a bacterial genome of known sequence, such as *E.coli* MG1655. The skilled person could amplify the nucleic acid molecule of known sequence using PCR in the presence of the low bias DNA polymerase. The skilled person could then sequence the amplified nucleic acid molecule and determine whether its sequence is the same as the original known sequence. If not, the skilled person could determine the nature of the mutations. For example, if the skilled person wished to determine whether a DNA polymerase is able to mutate adenine using a nucleotide analog, the skilled person could amplify the nucleic acid molecule of known sequence using PCR in the presence of the nucleotide analog, and sequence the resulting amplified nucleic acid molecule. If the amplified DNA has mutations in positions corresponding to adenine nucleotides in the known sequence, then the skilled person would know that the DNA polymerase could mutate adenine using a nucleotide analog.

Rate ratios can be calculated in a similar manner. For example, if the skilled person wishes to determine the rate ratio at which guanine and cytosine nucleotides are mutated, the skilled person could amplify a nucleic acid molecule having a known sequence using PCR in the presence of the low bias DNA polymerase. The skilled person could then sequence the resulting amplified nucleic acid molecule and identify how many of the guanine nucleotides have been substituted or deleted and how many of the cytosine nucleotides have been substituted or deleted. The rate ratio is the ratio of the number of guanine nucleotides that have been substituted or deleted to the number of cytosine nucleotides that have been substituted or deleted. For example, if 16 guanine nucleotides have been replaced or deleted and 8 cytosine nucleotides have been replaced or deleted, the guanine and cytosine nucleotides have been mutated at a rate ratio of 16:8 or 2:1 respectively.

*Using nucleotide analogs*

The low bias DNA polymerase may not be able to replace nucleotides with other nucleotides directly (at least not with high frequency), but the low bias DNA polymerase may still be able to mutate a nucleic acid molecule using a nucleotide analog. The low bias DNA polymerase may be able to replace nucleotides with other natural nucleotides (*i.e.* cytosine, guanine, adenine or thymine) or with nucleotide analogs.

For example, the low bias DNA polymerase may be a high fidelity DNA polymerase. High fidelity DNA polymerases tend to introduce very few mutations in general, as they are highly accurate. However, the present inventors have found that some high fidelity DNA polymerases may still be able to mutate a target template nucleic acid molecule, as they may be able to introduce nucleotide analogs into a target template nucleic acid molecule.

In an embodiment, in the absence of nucleotide analogs, the high fidelity DNA polymerase introduces less than 0.01%, less than 0.0015%, less than 0.001%, between 0% and 0.0015%, or between 0% and 0.001% mutations per round of replication.

In an embodiment, the low bias DNA polymerase is able to incorporate nucleotide analogs into the at least one target template nucleic acid molecule. In an embodiment, the low bias DNA polymerase incorporates nucleotide analogs into the at least one target template nucleic acid molecule. In an embodiment, the low bias DNA polymerase can mutate adenine, thymine, guanine, and/or cytosine using a nucleotide analog. In an embodiment, the low bias DNA polymerase mutates adenine, thymine, guanine, and/or cytosine in the at least one target template nucleic acid molecule using a nucleotide analog. In an embodiment, the DNA polymerase replaces guanine, cytosine, adenine and/or thymine with a nucleotide analog. In an embodiment, the DNA polymerase can replace guanine, cytosine, adenine and/or thymine with a nucleotide analog.

Incorporating nucleotide analogs into the at least one target template nucleic acid molecule can be used to mutate nucleotides, as they may be incorporated in place of existing nucleotides and they may pair with nucleotides in the opposite strand. For example dPTP can be incorporated into a nucleic acid molecule in place of a pyrimidine nucleotide (may replace thymine or cytosine). Once in a nucleic acid strand, it may pair with adenine when in an imino tautomeric form. Thus, when a complementary strand is formed, that complementary strand may have an adenine present at a position complementary to the dPTP. Similarly, once in a nucleic acid strand, it may pair with guanine when in an amino tautomeric form. Thus, when a complementary strand is formed, that complementary strand may have a guanine present at a position complementary to the dPTP.

For example, if a dPTP is introduced into the at least one target template nucleic acid molecule of the invention, when an at least one nucleic acid molecule complementary to the at least one target template nucleic acid molecule is formed, the at least one nucleic acid molecule complementary to the at least one target template nucleic acid molecule will comprise an adenine or a guanine at a position complementary to the dPTP in the at least one target template nucleic acid molecule (depending on whether the dPTP is in its amino or imino form). When the at least one nucleic acid molecule complementary to the at least one target template nucleic acid molecule is replicated, the resulting replicate of the at least one target template nucleic acid molecule will comprise a thymine or a cytosine in a position corresponding to the dPTP in the at least one target template nucleic acid molecule. Thus, a mutation to thymine or cytosine can be introduced into the mutated at least one target template nucleic acid molecule.

Alternatively, if a dPTP is introduced in at least one nucleic acid molecule complementary to the at least one target template nucleic acid molecule, when a replicate of the at least one target template nucleic acid molecule is formed, the replicate of the at least one target template nucleic acid molecule will comprise an adenine or a guanine at a position complementary to the dPTP in the at least one nucleic acid molecule complementary to the at least one target template nucleic acid molecule (depending on the tautomeric form of the dPTP). Thus, a mutation to adenine or guanine can be introduced into the mutated at least one target template nucleic acid molecule.

In an embodiment, the low bias DNA polymerase can replace cytosine or thymine with a nucleotide analog. In a further embodiment, the low bias DNA polymerase introduces guanine or adenine nucleotides using a nucleotide analog at a rate ratio of 0.5-1.5:0.5-1.5, 0.6-1.4:0.6-1.4, 0.7-1.3:0.7-1.3, 0.8-1.2:0.8-1.2, or around 1:1 respectively. The guanine or adenine nucleotides may be introduced by the low bias DNA polymerase pairing them opposite a nucleotide analog such as dPTP. In a further embodiment, the low bias DNA polymerase introduces guanine or adenine nucleotides using a nucleotide analog at a rate ratio of 0.7-1.3:0.7-1.3 respectively.

The skilled person can determine, using conventional methods, whether the low bias DNA polymerase is able to incorporate nucleotide analogs into the at least one target template nucleic acid molecule or mutate adenine, thymine, guanine, and/or cytosine in the at least one target template nucleic acid molecule using a nucleotide analog using conventional methods.

For example, in order to determine whether the low bias DNA polymerase is able to incorporate nucleotide analogs into the at least one target template nucleic acid molecule, the skilled person could amplify a nucleic acid molecule using a low bias DNA polymerase for two rounds of replication. The first round of replication should take place in the presence of the nucleotide analog, and the second round of replication should take place in the absence of the nucleotide analog. The resulting amplified nucleic acid molecules could be sequenced to see whether mutations have been introduced, and if so, how many mutations. The user should repeat the experiment without the nucleotide analog, and compare the number of mutations introduced with and without the nucleotide analog. If the number of mutations that have been introduced with the nucleotide analog is significantly higher than the number of mutations that have been introduced without the nucleotide analog, the user can conclude that the low bias DNA polymerase is able to incorporate nucleotide analogs. Similarly, the skilled person can determine whether a DNA polymerase incorporates nucleotide analogs or mutates adenine, thymine, guanine, and/or cytosine using a nucleotide analog. The skilled person merely need perform the method in the presence of nucleotide analogs, and see whether the method leads to mutations at positions originally occupied by adenine, thymine, guanine, and/or cytosine. If the user wishes to mutate the at least one target template nucleic acid molecule using a nucleotide analog, the method may comprise a step of amplifying the at least one target template nucleic acid molecule using a low bias DNA polymerase, where the step of amplifying the at least one target template nucleic acid molecule using a low bias DNA polymerase is carried out in the presence of the nucleotide analog, and the step of amplifying the at least one target template nucleic acid molecule provides at least one target template nucleic acid molecule comprising the nucleotide analog.

Suitable nucleotide analogs include dPTP (2'deoxy-P-nucleoside-5'-triphosphate), 8-Oxo-dGTP (7,8-dihydro-8-oxoguanine), 5Br-dUTP (5-bromo-2'-deoxy-uridine-5'-triphosphate), 2OH-dATP (2-hydroxy-2'-deoxyadenosine-5'-triphosphate), dKTP (9-(2-Deoxy-β-D-ribofuranosyl)-N6-methoxy-2,6,-diaminopurine-5'-triphosphate) and dITP (2'-deoxy-yinosine 5'-trisphosphate). The nucleotide analog may be dPTP. The nucleotide analogs may be used to introduce the substitution mutations described in Table 1.

Table 1

| Nucleotide | Substitution |
|---|---|
| 8-oxo-dGTP | A:T to C:G and T:A to G:C |

(continued)

| Nucleotide | Substitution |
|------------|--------------|
| dPTP | A:T to G:C and G:C to A:T |
| 5Br-dUTP | A:T to G:C and T:A to C:G |
| 2OH-dATP | A:T to C:G, G:C to T:A and A:T to G:C |
| dITP | A:T to G:C and G:C to A:T |
| dKTP | A:T to G:C and G:C to A:T |

The different nucleotide analogs can be used, alone or in combination, to introduce different mutations into the at least one target template nucleic acid molecule. Accordingly, the low bias DNA polymerase may introduce guanine to adenine substitution mutations, cytosine to thymine substitution mutations, adenine to guanine substitution mutations, and thymine to cytosine substitution mutations using a nucleotide analog. The low bias DNA polymerase may be able to introduce guanine to adenine substitution mutations, cytosine to thymine substitution mutations, adenine to guanine substitution mutations, and thymine to cytosine substitution mutations, optionally using a nucleotide analog.

The low bias DNA polymerase may be able to introduce guanine to adenine substitution mutations, cytosine to thymine substitution mutations, adenine to guanine substitution mutations, and thymine to cytosine substitution mutations at a rate ratio of 0.5-1.5:0.5-1.5:0.5-1.5:0.5-1.5, 0.6-1.4:0.6-1.4:0.6-1.4:0.6-1.4, 0.7-1.3:0.7-1.3:0.7-1.3:0.7-1.3, 0.8-1.2:0.8-1.2:0.8-1.2:0.8-1.2, or around 1:1:1:1 respectively. Preferably, the low bias DNA polymerase is able to introduce guanine to adenine substitution mutations, cytosine to thymine substitution mutations, adenine to guanine substitution mutations, and thymine to cytosine substitution mutations at a rate ratio of 0.7-1.3:0.7-1.3:0.7-1.3:0.7-1.3 respectively. Suitable methods for determining whether the low bias DNA polymerase is able to introduce substitution mutations and at what rate ratio are described under the heading *"whether a DNA polymerase is able to mutate a nucleotide and, if so, at what rate* ".

In some methods the low bias DNA polymerase introduces guanine to adenine substitution mutations, cytosine to thymine substitution mutations, adenine to guanine substitution mutations, and thymine to cytosine substitution mutations at a rate ratio of 0.5-1.5:0.5-1.5:0.5-1.5:0.5-1.5, 0.6-1.4:0.6-1.4:0.6-1.4:0.6-1.4, 0.7-1.3:0.7-1.3:0.7-1.3:0.7-1.3, 0.8-1.2:0.8-1.2:0.8-1.2:0.8-1.2, or around 1:1:1:1 respectively. Preferably, the low bias DNA polymerase introduces guanine to adenine substitution mutations, cytosine to thymine substitution mutations, adenine to guanine substitution mutations, and thymine to cytosine substitution mutations at a rate ratio of 0.7-1.3:0.7-1.3:0.7-1.3:0.7-1.3 respectively. Suitable methods for determining whether substitution mutations are introduced and at what rate ratio are described under the heading *"whether a DNA polymerase is able to mutate a nucleotide and, if so, at what rate* ".

Generally, when a low bias DNA polymerase uses a nucleotide analog to introduce a mutation, this requires more than one round of replication. In the first round of replication the low bias DNA polymerase introduces the nucleotide analog in place of a nucleotide, and in a second round of replication, that nucleotide analog pairs with a natural nucleotide to introduce a substitution mutation in the complementary strand. The second round of replication may be carried out in the presence of the nucleotide analog. However, the method may further comprise a step of amplifying the at least one target template nucleic acid molecule in a second of the pair of samples comprising nucleotide analogs in the absence of nucleotide analogs. The step of amplifying the at least one target template nucleic acid molecule comprising nucleotide analogs in the absence of nucleotide analogs may be carried out using the low bias DNA polymerase.

*Low template amplification bias*

The low bias DNA polymerase may have low template amplification bias. A low bias DNA polymerase has low template amplification bias, if it is able to amplify different target template nucleic acid molecules with similar degrees of success per cycle. High bias DNA polymerases may struggle to amplify template nucleic acid molecules that comprise a high G:C content or contain a large degree of secondary structure. In an embodiment, the low bias DNA polymerase has low template amplification bias for template nucleic acid molecules that are less than 25 000, less than 10 000, between 1 and 15 000, or between 1 and 10 000 nucleotides in length.

In an embodiment, to determine whether a DNA polymerase has low template amplification bias, the skilled person could amplify a range of different sequences using the DNA polymerase, and see whether the different sequences are amplified at different levels by sequencing the resultant amplified DNA. For example, the skilled person could select a range of short (possibly 50 nucleotide) nucleic acid molecules having different characteristics, including a nucleic acid molecule having high GC content, a nucleic acid molecule having low GC content, a nucleic acid molecule having a large degree of secondary structure and a nucleic acid molecule have a low degree of second structure.

The user could then amplify those sequences using the DNA polymerase and quantify the level at which each of the nucleic acid molecules is amplified to. In an embodiment, if the levels are within 25%, 20%, 10%, or 5% of one another, then the DNA polymerase has low template amplification bias.

Alternatively, in an embodiment, a DNA polymerase has low template amplification bias if it is able to amplify 7-10 kbp fragments with a Kolmolgorov-Smirnov D of less than 0.1, less than 0.09, or less than 0.08. The Kolmolgorov-Smirnov D with which a particular low bias DNA polymerase is able to amplify 7-10 kbp fragments may be determined using an assay provided in Example 4.

The low bias DNA polymerase may be a high fidelity DNA polymerase. A high fidelity DNA polymerase is a DNA polymerase which is not highly error-prone, and so does not generally introduce a large number of mutations when used to amplify a target template nucleic acid molecule in the absence of nucleotide analogs. High fidelity DNA polymerases are not generally used in methods for introducing mutations, as it is generally considered that error-prone DNA polymerases are more effective. However, the present application demonstrates that certain high fidelity polymerases are able to introduce mutations using a nucleotide analog, and that those mutations may be introduced with lower bias compared to error-prone DNA polymerases such as Taq polymerase.

High fidelity DNA polymerases have an additional advantage. High fidelity DNA polymerases can be used to introduce mutations when used with nucleotide analogs, but in the absence of nucleotide analogs they can replicate a target template nucleic acid molecule highly accurately. This means that the user can mutate the at least one target template nucleic acid molecule to high effect and amplify the mutated at least one target template nucleic acid molecule with high accuracy using the same DNA polymerase. If a low fidelity DNA polymerase is used to mutate the target template nucleic acid molecule, it may need to be removed from the reaction mixture before the target template nucleic acid molecule is amplified.

High fidelity DNA polymerases may have a proof-reading activity. A proof-reading activity may help the DNA polymerase to amplify a target template nucleic acid sequence with high accuracy. For example, a low bias DNA polymerase may comprise a proof-reading domain. A proof reading domain may confirm whether a nucleotide that has been added by the polymerase is correct (checks that it correctly pairs with the corresponding nucleic acid of the complementary strand) and, if not, excises it from the nucleic acid molecule. The inventors have surprisingly found that in some DNA polymerases, the proof-reading domain will accept pairings of natural nucleotides with nucleotide analogs. The structure and sequence of suitable proof-reading domains are known to the skilled person. DNA polymerases that comprise a proof-reading domain include members of DNA polymerase families I, II and III, such as Pfu polymerase (derived from Pyrococcus furiosus), T4 polymerase (derived from bacteriophage T4) and the Thermococcal polymerases that are described in more detail below.

In an embodiment, in the absence of nucleotide analogs, the high fidelity DNA polymerase introduces less than 0.01%, less than 0.0015%, less than 0.001%, between 0% and 0.0015%, or between 0% and 0.001% mutations per round of replication.

In addition, the low bias DNA polymerase may comprise a processivity enhancing domain. A processivity enhancing domain allows a DNA polymerase to amplify a target template nucleic acid molecule more quickly. This is advantageous as it allows the methods of the invention to be performed more quickly.

*Thermococcal polymerases*

In an embodiment, the low bias DNA polymerase is a fragment or variant of a polypeptide comprising SEQ ID NO. 2, SEQ ID NO. 4, SEQ ID NO. 6, or SEQ ID NO.7. The polypeptides of SEQ ID NO. 2, 4, 6 and 7 are thermococcal polymerases. The polymerases of SEQ ID NO. 2, SEQ ID NO. 4, SEQ ID NO. 6, or SEQ ID NO. 7 are low bias DNA polymerases having high fidelity, and they can mutate target template nucleic acid molecules by incorporating a nucleotide analog such as dPTP. The polymerases of SEQ ID NO. 2, SEQ ID NO. 4, SEQ ID NO. 6, or SEQ ID NO. 7 are particularly advantageous as they have low mutation bias and low template amplification bias. They are also highly processive and are high fidelity polymerases comprising a proof-reading domain, meaning that, in the absence of nucleotide analogs, they can amplify mutated target template nucleic acid molecules quickly and accurately.

The low bias DNA polymerase may comprise a fragment of at least 400, at least 500, at least 600, at least 700, or at least 750 contiguous amino acids of:

> a. a sequence of SEQ ID NO. 2;
> b. a sequence at least 95%, at least 98%, or at least 99% identical to SEQ ID NO. 2;
> c. a sequence of SEQ ID NO. 4;
> d. a sequence at least 95%, at least 98%, or at least 99% identical to SEQ ID NO. 4;
> e. a sequence of SEQ ID NO. 6;
> f. a sequence at least 95%, at least 98%, or at least 99% identical to SEQ ID NO. 6;
> g. a sequence of SEQ ID NO. 7; or

h. a sequence at least 95%, at least 98%, or at least 99% identical to SEQ ID NO. 7.

Preferably, the low bias DNA polymerase comprises a fragment of at least 700 contiguous amino acids of:

a. a sequence of SEQ ID NO. 2;
b. a sequence at least 98%, or at least 99% identical to SEQ ID NO. 2;
c. a sequence of SEQ ID NO. 4;
d. a sequence at least 98%, or at least 99% identical to SEQ ID NO. 4;
e. a sequence of SEQ ID NO. 6;
f. a sequence at least 98%, or at least 99% identical to SEQ ID NO. 6;
g. a sequence of SEQ ID NO. 7; or
h. a sequence at least 98%, or at least 99% identical to SEQ ID NO. 7.

The low bias DNA polymerase may comprise:

a. a sequence of SEQ ID NO. 2;
b. a sequence at least 95%, at least 98%, or at least 99% identical to SEQ ID NO. 2;
c. a sequence of SEQ ID NO. 4;
d. a sequence at least 95%, at least 98%, or at least 99% identical to SEQ ID NO. 4;
e. a sequence of SEQ ID NO. 6;
f. a sequence at least 95%, at least 98%, or at least 99% identical to SEQ ID NO. 6;
g. a sequence of SEQ ID NO. 7; or
h. a sequence at least 95%, at least 98%, or at least 99% identical to SEQ ID NO. 7.

Preferably, the low bias DNA polymerase comprises:

a. a sequence of SEQ ID NO. 2;
b. a sequence at least 98%, or at least 99% identical to SEQ ID NO. 2;
c. a sequence of SEQ ID NO. 4;
d. a sequence at least 98%, or at least 99% identical to SEQ ID NO. 4;
e. a sequence of SEQ ID NO. 6;
f. a sequence at least 98%, or at least 99% identical to SEQ ID NO. 6;
g. a sequence of SEQ ID NO. 7; or
h. a sequence at least 98%, or at least 99% identical to SEQ ID NO. 7.

The low bias DNA polymerase may be a thermococcal polymerase, or derivative thereof. The DNA polymerases of SEQ ID NO 2, 4, 6 and 7 are thermococcal polymerases. Thermococcal polymerases are advantageous, as they are generally high fidelity polymerases that can be used to introduce mutations using a nucleotide analog with low mutation and template amplification bias.

A thermococcal polymerase is a polymerase having the polypeptide sequence of a polymerase isolated from a strain of the *Thermococcus* genus. A derivative of a thermococcal polymerase may be a fragment of at least 400, at least 500, at least 600, at least 700, or at least 750 contiguous amino acids of a thermococcal polymerase, or at least 95%, at least 98%, at least 99%, or 100% identical to a fragment of at least 400, at least 500, at least 600, at least 700 or at least 750 contiguous amino acids of a thermococcal polymerase. The derivative of a thermococcal polymerase may be at least 95%, at least 98%, at least 99%, or 100% identical to a thermococcal polymerase. The derivative of a thermococcal polymerase may be at least 98% identical to a thermococcal polymerase.

A thermococcal polymerase from any strain may be effective in the context of the present invention. In an embodiment, the thermococcal polymerase is derived from a thermococcal strain selected from the group consisting of *T. kodakarensis, T. celer, T. siculi,* and *T. sp* KS-1. Thermococccal polymerases from these strains are described in SEQ ID NO. 2, SEQ ID NO. 4, SEQ ID NO. 6 and SEQ ID NO. 7.

Optionally, the low bias DNA polymerase is a polymerase that has high catalytic activity at temperatures between 50°C and 90°C, between 60°C and 80°C, or around 68°C.

**Examples**

Example 1 - Mutating nucleic acid molecules using PrimeStar GXL or other polymerases

DNA molecules were fragmented to the appropriate size (*e.g.* 10 kb) and a defined sequence priming site (adapter) was attached on each end using tagmentation.

The first step is a tagmentation reaction to fragment the DNA. 50 ng high molecular weight genomic DNA in 4μl or less volume of one or more bacterial strains was subjected to tagmentation under the following conditions. 50 ng DNA is combined with 4 μl Nextera Transposase (diluted to 1:50), and 8 μl 2X tagmentation buffer (20mM Tris [pH7.6], 20mM MgCl, 20% (v/v) dimethylformamide) in a total volume of 16 μl. The reaction was incubated at 55°C for 5 minutes, 4μl of NT buffer (or 0.2% SDS) was added to the reaction and the reaction was incubated at room temperature for 5 minutes.

The tagmentation reaction was cleaned using SPRIselect beads (Beckman Coulter) following the manufacturer's instructions for a left side size selection using 0.6 volume of beads, and the DNA was eluted in molecular grade water.

This was followed by PCR with a combination of standard dNTPs and dPTP for a limited 6 cycles. Using Primestar GXL, 12.5 ng of tagmented and purified DNA was added to a total reaction volume of 25μl, containing 1 × GXL buffer, 200 μM each of dATP, dTTP, dGTP and dCTP, as well as 0.5 mM dPTP, and 0.4 μM custom primers (Table 2).

Table 2:

| | | | | |
|---|---|---|---|---|
| i7 custom index primer | CAAGCAGAAGACGGCA TACGAGAT | NNN<br><br>NNN | XXX<br>X<br>X<br><br>X | GTCTCGTGG GCTCGG |
| i5 custom index primer | AATGATACGGCGACCA CCGAGATCTACAC | XXX<br><br>XXX | NNN<br>N<br>N<br><br>N | TCGTCGGCA GCGTC |

**Table 2.** Custom primers used for mutagenesis PCR on 10kbp templates. XXXXXX is a defined, sample-specific 6-8nt barcode (sample tag) sequence. NNNNNN is a 6nt region of random nucleotides.

The reaction was subject to the following thermal cycling in the presence of Primestar GXL. Initial gap extension at 68°C for 3 minutes, followed by 6 cycles of 98°C for 10 seconds, 55°C for 15 seconds and 68°C for 10 minutes.

The next stage is a PCR without dPTP, to remove dPTP from the templates and replace them with a transition mutation ("recovery PCR"). PCR reactions were cleaned with SPRIselect beads to remove excess dPTP and primers, then subjected to a further 10 rounds (minimum 1 round, maximum 20) of amplification using primers that anneal to the fragment ends introduced during the dPTP incorporation cycles (Table 3).

Table 3

| | |
|---|---|
| i7 flow cell primer | CAAGCAGAAGAC GGCATACGA |
| i5 flow cell primer | AATGATACGGCG ACCACCGA |

This was followed by a gel extraction step to size select amplified and mutated fragments in a desired size range, for example from 7-10 kb. The gel extraction can be done manually or via an automated system such as a BluePippin. This was followed by an additional round of PCR for 16-20 cycles ("*enrichment PCR*").

After amplifying a defined number of long mutated templates, random fragmentation of the templates was carried out to generate a group of overlapping shorter fragments for sequencing. Fragmentation was performed by tagmentation.

Long DNA fragments from the previous step were subject to a standard tagmentation reaction (*e.g.* Nextera XT or Nextera Flex), except that the reaction was split into three pools for the PCR amplification. This enables selective amplification of fragments derived from each end of the original template (including the sample tag) as well as internal fragments from the long template that have been newly tagmented at both ends. This effectively creates three pools for sequencing on an Illumina instrument (*e.g.* MiSeq or HiSeq).

The method was repeated using a standard Taq (Jena Biosciences) and a blend of Taq and a proofreading polymerase (DeepVent) called LongAmp (New England Biolabs).

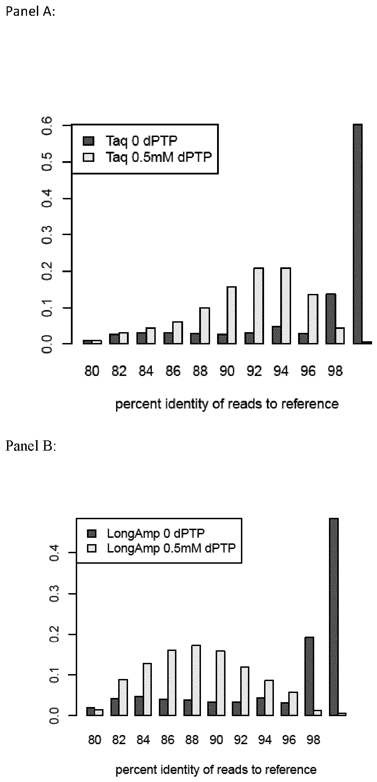

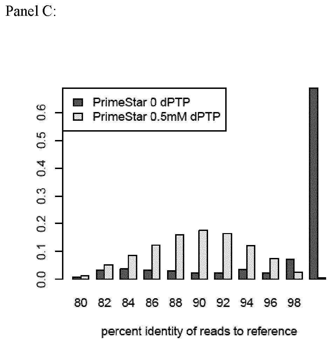

The data obtained from this experiment is depicted in Figure 1. No dPTP was used a control. Reads were mapped against the *E. coli* genome, and a median mutation rate of ~ 8% was achieved.

Example 2 - Comparison of mutation frequencies of different DNA polymerases

Mutagenesis was performed with a range of different DNA polymerases (Table 4). Genomic DNA from *E. coli* strain MG1655 was tagmented to produce long fragments and bead cleaned as described in the method of Example 1. This was followed by "mutagenesis PCR" for 6 cycles in the presence of 0.5 mM dPTP, SPRIselect bead purification and an additional 14-16 cycles of "recovery PCR" in the absence of dPTP. The resulting long mutated templates were then subjected to a standard tagmentation reaction (see Example 1) and "internal" fragments were amplified and sequenced on an Illumina MiSeq instrument.

The mutation rates are described in Table 4, which normalized frequencies of base substitution via dPTP mutagenesis reactions as measured using Illumina sequencing of DNA from the known reference genome. For Taq polymerase, only ~12% of mutations occur at template G+C sites, even when used in buffer optimised for Thermococcus polymerases. Thermococcus-like polymerases result in 58-69% of mutations at template G+C sites, while polymerase derived from Pyrococcus gives 88% of mutations at template G+C sites.

Enzymes were obtained from Jena Biosciences (Taq), Takara (Primestar variants), Merck Millipore (KOD DNA Polymerase) and New England Biolabs (Phusion).

Taq was tested with the supplied buffer, and also with Primestar GXL Buffer (Takara) for this experiment. All other reactions were carried out with the standard supplied buffer for each polymerase.

**Table 4**

| Polymerase[1] | Origin | Mutation frequency (% of total observed mutations) | | | | |
|---|---|---|---|---|---|---|
| | | A → G | T → C | G → A | C → T | Other (transversi on) |
| Taq (standard buffer) | *Thermus aquaticus* | 43.1 | 41.7 | 6.3 | 6.1 | 2.7 |
| Taq (*Thermococcus* buffer[2]) | *Thermus aquaticus* | 48.9 | 47.5 | 2.9 | 0.7 | 0.0 |
| Primestar GXL | *Thermococcus* | 21.5 | 20.1 | 29.5 | 28.9 | 0.0 |
| Primestar HS | *Thermococcus* | 16.3 | 15.2 | 30.1 | 38.4 | 0.0 |
| Primestar Max | *Thermococcus* | 16.5 | 14.6 | 33.2 | 35.7 | 0.0 |
| KOD DNA polymerase | *Thermococcus* | 20.5 | 16.1 | 31.8 | 31.5 | 0.0 |
| Phusion | *Pyrococcus* | 5.4 | 6.4 | 44.1 | 44.1 | 0.0 |

Example 3 - determining dPTP mutagenesis rates

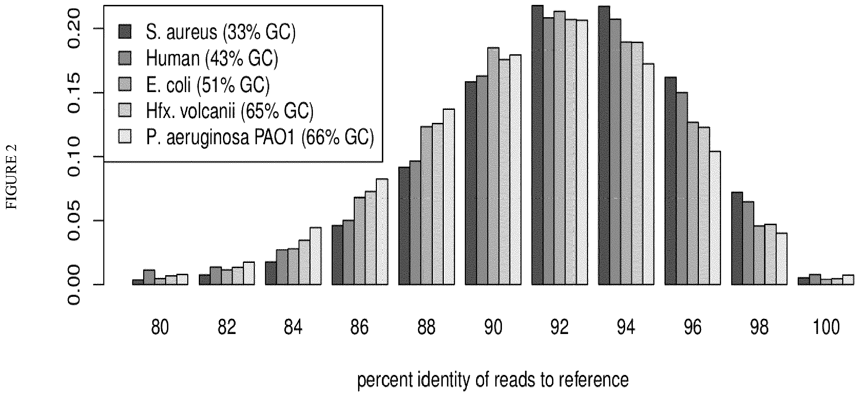

We performed dPTP mutagenesis on a range of genomic DNA samples with different levels of G+C content (33-66%) using a *Thermococcus* polymerase (Primestar GXL; Takara) under a single set of reaction conditions. Mutagenesis and sequencing was performed as described in the method of example 1, except that 10 cycles of "recovery PCR" were performed. As predicted, mutation rates were roughly similar between samples (median rate 7-8%) despite the diversity of G+C content (figure 2).

Example 4 - measuring template amplification bias

Template amplification bias was measured for two polymerases: Kapa HiFi, which is a proofreading polymerase commonly used in Illumina sequencing protocols, and Prime Star GXL, which is a KOD family polymerase known for its ability to amplify long fragments. In the first experiment Kapa HiFi was used to amplify a limited number of *E. coli* genomic DNA templates with sizes around 2kbp. The ends of these amplified fragments were then sequenced. A similar experiment was done with PrimeStar GXL on fragments around 7-10kbp from *E. coli*. The positions of each end sequence read were determined by mapping to the *E. coli* reference genome. The distances between neighboring fragment ends was measured. These distances were compared to a set of distances randomly sampled from the uniform distribution. The comparison was carried out via the nonparametric Kolmolgorov-Smirnov test, D. When two samples come from the same distribution, the value of D approaches zero. For the low bias PrimeStar polymerase, we observed D=0.07 when measured on 50,000 fragment ends, compared to a uniform random sample of 50,000 genomic positions. For the Kapa HiFi polymerase we observed D=0.14 on 50,000 fragment ends.

Example 5 - measuring size range of reconstruction

Mutated and non mutated sequence reads were generated, and a sequence for the non-mutated sequence reads was determined using computer implemented method steps.

To generate the mutated sequence reads, mutated target template nucleic acid molecule fragments were generated using the method described in Example 1, except that the fragment size range was restricted to 1-2kb. The mutated target template nucleic acid molecule fragments were sequence using an Illumnia MiSeq with a V2 500 cycle flowcell.

To generate non-mutated sequence reads, the following steps were performed. The first step is a tagmentation reaction to fragment the DNA. 50 ng high molecular weight genomic DNA in $4\mu$l or less volume of one or more bacterial strains was subjected to tagmentation under the following conditions. 50 ng DNA is combined with 4 $\mu$l Nextera Transposase (diluted to 1:50), and 8 $\mu$l 2X tagmentation buffer (20mM Tris [pH7.6], 20mM MgCl, 20% (v/v) dimethylformamide) in a total volume of 16 $\mu$l. The reaction was incubated at 55°C for 5 minutes, $4\mu$l of NT buffer (or 0.2% SDS) was added to the reaction and the reaction was incubated at room temperature for 5 minutes.

The tagmentation reaction was cleaned using SPRIselect beads (Beckman Coulter) following the manufacturer's instructions for a left side size selection using 0.6 volume of beads, and the DNA was eluted in molecular grade water. Long DNA fragments from the previous step were subject to a standard tagmentation reaction (e.g. Nextera XT or Nextera Flex), except that the reaction was split into three pools for the PCR amplification. This enables selective amplification of fragments derived from each end of the original template (including the sample tag) as well as internal fragments from the long template that have been newly tagmented at both ends. This effectively creates three pools for sequencing on an Illumina instrument (*e.g.* MiSeq or HiSeq).

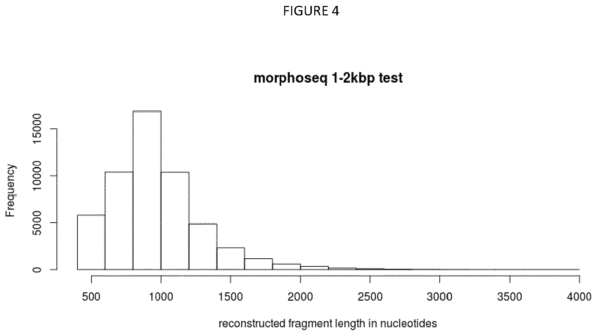

Sequences for the target template nucleic acid molecules were determined by pre-clustering the mutated sequence reads into read groups, then each group of mutated reads was subjected to de novo assembly using steps 1 and 2 of the A5-miseq assembly pipeline (Coil et al 2015 Bioinformatics).The analysis yielded 53,053 virtual fragments with lengths distributed as shown in figure 4.

Example 6 - testing probability algorithm

A probability algorithm was used to determine whether two mutated sequence reads were derived from the same original at least one template nucleic acid molecule. The details of the probability algorithm are as follows.

Given two non mutated sequence reads $S_1$ and $S_2$, in the mutated sequence read set that have been aligned to an unmutated reference sequence $R$, the model described here seeks to determine if $S_1$ and $S_2$ have been sequenced from the same at least one mutated template nucleic acid molecule or from different templates. The alignment of these three sequences can be represented as a $3 \times N$ matrix $\mathbf{N}$ of aligned sites, *e.g.* $N$ 3-tuples of individual nucleotides $s_{1,i}$ : $s_{2,j} : r_k$ with aligned nucleotides occurring in the same column $y$ of N, *e.g.* $n_{\cdot,y}$. For convenience, define a mapping from the nucleotides A, C, G and T to the integers 1, 2, 3 and 4 such that A maps to 1, C maps to 2, etc. This mapping is implied in the remainder of the description below. Next, define two $4 \times 4$ probability matrices: $\mathbf{M}$ and $\mathbf{E}$. Each entry $m_{i,j}$ records the probability that nucleotide $i$ was mutated via the mutagenesis process into nucleotide $j$ for $i,j \in \{ A, C, G, T \}$. Similarly, the entry $e_{i,j}$ records the conditional probability that the nucleotide $i$ was erroneously read as the nucleotide $j$, for $ij \in \{ A, C, G, T \}$ conditional on the nucleotide having been read erroneously. Further, define a $2 \times N$ matrix $\mathbf{Q}$ with entries $q_{1,y}$ and $q_{2,y}$ denoting the probability, as reported by the sequencing instrument, that the nucleotide in alignment position $y$ was read erroneously for sequences $S_1$ and $S_2$ respectively. Finally, use $z \in \{ 0, 1 \}$ as an indicator value for whether two sequence reads have derived from the same mutated template, with $z = 1$ indicating that $S_1$ and $S_2$ have been sequenced from the same template fragment and $z = 0$ indicating that $S_1$ and $S_2$ have been sequenced from different template fragments.

The values of $\mathbf{Q}$ and $\mathbf{N}$ are provided/determined by the sequencing and subsequent read mapping processes, however the values **of M, E** and $z$ are generally unknown. Fortunately, these values (and any other unknown parameters) can be estimated from the data using any one of a wide range of techniques. Prior distributions can be imposed on the values of unknown parameters based on knowledge of the mutation process. A Dirichlet distribution is imposed over the rows **of M,** such that: $m_{1,\cdot} \sim$ Dirichlet($\alpha+\beta$, 1-$\beta$, 1-$\alpha$, 1-$\beta$), where the entries correspond to the events A → A (no mutation), A → C (a transversion), A → G (a transition), A → T (a transversion). Here $\alpha$ is the unknown transition rate hyperparameter, and $\beta$ is the unknown transversion rate hyperparameter. The complete prior for **M** is specified as:

$$m_{1,\cdot} \sim \text{Dirichlet} \ (\alpha+\beta, \ 1-\beta, \ 1-\alpha, \ 1-\beta)$$

$$m_{2,\cdot} \sim \text{Dirichlet} \ (1-\beta, \ \alpha+\beta, \ 1-\beta, \ 1-\alpha)$$

$$m_{3,\cdot} \sim \text{Dirichlet} \ (1-\alpha, \ 1-\beta, \ \alpha+\beta, \ 1-\beta)$$

$$m_{4,\cdot} \sim \text{Dirichlet} \ (1-\beta, \ 1-\alpha, \ 1-\beta, \ \alpha+\beta)$$

Prior knowledge of the mutation process is generally available to the experimenter (*e.g.* the knowledge of the properties of the polymerase or other mutagen) and may allow hyperpriors on the $\alpha$ and $\beta$ terms to be applied. More general structures for the prior on **M** are possible. Uniform priors are applied on the matrix **E,** as well as z.

Given the above notation, the likelihood of the data given the model can be expressed as:

$$P(\mathbf{N},\mathbf{Q}|\mathbf{M},\mathbf{E},z) = \prod_{i=1}^{\square} \ (z) \ f(\mathbf{N},\mathbf{Q}|\mathbf{M},\mathbf{E},i) + (1 - z) \ g(\mathbf{N},\mathbf{Q}|\mathbf{M},\mathbf{E},i)$$

where:

$$f(\mathbf{N}, \mathbf{Q}|\mathbf{M}, \mathbf{E}, i) = \mathbb{1}_{n_{1,i}=n_{2,i}}\left\{ m_{n_{3,i},n_{1,i}}(1 - q_{1,i})(1 - q_{2,i}) \right\}+$$
$$\mathbb{1}_{n_{1,i}\neq n_{2,i}}\left\{ m_{n_{3,i},n_{1,i}}(1 - q_{1,i})q_{2,i}\frac{e_{n_{1,i},n_{2,i}}}{\sum e_{\cdot,n_{2,i}}} \right\}+$$
$$\mathbb{1}_{n_{1,i}\neq n_{2,i}}\left\{ m_{n_{3,i},n_{2,i}}q_{1,i}(1 - q_{2,i})\frac{e_{n_{2,i},n_{1,i}}}{\sum e_{\cdot,n_{1,i}}} \right\}+$$
$$\sum_{j=1..4} \frac{m_{n_{3,i},j}q_{1,i}e_{n_{2,i},n_{1,i}}q_{2,i}e_{n_{1,i},n_{2,i}}}{\sum e_{\cdot,n_{1,i}} \sum e_{\cdot,n_{2,i}}}$$

$$g(\mathbf{N}, \mathbf{Q}|\mathbf{M}, \mathbf{E}, i) = \left((1-q_{1,i})m_{n_{3,i},n_{1,i}}+q_{1,i}m_{n_{3,i},\cdot}e_{\cdot,n_{1,i}}\right)\left((1-q_{2,i})m_{n_{3,i},n_{2,i}}+q_{2,i}m_{n_{3,i},\cdot}e_{\cdot,n_{2,i}}\right)$$

Here the center dot in a matrix subscript connotes all members of the row or column, and vector multiplication implies the dot product. **1_{}** is the indicator function, taking the value 1 if the expression in the subscript is true, 0 otherwise.

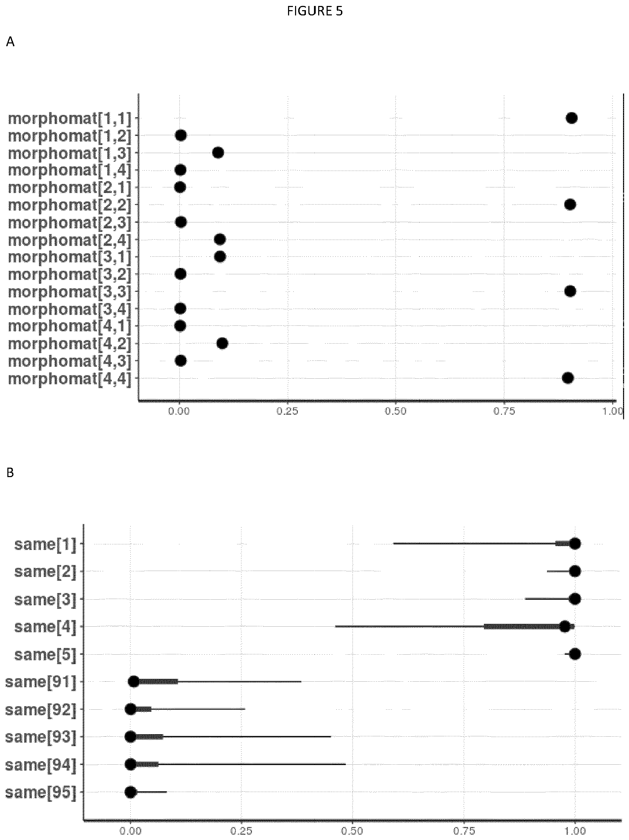

Combining likelihood with the aforementioned priors produces the elements required to conduct Bayesian inference on the unknown values. There are many ways to implement Bayesian inference including exact methods for analytically tractable posterior probability distributions as well as a range of Monte Carlo and related methods to approximate posterior distributions. In the present case, the model was implemented in the Stan modelling language (see code listing X1), which facilitates inference using Hamiltonian Monte Carlo as well as variational inference using mean-field and full-rank approximations. The variational inference approximation method used depends on stochastic gradient descent to maximize the evidence lower bound (ELBO) (Kucukelbir *et al* 2015 https://arxiv.org/abs/1506.03431), and this requires that the probability model be continuous and differentiable. To accommodate this requirement $z$ is implemented as a continuous parameter on the support [0, 1], and the Beta(0.1, 0.1) distribution is employed as a sparsifying prior to concentrate the posterior mass of $z$ around 0 and 1. This approach of employing a continuous relaxation of a discrete random variable has been called a "Concrete distribution" and is described in https://arxiv.org/abs/1611.00712. Fitting of the model to a collection of about 100 simulated sequence alignments of at least 100 bases in length using Variational Inference takes only a few minutes of CPU time on a laptop to approximate the posterior over unknown parameters and yields the posterior distribution of model parameters shown in Figure 5.

Even though variational inference is faster than many Monte Carlo methods it is not fast enough for analysing the millions of sequence reads generated in a typical sequencing run so a faster way to compute the probabilities that two reads, $r_0$ and $r_1$ either do or do not originate from the same at least one mutated target template nucleic acid molecule was developed. Given a mutagenic process and sequencing error these probabilities can be expressed as:

$$P_{same\_template}(r_0,r_1) = P(\mathbf{N},\mathbf{Q}|\mathbf{M},\mathbf{E},z=1) = \prod\nolimits_{=1} f(\mathbf{N},\mathbf{Q}|\mathbf{M},\mathbf{E},i)$$

$$(eq.\ 1)$$

$$P_{diff\_template}(r_0,r_1) = P(\mathbf{N},\mathbf{Q}|\mathbf{M},\mathbf{E},z=0) = \prod\nolimits_{=1} g(\mathbf{N},\mathbf{Q}|\mathbf{M},\mathbf{E},i)$$

$$(eq.\ 2)$$

Where the values of **M** and **E** have been fixed to *maximum a posteriori* or similar values with high posterior probability as determined by Bayesian (or Maximum Likelihood) inference using a small subset of the total data set. The values of **N and Q** are taken to correspond to the alignments of $r_0$ and $r_1$ to the reference sequence. Then, a log-odds score for two reads originating from a common template can simply be computed as:

$$score = \log(P_{same\_template}) - \log(P_{diff\_template}) \qquad (eq.\ 3)$$

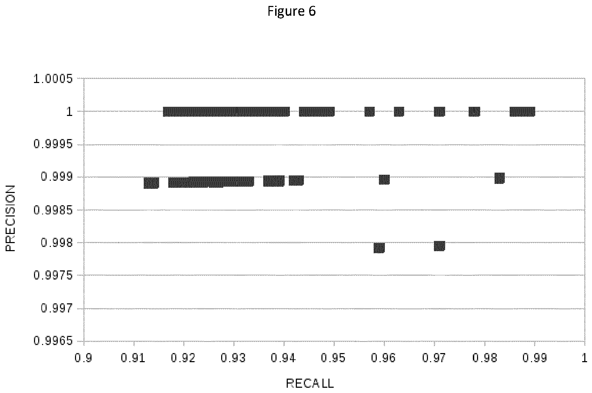

Mutated sequence reads are considered to have originated from the same at least one target template nucleic acid molecule if their pairwise score is higher than some predefined cutoff. In the present case this is set at 1,000. Tests on simulated data indicate that this log odds score can discriminate whether or not two mutated reads derive from common at least one target template nucleic acid molecules with high precision and recall (Figure 6).

Example 7 - Using two identical primer binding sites and a single primer sequence for preferential amplification of longer templates

As described above, tagmentation can be used to fragment DNA molecules and simultaneously introduce primer binding sites (adapters) onto the ends of the fragments. The Nextera tagmentation system (Illumina) utilises transposase enzymes loaded with one of two unique adapters (referred to here as X and Y). This generates a random mixture of products, some with identical end sequences (X-X, Y-Y) and some with unique ends (X-Y). Standard Nextera protocols use two distinct primer sequences to selectively amplify "X-Y" products containing different adapters on each end (as required for sequencing with Illumina technology). However, it is also possible to use a single primer sequence to amplify "X-X" or "Y-Y" fragments with identical end adapters.

To generate long mutated templates containing identical end adapters, 50 ng of high molecular weight genomic DNA (*E. coli* strain MG1655) was first subjected to tagmentation and then cleaned with SPRIselect beads as described in Example 1. This was followed by 5 cycles of "mutagenesis PCR" with a combination of standard dNTPs and dPTP, which was performed as detailed in Example 1 except that a single primer sequence was used (Table 5).

The PCR reaction was cleaned with SPRIselect beads to remove excess dPTP and primers, then subjected to a further 10 cycles of "recovery PCR" in the absence of dPTP to replace dPTP in the templates with transition mutations. Recovery PCR was performed with a single primer that anneals to the fragment ends introduced during the dPTP incorporation cycles, thereby enabling selective amplification of mutated templates generated in the previous PCR step.

Table 5:

| Primer name | Step | Sequence | | | |
|---|---|---|---|---|---|
| single_mut | mutagenesis | TCGGTCTGCGCCTCTAGC | NNN | XXXXXXXXXXXX | GTCTCGTGGGCTCGGAG |
| single_rec | recovery | CAAGCAGAAGACGGCATACGAGAT | TCGGTCTGCGCCTCTAGC | | |

**Table 5.** Primers used to generate mutated templates with the same basic adapter structure on both ends. Primer "single_mut" was used for mutagenesis PCR on DNA fragments generated by Nextera tagmentation. This primer contains a 5' portion that introduces an additional primer binding site at the fragment ends. Primer "single_rec" is capable of annealing to this site, and was used during recovery PCR to selectively amplify mutated templates generated with the single_mut primer. XXXXXXXXXXXXX is a defined, sample-specific 13nt tag sequence. NNN is a 3nt region of random nucleotides.

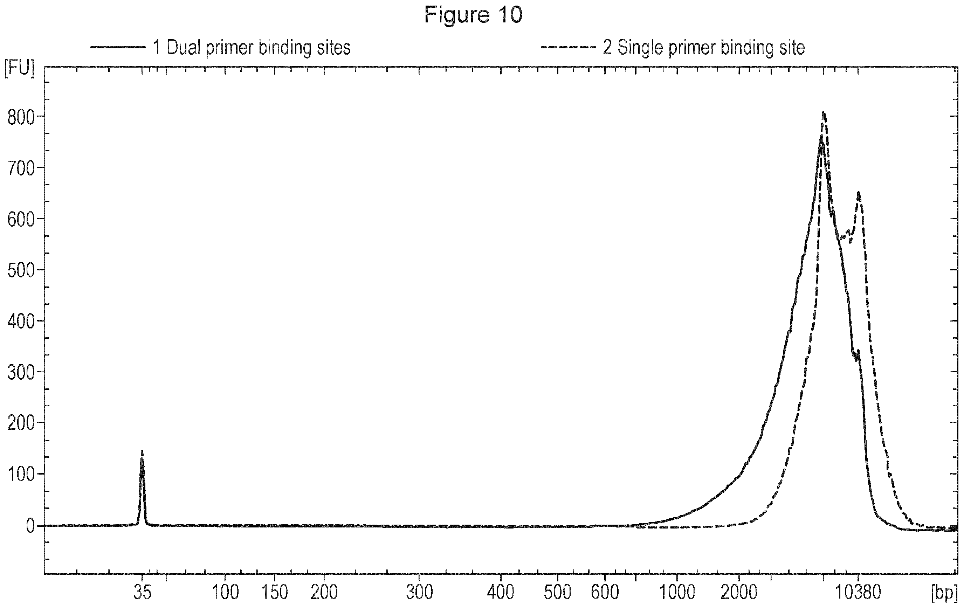

As a control, mutated templates with different adapters on each end were generated using an identical protocol to that described above, except that two distinct primer sequences were used during both mutagenesis PCR (shown in Table 2) and recovery PCR (Table 3). Final PCR products were cleaned with SPRIselect beads and analysed on a High Sensitivity DNA Chip using the 2100 Bioanalzyer System (Agilent). As shown in Figure 10, the templates generated with identical end adapters were significantly longer on average than the control sample containing dual adapters. Control templates could be detected down to a minimum size of ~800 bp, while no templates below 2000 bp were observed for the single adapter sample.

Mutated templates with identical end adapters (blue) and control templates with dual adapters were run on an Agilent 2100 Bioanalyzer (High Sensitivity DNA Kit) to compare size profiles. The use of identical end adapters inhibits the amplification of templates < 2kbp. The data is presented in Figure 10.

Example 8 - Sample dilution and end sequencing to quantitate DNA templates

An initial sample of long mutated templates for analysis was diluted down to a defined number of unique template molecules in preparation for downstream processing, sequencing and analysis to ensure that sufficient sequence data is generated per template for effective template assembly.

First, long mutated templates were prepared from human genomic DNA (genome NA12878) using the approach outlined in Example 7. Five mutagenesis PCR cycles and six recovery cycles were performed, followed by gel extraction to select templates over the size range 8-10 kb. Primers shown in Table 5 were used, generating templates flanked by identical adapter sequences.

The size selected template sample was then serially diluted in 10-fold steps, and DNA sequencing was used to determine the number of unique templates present in each dilution. This involved first amplifying the diluted samples to generate many copies of each unique template. PCR was performed with a single primer (5'-CAAGCAGAAGACGGCATACGA-3') that anneals to the fragment ends introduced during the previous recovery PCR step, thereby selectively amplifying templates that had completed the process of dPTP incorporation and replacement to generate transition mutations. A total of 16-30 PCR cycles were required (depending on the sample dilution factor) to generate enough material for downstream processing.

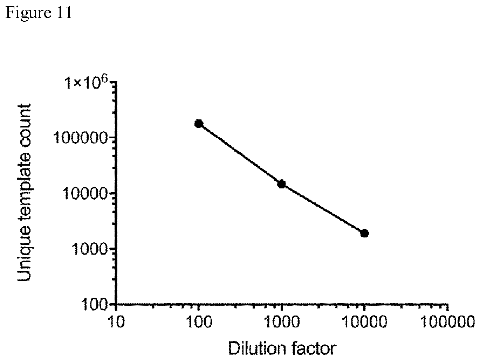

Each PCR product was then fragmented using a standard tagmentation reaction (see Example 1), and fragments derived from the template ends (including the sample tag and unique molecular tag) were selectively amplified in preparation for Illumina sequencing. This was achieved using a pair of primers, one that specifically anneals to the original template end (5'-CAAGCAGAAGACGGCATACGA-3') and one that anneals to the adapter introduced during tagmentation (i5 custom index primer; Table 2). After sequencing the samples on an Illumina MiSeq instrument, unique templates were identified based on sequence information corresponding to the extreme ends of the original template molecules. To do this, a clustering algorithm (*e.g.* vsearch) was used to group together reads with identical sequences that likely derived from the same original unique template. Other types of sequence information, such as unique molecular tags, could also be used for this purpose. As shown in Figure 11, a clear linear relationship was observed between the sample dilution factor and the observed number of unique templates. Using this information, it is possible to determine the precise dilution factor that would be required to control the number of mutated target template nucleic acid molecules in the second sample to a desired number of unique templates, in preparation for subsequent sequencing and template assembly.

Example 9 - Dilution and end sequencing to normalise pooled template samples

The sample dilution and end sequencing approach described above was used to quantitate multiple template libraries in a preliminary pooled sample. This information was subsequently used to normalise the numbers of templates between individual samples in a pooled sample.

First, genomic DNA samples from 96 different bacterial strains were subjected to tagmentation and 5 cycles of mutagenesis PCR as outlined in Example 5, using a single primer with a unique sample tag for each reaction (single_mut design; Table 5). Equal volumes of each sample tagged mutagenesis product were then pooled, and the pooled sample was cleaned with SPRIselect beads to remove excess dPTP and primers. This was followed by 6 cycles of recovery PCR using the single_rec primer (Table 5) and gel extraction to select templates over the size range 8-10 kb. The pooled template sample was then diluted 1 in 1000, and end sequencing was performed to determine the number of unique templates present for each bacterial strain in the diluted pool. This was achieved using the approach outlined in Example 7.

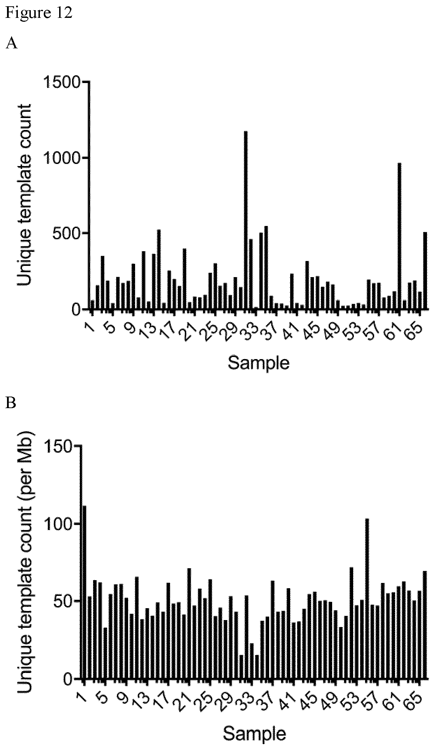

Template counts were found to be highly variable between strains in the diluted pool, ranging from no detectable templates for several strains to over 1000 unique templates for others. Sixty six strains with non-zero template counts were selected for normalisation. Based on the observed template count and the known genome size of each strain, a normalised pool was prepared by combining different volumes of the sample tagged mutagenesis PCR products, aiming to achieve a constant number of unique templates per unit of genome content (*e.g.* per Mb) for each strain. The normalised pool was then processed for end sequencing as described above, and the number of unique templates per strain was determined. As expected, template counts were far less variable between strains following normalisation (Figure 12).

Example 10 - Utilisation of assembly algorithm to assemble bacterial genome sequences *Bacterial strains and DNA preparation*

DNA from 62 bacterial strains was obtained from BEI resources. These strains are isolates that were sequenced as part of the Human Microbiome Project. They represent a range of GC contents (25% to 69%) and further details are provided in Table 6.

Table 6

| Morphoseq index | Strain number | Name | Phylum | Estimated genome size | GC content |
|---|---|---|---|---|---|
| A02 | HM-119 | Staphylococcus hominis, Strain SK119 Staphylococcus hominis | Firmicutes | 2,226,236 | 0.31 |
| A03 | HM-209 | Propionibacterium propionicum, Oral Taxon 739, Strain F0230 | Actinobacteria | 3,449,360 | 0.66 |
| A04 | HM-214 | Pseudomonas sp., Strain 2_1_26 | Proteobacteria | 6,447,478 | 0.66 |
| A05 | HM-466 | Staphylococcus aureus, Strain MRSA131 | Firmicutes | 2,817,572 | 0.32 |
| A06 | HM-118 | Staphylococcus epidermidis, Strain SK135 | Firmicutes | 2,518,045 | 0.32 |
| A07 | ATCC 25923 | Staphylococcus aureus, Strain ATCC 25923 | Firmicutes | 2,778,854 | 0.33 |
| A09 | HM-109 | Corynebacterium amycolatum, Strain SK46 | Actinobacteria | 2,513,912 | 0.59 |
| A10 | HM-200 | Enterococcus faecalis, Strain HH22 | Firmicutes | 3,129,930 | 0.37 |
| A11 | HM-201 | Enterococcus faecalis, Strain TX0104 | Firmicutes | 3,156,478 | 0.37 |
| A12 | HM-343 | Escherichia coli, Strain MS 110-3 | Proteobacteria | 5,071,839 | 0.5 |
| B01 | HM-345 | Escherichia coli, Strain MS 16-3 | Proteobacteria | 4,982,157 | 0.51 |
| B02 | HM-153 | Lachnospiraceae sp., Strain 7_1_58FAA | Firmicutes | 5,668,091 | 0.58 |
| B03 | HM-169 | Parabacteroides distasonis, Strain 31_2 | Bacteroidetes | 4,887,873 | 0.45 |
| B04 | HM-77 | Parabacteroides sp., Strain D13 | Bacteroidetes | 5,370,710 | 0.45 |
| B05 | HM-567 | Peptoniphilus sp., Oral Taxon 375, Strain F0436 | Firmicutes | 1,950,550 | 0.35 |
| B07 | DS2 | Haloferax volcanii, Strain DS2 | Euryarchaeota | 4,773,000 | 0.67 |
| B08 | HM-20 | Bacteroides fragilis, Strain 3_1_12 | Bacteroidetes | 5,530,115 | 0.44 |
| B10 | HM-267 | Capnocytophaga sp. Oral Taxon 329, Strain F0087 | Bacteroidetes | 2,536,778 | 0.4 |
| B11 | HM-34 | Citrobacter sp., Strain 30_2 | Proteobacteria | 5,023,211 | 0.52 |
| C03 | HM-298 | Arcobacter butzleri, Strain JV22 | Proteobacteria | 2,302,726 | 0.27 |
| C04 | HM-210 | Bacteroides eggerthii, Strain 1_2_48FAA | Bacteroidetes | 4,611,535 | 0.45 |
| C05 | HM-222 | Bacteroides ovatus, Strain 3_8_47FAA | | 6,549,476 | |
| C06 | HM-272 | Streptococcus gallolyticus subsp. gallolyticus, Strain TX20005 | | 2,246,969 | |

(continued)

| Morphoseq index | Strain number | Name | Phylum | Estimated genome size | GC content |
|---|---|---|---|---|---|
| C08 | HM-463 | Enterococcus faecium, Strain TX0133a04 | Firmicutes | 2,922,651 | 0.38 |
| C09 | HM-204 | Enterococcus faecium, Strain TX1330 | Firmicutes | 2,777,972 | 0.38 |
| C10 | HM-293 | Finegoldia magna, Strain SY01 | | 2,032,717 | |
| C11 | HM-44 | Klebsiella sp., Strain 1_1_55 | Proteobacteria | 5,459,739 | 0.58 |
| D03 | HM-104 | Lactobacillus gasseri, Strain JV-V03 | Firmicutes | 2,011,855 | 0.35 |
| D05 | HM-87 | Shigella sp., Strain D9 | Proteobacteria | 4,764,345 | 0.51 |
| D06 | HM-102 | Lactobacillus reuteri, Strain CF48-3A | | 2,107,903 | |
| D07, D12 | MG1655 | Escherichia coli, Strain MG1655 | | 4,653,240 | |
| D08 | HM-23 | Bacteroides sp., Strain 1_1_6 | Bacteroidetes | 6,760,735 | 0.43 |
| D09 | HM-296 | Campylobacter coli, Strain JV20 | Proteobacteria | 1,705,064 | 0.31 |
| E01 | HM-242 | Neisseria mucosa, Strain C102 | Proteobacteria | 2,169,437 | 0.5 |
| E02 | HM-308 | Clostridium hathewayi, Strain WAL-18680 | | 5,697,783 | |
| E04 | HM-147 | Actinomyces cardiffensis, Strain F0333 | Actinobacteria | 2,214,851 | 0.61 |
| E05 | HM-94 | Actinomyces odontolyticus, Strain F0309 | Actinobacteria | 2,431,995 | 0.65 |
| E06 | HM-90 | Actinomyces sp., Oral Taxon 848, Strain F0332 | | 2,520,418 | |
| E07 | HM-238 | Actinomyces viscosus, Strain C505 | Actinobacteria | 3,134,496 | 0.69 |
| E08 | HM-30 | Bifidobacterium sp., Strain 12_1_47BFAA | | 2,405,990 | |
| E09 | HM-297 | Campylobacter upsaliensis, Strain JV21 | Proteobacteria | 1,649,151 | 0.35 |
| E10 | HM-299 | Citrobacter freundii, Strain 4_7_47CFAA | | 5,122,674 | |
| F01 | HM-318 | Clostridium bolteae, Strain WAL-14578 | | 6,604,884 | |
| F03 | HM-306 | Clostridium clostridioforme, Strain 2_1_49FAA | Firmicutes | 5,500,475 | 0.49 |
| F04 | HM-316 | Clostridium citroniae, Strain WAL-19142 | | 6,252,818 | |
| F05 | HM-317 | Clostridium clostridioforme, Strain WAL-7855 | Firmicutes | 5,459,495 | 0.49 |
| F06 | HM-287 | Clostridium sp., Strain HGF2 | Firmicutes | 4,099,852 | 0.44 |
| F08 | HM-173 | Clostridium innocuum, Strain 6_1_30 | | | |
| F09 | HM-303 | Clostridium orbiscindens, Strain 1_3_50AFAA | Firmicutes | 4,383,642 | 0.61 |
| F10 | HM-310 | Clostridium perfringens, Strain WAL-14572 | Firmicutes | 3,466,039 | 0.28 |
| F11 | HM-36 | Clostridium sp., Strain 7_2_43FAA | | | |
| G01 | HM-746 | Clostridium difficile, Strain 002-P50-2011 | | 4,103,061 | |
| G04 | HM-51 | Enterococcus faecalis, Strain TUSoD Ef11 | Firmicutes | 2,836,650 | 0.38 |

(continued)

| Morphoseq index | Strain number | Name | Phylum | Estimated genome size | GC content |
|---|---|---|---|---|---|
| G06 | HM-50 | Escherichia coli, Strain 83972 | Proteobacteria | 5,106,156 | 0.51 |
| G07 | HM-337 | Escherichia coli, Strain MS 85-1 | | | |
| G08 | HM-38 | Escherichia sp., Strain 3 2 53FAA | Proteobacteria | 5,153,453 | 0.51 |
| G09 | HM-644 | Lactobacillus gasseri, Strain MV-22 | Firmicutes | 1,930,436 | 0.35 |
| G10 | HM-105 | Lactobacillus jensenii, Strain JV-V16 | Firmicutes | 1,604,632 | 0.34 |
| G12 | HM-125 | Mobiluncus mulieris, Strain UPII 28-I | Actinobacteria | 2,452,380 | 0.55 |
| H01 | HM-91 | Neisseria sp., Oral Taxon 014, Strain F0314 | | 2,515,760 | |
| H02 | HM-480 | Stomatobaculum longum (Deposited as Lachnospiraceae sp.), Strain ACC2 | Firmicutes | 2,313,632 | 0.55 |
| H07 | HM-130 | Porphyromonas uenonis, Strain UPII 60-3 | Bacteroidetes | 2,242,885 | 0.52 |
| H10 | HM-137 | Prevotella buccalis, Strain CRIS 12C-C (ATCC 35310) | Bacteroidetes | 3,033,961 | 0.45 |
| H11 | HM-80 | Prevotella melaninogenica, Strain D18 | Bacteroidetes | 3,292,341 | 0.41 |
| H12 | HM-158 | Ralstonia sp., Strain 5_2_56FAA | | 5,254,771 | |

Three additional strains with well characterised genomes, also covering a wide range of GC contents, were included as controls (*Escherichia coli* K12 MG1655, *Staphylococcus aureus* ATCC 25923, and *Haloferax volcanii* DS2). DNA was prepared from these strains using the Qiagen DNeasy UltraClean Microbial Kit according to the manufacturer's instructions, with the following changes. Overnight cultures (20 mL for each strain) were centrifuged at 3200g for 5 min to obtain a cell pellet, and each pellet washed with 5 mL sterile 0.9% sodium chloride solution. Each pellet was resuspended in 300ul PowerBead solution before continuing with the manufacturers protocol. DNA was eluted with 50uL elution buffer pre-warmed to 42°C for *E. coli* and *S. aureus,* while *H. volcanii* DNA was eluted in 35uL elution buffer.

DNA concentrations for all samples were measured using the Quant-iT PicoGreen dsDNA kit (Thermo Scientific). For a subset of species, DNA purity and molecular weight was also assessed via Nanodrop (Thermo Scientific) spectrophotometry and agarose gel electrophoresis.

*Morphoseq library preparation*

*Tagmentation to generate long fragments*

DNA from each bacterial genome was arrayed into a 96 well plate, and the concentration normalised to 10ng/ul. *E. coli* MG1655 DNA was included in two independent wells to provide an internal control for sample processing and downstream data analysis.

Tagmentation was performed using Nextera DNA Tagment Enzyme (TDE1; Illumina) that had been diluted 1 in 50 in storage buffer (5 mM Tris-HCl [pH 8.0], 0.5 mM EDTA, 50% (v/v) glycerol). For each sample, a 16 $\mu$l tagmentation reaction was prepared containing 50 ng DNA and 4 $\mu$l of diluted TDE1 in 1X tagmentation buffer (10mM Tris-HCl [pH7.6], 10mM MgCl, 10% (v/v) dimethylformamide. Each reaction was incubated at 55°C for 5 mins, then cooled to 10°C. SDS was added to a final concentration of 0.04%, and the reactions incubated for a further 15 minutes at 25°C. Reactions were subject to a left-side clean up using SPRIselect magnetic beads (Beckman Coulter) with 0.6X volume of beads, and eluted in 20 $\mu$l molecular grade water following the manufacturer's instructions.

*Mutagenesis of long DNA fragments*

A PCR to incorporate the mutagenic nucleotide analogue dPTP was performed as follows. 5 $\mu$l of each cleaned tagmentation reaction above was used as template in a 25 $\mu$l PCR reaction containing 0.625 U PrimeStar GXL polymerase, 1X Primestar GXL buffer and 0.2 mM dNTPs (all obtained from Takara), along with 0.5 mM dPTP (TriLink Biotechnologies) and 0.4 mM Morphoseq index primer (see Table 7; unique index for each sample). A single primer was used during the mutagenesis PCR to amplify templates containing the same Nextera tagmentation adapter sequence on both ends. Reactions were subject to the following cycling conditions: 68°C for 3 minutes, followed by 5 cycles of 98°C for 10 seconds, 55°C for 15 seconds and 68°C for 10 minutes.

At this point, equal volumes of each reaction (4 $\mu$l) were combined into a single pool, and the pool subject to a further SPRIselect left-sided bead clean using 0.6X volume of beads. The purified pool was eluted in 45 $\mu$l of molecular grade water and quantified using the Qubit dsDNA HS assay kit (Thermo Fisher Scientific).

The pooled sample of dPTP-containing templates was then further amplified in the absence of dPTP, thereby replacing the nucleotide analogue with natural dNTPs and generating transition mutations through the ambivalent base-pairing properties of dPTP. This "recovery" PCR contained 1.25 U PrimeStar GXL polymerase, 1X Primestar GXL buffer and 0.2 mM dNTPs (Takara), along with 0.4 $\mu$M recovery primer (see Table 7) and 10 ng of the pooled template sample in a total volume of 50 $\mu$l. The reaction was subject to 6 cycles of 98°C for 10 seconds, 55°C for 15 seconds and 68°C for 10 minutes.

*Long template size selection*

The recovery PCR product was size selected to remove unwanted short fragments using a DNA gel electrophoresis approach. 25 $\mu$l of the recovery PCR reaction, along with DNA size standards, was loaded onto a 0.9% agarose gel and run in 1X TBE buffer overnight (900 minutes) at 18V. A gel slice corresponding to the 8-10kb size region was excised, and DNA extracted using the Wizard SV Gel and PCR Clean-Up kit (Promega), as per the manufacturer's instructions. Size selected DNA was quantified using the Qubit dsDNA HS assay kit (Thermo Fisher Scientific), and the size range confirmed using a Bioanalyzer high sensitivity DNA chip (Agilent).

*Template normalisation and quantitation*

The following approach was used to assess the abundance of templates among individual sample tagged samples within the pooled and size-selected product. First, the size selected DNA was diluted to 0.1 pg/$\mu$l and 2 $\mu$l of the dilution (0.2 pg) was used as input for an enrichment PCR to make many copies of each unique template. Preliminary experiments showed that this level of dilution constrained the diversity of unique templates enough to allow accurate template quantitation from the sequence output of a single Illumina MiSeq run. The 50 $\mu$l enrichment PCR also contained 1.25 U PrimeStar GXL polymerase, 1X Primestar GXL buffer and 0.2 mM dNTPs (Takara), along with 0.4 $\mu$M enrichment primer (see Table 7). The enrichment primer was designed to anneal to fragment end adapters introduced during the previous recovery PCR step, thereby selectively amplifying templates that had completed the process of dPTP incorporation and replacement to generate transition mutations. The reaction was subject to 22 cycles of 98°C for 10 seconds, 55°C for 15 seconds and 68°C for 10 minutes, followed by purification via a SPRIselect left-sided bead clean using 0.6X volume of beads, and elution into 20 $\mu$l of molecular grade water. The sample was then quantified using using the Qubit dsDNA HS assay kit (Thermo Fisher Scientific), and the size range confirmed using a Bioanalyzer high sensitivity DNA chip (Agilent).

Next, the full-length enrichment product was fragmented via a second tagmentation reaction, and fragments derived from the original template ends (including sample barcodes) were amplified for Illumina sequencing. Tagmentation was performed as described above for long template generation, except that 2 ng rather than 50 ng of starting DNA was used. Following SDS treatment, an end library PCR reaction was prepared by adding KAPA HiFi HotStart ReadyMix (Kapa Biosystems) to a final concentration of 1X, along with 0.23 $\mu$M enrichment primer (which anneals to the Illumina p7 flow cell adapter located at the extreme end of the full-length template) and 0.23 $\mu$M custom i5 index primer (which anneals to an internal adapter introduced during the second round of tagmentation; see Table 7). The reaction was cycled as follows; 72°C for 3 minutes, 98°C for 30 seconds, 12 cycles of 98°C for 15 seconds, 55°C for 30 seconds and 72°C for 30 seconds, followed by a final extension at 72°C for 5 minutes. The end library was then purified and quantitated as described above for the full-length enrichment product.

Illumina sequencing was performed on a MiSeq using V3 chemistry and 2 x 75 nt paired-end reads were generated. Unique template counts were determined for each individual bacterial genome sample in the diluted pool by first demultiplexing the end-read data based on the index 1 (i7) read sequence, then mapping read 2 sequences (corresponding to the extreme end of the original genomic insert) to the publically-available reference genomes for each strain. The number of unique templates was calculated by counting the number of unique mapping start sites (corresponding to the start or end of a template), noting that two sites are expected per template.

Observed template counts varied for individual genomes in the diluted pool, ranging from no detectable templates for several samples to over 1000 unique templates for others. For simplicity, 66 samples with non-zero template counts were chosen for further processing, sequencing and assembly. Based on the observed template count and known genome size for each of these samples, a normalised pool was prepared by combining different volumes of the original barcoded mutagenesis PCR products, aiming to achieve a constant number of unique templates per unit of genome content (e.g. per Mb) for each strain. To verify that normalisation had been successful, the normalised pool was further processed for template quantitation by repeating all subsequent stages of library preparation and sequencing described above (recovery PCR, size selection, template dilution and enrichment, end library preparation, Illumina sequencing and analysis). As expected, template counts were far less variable between strains following normalisation (Figure 11).

*Template bottlenecking, enrichment and short-read library processing*

Based on the template quantitation data from the normalised sample pool, as well as the known size of long fragments, we selected a target of 1.5 million total unique templates to process for Morphoseq sequencing and assembly. This would ensure a theoretical longtemplate coverage of at least 20x per individual genome (up to 90x). To this end, a final long template sample was prepared by diluting the size-selected recovery PCR product from the previous step to 0.75 million templates/$\mu$l and using 2 $\mu$l of the dilution as input for an enrichment PCR to make many copies of each unique template. Enrichment PCR was carried out as described above, except that 16 rather than 22 amplification cycles were performed.

To process the final long template sample for short-read (Illumina) sequencing, a barcoded end library was first prepared, purified and quantitated according to the method outlined in the previous section. A second library was also prepared, containing randomly generated internal fragments from the long templates, using the Nextera DNA Flex Library Prep Kit (Illumina) with some modifications to the manufacturer's protocol. Specifically, the BLT (Bead-Linked Transposomes) reagent was diluted 1 in 50 in molecular grade water and 10 $\mu$l of this diluted solution was used in a tagmentation reaction with 10 ng of long template DNA. Twelve cycles of library amplification were performed, using custom i5 and i7 index primers (Table 7) rather than the standard Illumina adapters.

*Preparation of unmutated reference libraries*

Reference libraries were generated for all 66 genomes included in the final Morphoseq pool. Using 10 ng of genomic DNA as input, library preparation was performed according to the procedure outlined above for internal Morphoseq libraries but with further modifications to the Nextera DNA Flex method. Specifically, the Illumina TB1 buffer was replaced with custom tagmentation buffer (see earlier), KAPA HiFi HotStart ReadyMix (1x final concentration; Kapa Biosystems) was used in place of the kit polymerase, and the Illumina Sample Purification Beads (SPB) were substituted with SPRIselect magnetic beads (Beckman Coulter). Thermal cycling conditions for reference library amplification were as follows; 72°C for 3 minutes, 98°C for 30 seconds, 12 cycles of 98°C for 15 seconds, 55°C for 30 seconds and 72°C for 30 seconds, followed by a final extension at 72°C for 5 minutes.

To normalise the reference libraries, equal volumes of each sample were first combined and the pooled library was sequenced using a MiSeq Reagent Nano Kit (Illumina), generating 2 x 150 nt paired-end reads with MiSeq V2 chemistry. Read counts were determined for each individual genome by demultiplexing the resulting sequence data. These counts were then used to prepare a normalised pool by combining different volumes of each original reference library, aiming to achieve equal coverage per genome.

*Illumina sequencing*

A final sample was prepared for Illumina sequencing by combining the normalised reference pool, the morphoseq end library and the morphoseq internal library at a molar ratio of 1:1:20 respectively. Sequencing was conducted at the Ramaciotti Centre for Genomics at the University of New South Wales (Sydney, Australia), using a NovaSeq 6000 instrument and an S1 flow cell to generate 2 x 150 nt paired-end reads.

*Assembly of bacterial genomes*

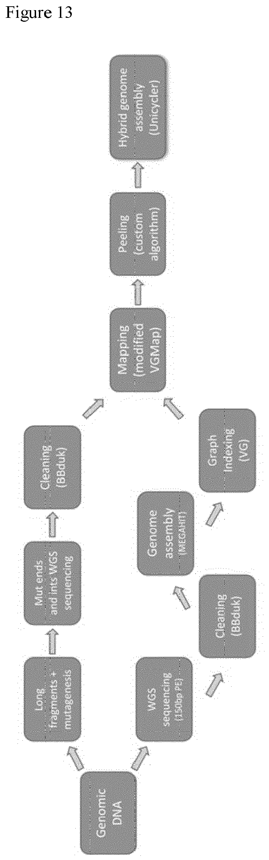

An overview of the workflow for assembly of bacterial genomes is represented in Figure 13.

*Non-mutated reference assemblies*

Genomes of each bacterial strain were assembled from non-mutated, paired-end 150 base pair reads. Initial quality filtering to remove low quality sequences and trim library adaptors was performed with bbduk v36.99. Reads were demultiplexed using a custom python script and assembled using MEGAHIT v1.1.3 with custom parameters: prunelevel=3, low-local-ratio=0.1 and max-tip-len=280 which were chosen to reduce the complexity of the resulting genome graphs, and facilitate better mapping of the mutated sequences in the next stage (described below). The resulting graphical fragment assembly (gfa file) was used an input to VG (index) v1.14.0 to create an index suitable for mapping.

The resulting graph is referred to as the "indexed un-mutated reference assembly graph" or just the "indexed graph".

*Generation of synthetic long reads (morphoreads)*

Mutated reads from each End library (end reads) and the pooled Internal library (int reads) were mapped to their corresponding indexed VG bacterial genome assembly using VG (map) v1.14.0 with default parameters to produce a pair of graphical alignment map (GAM) files for each sample. Data from each sample's GAM pair was combined with information from the corresponding un-mutated reference assembly, processed using a custom tool and stored in a HDF5 formatted database that facilitates parallel processing for many of the remaining steps that reconstruct the sequence of the original templates. The morphoread generation process consists of three main stages: "end-wall identification", "seeding", and "extending".

The nature of the processes used to fragment the target DNA into long fragments and to generate final short read libraries creates a situation where the sequences at the very end of any original templates will only be found in the second read of a paired Illumina library. When these reads are mapped to a reference genome they will appear to pile up suddenly at locations corresponding to the ends of the original long DNA templates. These locations are referred to as "end walls" and are identified by finding groups of end and int reads that map to identical positions in the reference assembly. Any site which has at least five end reads mapping in the pattern described above are marked as end walls. Int reads are used to augment the mapping count at sites that have between two and four mapping end reads and if the total augmented count is at least five then these sites also marked as end walls.

End walls dictate the locations in the reference assembly where the algorithm will begin constructing synthetic long reads, however it is possible to have single end walls that correspond to more than one of the original DNA templates whenever 2 or more templates have identical start or end locations. Each DNA template will have a unique pattern of mutations and so the reads originating from a given template will contain subsets of its pattern which will appear as transition mismatches in the VG mapping. The "seeding" stage analyses these mutation patterns in the end and int reads at each end wall, clusters reads with like patterns together and creates a single short (400-600 bp) morphoread instance for each cluster. Each morphoread instance includes a directed acyclic graphbased representation of the mapped mutated reads it contains called a "consensus graph". The structure of the consensus graph roughly corresponds to a subgraph of the indexed graph and the positions of the reads in the consensus graph correspond to the mapping positions of the reads against the indexed graph. The main differences between the consensus graph and the subgraph of the indexed graph it corresponds to are that edges between nodes in the consensus graph represent the paths of mapped reads through the indexed graph and whenever such a path follows a loop in indexed graph the nodes in that loop are duplicated, effectively rolling out the loop in the indexed graph removing any cycles. Thus individual nodes in the indexed graph correspond to potentially multiple nodes in the consensus graph and the edges in the consensus graph often, but not always correspond to the edges in the indexed graph. The consensus graph stores information about the indexed assembly and the mapped mutated reads so it can be used to create a "consensus sequence" that corresponds to a path through the indexed graph (ie. does not contain any mutations) and a "mutation set" containing a consensus of mutation patterns found in all included int and end reads.

During the "extending" stage the algorithm walks along the consensus graph starting from the end wall and iteratively adds end and int reads to the morphoread if they match the consensus sequence (>90% identity, >=100 bp overlap), and their mutation pattern shares at least 3 mutations with the mutation set, and contains no more than five mutations differing from the mutation set. The high number of differing mutations is needed to reduce the effects of errors in individual reads masquerading as mutations and also because reads that are tested for inclusion to the morphoread could map to nodes that extend beyond the end of the current consensus graph and may contain mutations not yet included in the morphoread's mutation set. Each time a new read is included in the morphoread new nodes can be added to the consensus graph and hence the consensus fragment can become longer. The algorithm continues to walk along the extending consensus graph until an end read is incorporated into morphoread indicating that the distal end of the original long DNA template has been reached or no reads can be found that could be used to continue extending. The final consensus fragment for each morphoread is written to a FASTA file and all morphoreads shorter than 500bp are discarded. The algorithm also produces a BAM file containing the positions of the included end and int reads wrt to the consensus sequence and some summary statistics for each morphoread.

*Hybrid Genome assembly*

High quality morphoreads along with unmutated reference reads were combined in hybrid genome assemblies using Unicycler v0.4.6 with default parameters.

*Results*

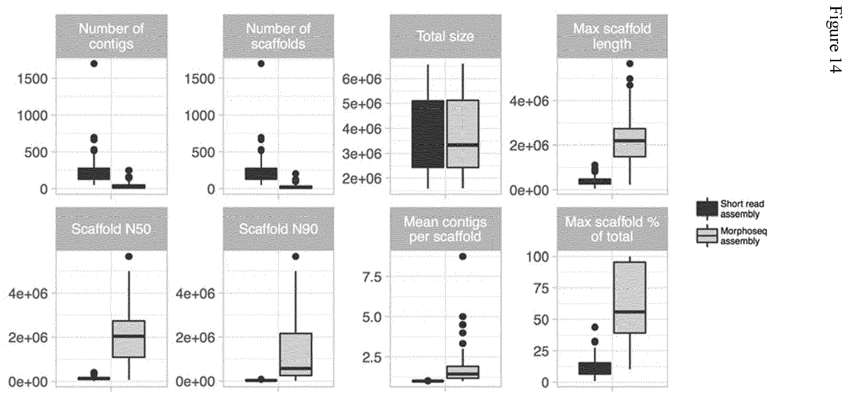

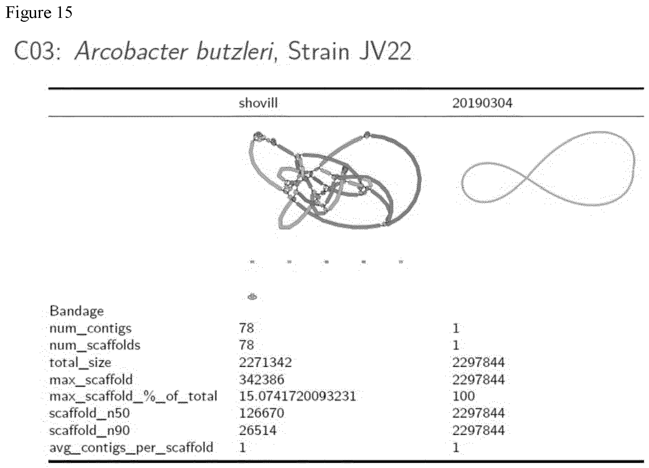

The Morphoseq method consistently produced assemblies with significantly fewer and larger scaffolds (Kruskal Wallis, $p < 0.001$) than the short read only assemblies (Figure 14). For Morphoseq and short read only assemblies respectively, the median maximum scaffold length as a percentage of genome size was 55.84% vs 10.15%, and the median number of scaffolds was 17 vs 192. Exemplary assembly metrics for a bacterial genome can be found in Figure 15.

Table 7

| Primer | Sequence[a] | Protocol step[b,c] |
|---|---|---|
| Morphoseq_index_A1 | TCGGTCTGCGCCTCTAGCNNN**CTCTATCGACGTAG**TCTCGTGGGCTCGGAG | Mutagenesis |
| Morphoseq_index_A2 | TCGGTCTGCGCCTCTAGCNNNT**AAGTCTGGTCTA**GTCTCGTGGGCTCGGAG | |
| Morphoseq_index_A3 | TCGGTCTGCGCCTCTAGCNNN**ACCTGCGTAACC**TGTCTCGTGGGCTCGGAG | |
| Morphoseq_index_A4 | TCGGTCTGCGCCTCTAGCNNN**CGTCTCTAGGATG**GTCTCGTGGGCTCGGAG | |
| Morphoseq_index_A5 | TCGGTCTGCGCCTCTAGCNNNT**CATTAGGTATA**TGTCTCGTGGGCTCGGAG | |
| Morphoseq_index_A6 | TCGGTCTGCGCCTCTAGCNNN**AAGTATTCCATGA**GTCTCGTGGGCTCGGAG | |
| Morphoseq_index_A7 | TCGGTCTGCGCCTCTAGCNNNTTCT**GGTACTTCA**GTCTCGTGGGCTCGGAG | |
| Morphoseq_index_A8 | TCGGTCTGCGCCTCTAGCNNN**ATGCCTCCTGC**TTGTCTCGTGGGCTCGGAG | |
| Morphoseq_index_A9 | TCGGTCTGCGCCTCTAGCNNNT**GGTAATACGCC**TGTCTCGTGGGCTCGGAG | |
| Morphoseq_index_A10 | TCGGTCTGCGCCTCTAGCNNN**ACTGACGATTGG**TGTCTCGTGGGCTCGGAG | |
| Morphoseq_index_A11 | TCGGTCTGCGCCTCTAGCNNNT**TAGAGTAGTTGCG**TCTCGTGGGCTCGGAG | |
| Morphoseq_index_A12 | TCGGTCTGCGCCTCTAGCNNN**AAGCCGTTGAATA**GTCTCGTGGGCTCGGAG | |
| Morphoseq_index_B1 | TCGGTCTGCGCCTCTAGCNNN**TAGCCTCGCTCTCGTC**TCGTGGGCTCGGAG | |
| Morphoseq_index_B2 | TCGGTCTGCGCCTCTAGCNNN**CTTGGCCTTGCAA**GTCTCGTGGGCTCGGAG | |
| Morphoseq_index_B3 | TCGGTCTGCGCCTCTAGCNNN**CTATCTTCAACTG**GTCTCGTGGGCTCGGAG | |
| Morphoseq_index_B4 | TCGGTCTGCGCCTCTAGCNNN**ATCCATACGGAC**TGTCTCGTGGGCTCGGAG | |
| Morphoseq_index_B5 | TCGGTCTGCGCCTCTAGCNNN**CGCTCGCTCATA**TGTCTCGTGGGCTCGGAG | |
| Morphoseq_index_B6 | TCGGTCTGCGCCTCTAGCNNN**CGTATCGAATTCA**GTCTCGTGGGCTCGGAG | |
| Morphoseq_index_B7 | TCGGTCTGCGCCTCTAGCNNN**ATTCTTCTCGGTA**GTCTCGTGGGCTCGGAG | |
| Morphoseq_index_B8 | TCGGTCTGCGCCTCTAGCNNN**CAAGTTGCAGCAG**GTCTCGTGGGCTCGGAG | |
| Morphoseq_index_B9 | TCGGTCTGCGCCTCTAGCNNN**ACTAATCTGGTAC**GTCTCGTGGGCTCGGAG | |
| Morphoseq_index_B10 | TCGGTCTGCGCCTCTAGCNNN**CAGGAAGATTAGTG**TCTCGTGGGCTCGGAG | |
| Morphoseq_index_B11 | TCGGTCTGCGCCTCTAGCNNN**AATAACTAGCTTG**GTCTCGTGGGCTCGGAG | |
| Morphoseq_index_B12 | TCGGTCTGCGCCTCTAGCNNN**TACGACTTACTAA**GTCTCGTGGGCTCGGAG | |
| Morphoseq_index_C1 | TCGGTCTGCGCCTCTAGCNNN**CTCGGCTTCTCC**TGTCTCGTGGGCTCGGAG | |

| Primer | Sequence[a] | Protocol step[b,c] |
|--------|-------------|---------------------|
| Morphoseq_index_C2 | TCGGTCTGCGCCTCTAGCNNN**TTCCTCTCTATCA**GTCTCGTGGGCTCGGAG | |
| Morphoseq_index_C3 | TCGGTCTGCGCCTCTAGCNNN**ATGGATTCCTAGA**GTCTCGTGGGCTCGGAG | |
| Morphoseq_index_C4 | TCGGTCTGCGCCTCTAGCNNN**TTCTTGAGTAAGG**GTCTCGTGGGCTCGGAG | |
| Morphoseq_index_C5 | TCGGTCTGCGCCTCTAGCNNN**ACTACTACGAAGG**GTCTCGTGGGCTCGGAG | |
| Morphoseq_index_C6 | TCGGTCTGCGCCTCTAGCNNN**CATCGCTATCG**TTGTCTCGTGGGCTCGGAG | |
| Morphoseq_index_C7 | TCGGTCTGCGCCTCTAGCNNN**AAGTTCCGCATTA**GTCTCGTGGGCTCGGAG | |
| Morphoseq_index_C8 | TCGGTCTGCGCCTCTAGCNNN**ACTTAAGTTGAAG**GTCTCGTGGGCTCGGAG | |
| Morphoseq_index_C9 | TCGGTCTGCGCCTCTAGCNNN**TGAGTAATTCGAC**GTCTCGTGGGCTCGGAG | |
| Morphoseq_index_C10 | TCGGTCTGCGCCTCTAGCNNN**AGCTGAAGACTTA**GTCTCGTGGGCTCGGAG | |
| Morphoseq_index_C11 | TCGGTCTGCGCCTCTAGCNNN**CAAGGATAGAA**TTGTCTCGTGGGCTCGGAG | |
| Morphoseq_index_C12 | TCGGTCTGCGCCTCTAGCNNN**AGCATGATTGCGG**GTCTCGTGGGCTCGGAG | |
| Morphoseq_index_D1 | TCGGTCTGCGCCTCTAGCNNN**ACCTGAAGCTGC**TGTCTCGTGGGCTCGGAG | |
| Morphoseq_index_D2 | TCGGTCTGCGCCTCTAGCNNN**CATATGGTAACG**TGTCTCGTGGGCTCGGAG | |
| Morphoseq_index_D3 | TCGGTCTGCGCCTCTAGCNNN**ATGGAATACGCGG**GTCTCGTGGGCTCGGAG | |
| Morphoseq_index_D4 | TCGGTCTGCGCCTCTAGCNNN**TCTATTACTCTCA**GTCTCGTGGGCTCGGAG | |
| Morphoseq_index_D5 | TCGGTCTGCGCCTCTAGCNNN**TCGATTACTCAAG**GTCTCGTGGGCTCGGAG | |
| Morphoseq_index_D6 | TCGGTCTGCGCCTCTAGCNNN**CTGCTTATATTCA**GTCTCGTGGGCTCGGAG | |
| Morphoseq_index_D7 | TCGGTCTGCGCCTCTAGCNNN**TATGCCATCTAG**TGTCTCGTGGGCTCGGAG | |
| Morphoseq_index_D8 | TCGGTCTGCGCCTCTAGCNNN**AATGCTTGAATGG**GTCTCGTGGGCTCGGAG | |
| Morphoseq_index_D9 | TCGGTCTGCGCCTCTAGCNNN**ACGTTCAGGAGA**TGTCTCGTGGGCTCGGAG | |
| Morphoseq_index_D10 | TCGGTCTGCGCCTCTAGCNNN**TCTTCCTAGCTTA**GTCTCGTGGGCTCGGAG | |
| Morphoseq_index_D11 | TCGGTCTGCGCCTCTAGCNNN**AAGTCGGATCATG**GTCTCGTGGGCTCGGAG | |
| Morphoseq_index_D12 | TCGGTCTGCGCCTCTAGCNNN**CAGAACCGGAAGA**GTCTCGTGGGCTCGGAG | |
| Morphoseq_index_E1 | TCGGTCTGCGCCTCTAGCNNN**ATGCTGGCTCTCG**GTCTCGTGGGCTCGGAG | |
| Morphoseq_index_E2 | TCGGTCTGCGCCTCTAGCNNN**TGGCCTGATGAAC**GTCTCGTGGGCTCGGAG | |

(continued)

| Primer | Sequence[a] | Protocol step[b,c] |
|---|---|---|
| Morphoseq_index_E3 | TCGGTCTGCGCCCTCTAGCNNNNAATGGACGCCAAGGTCTCGTGGGCTCGGAG | |
| Morphoseq_index_E4 | TCGGTCTGCGCCCTCTAGCNNNNCTCAACTGGACCTGTCTCGTGGGCTCGGAG | |
| Morphoseq_index_E5 | TCGGTCTGCGCCCTCTAGCNNNNAATTCATCGTCTGTCTCGTGGGCTCGGAG | |
| Morphoseq_index_E6 | TCGGTCTGCGCCCTCTAGCNNNNTCGGACTAAGGTAGTCTCGTGGGCTCGGAG | |
| Morphoseq_index_E7 | TCGGTCTGCGCCCTCTAGCNNNNCGAAGCTCCTCCAGTCTCGTGGGCTCGGAG | |
| Morphoseq_index_E8 | TCGGTCTGCGCCCTCTAGCNNNNTGCCATAGATAGCGTCTCGTGGGCTCGGAG | |
| Morphoseq_index_E9 | TCGGTCTGCGCCCTCTAGCNNNNTAACTCTCGGTATGTCTCGTGGGCTCGGAG | |
| Morphoseq_index_E10 | TCGGTCTGCGCCCTCTAGCNNNNAATTCTGGATCTCGTCTCGTGGGCTCGGAG | |
| Morphoseq_index_E11 | TCGGTCTGCGCCCTCTAGCNNNNATTGAAGAGAGTCGTCTCGTGGGCTCGGAG | |
| Morphoseq_index_E12 | TCGGTCTGCGCCCTCTAGCNNNNTCATAGGTTCTGAGTCTCGTGGGCTCGGAG | |
| Morphoseq_index_F1 | TCGGTCTGCGCCCTCTAGCNNNNATCATAGTATTATGTCTCGTGGGCTCGGAG | |
| Morphoseq_index_F2 | TCGGTCTGCGCCCTCTAGCNNNNCGCTGGATTCGGTGTCTCGTGGGCTCGGAG | |
| Morphoseq_index_F3 | TCGGTCTGCGCCCTCTAGCNNNNTTAGCGGAATGGAGTCTCGTGGGCTCGGAG | |
| Morphoseq_index_F4 | TCGGTCTGCGCCCTCTAGCNNNNAAGAAGTCGTCTGGTCTCGTGGGCTCGGAG | |
| Morphoseq_index_F5 | TCGGTCTGCGCCCTCTAGCNNNNAAGAAGGAGTTACGTCTCGTGGGCTCGGAG | |
| Morphoseq_index_F6 | TCGGTCTGCGCCCTCTAGCNNNNCGCTCGTCAGGGTCTCGTGGGCTCGGAG | |
| Morphoseq_index_F7 | TCGGTCTGCGCCCTCTAGCNNNNACCGCGTTCTCTTGTCTCGTGGGCTCGGAG | |
| Morphoseq_index_F8 | TCGGTCTGCGCCCTCTAGCNNNNTCCAGAAGAAGAAGTCTCGTGGGCTCGGAG | |
| Morphoseq_index_F9 | TCGGTCTGCGCCCTCTAGCNNNNTCTTCGGTCCAACGTCTCGTGGGCTCGGAG | |
| Morphoseq_index_F10 | TCGGTCTGCGCCCTCTAGCNNNNATATGCCAATAACGTCTCGTGGGCTCGGAG | |
| Morphoseq_index_F11 | TCGGTCTGCGCCCTCTAGCNNNNTCTATCGTAAGTCGTCTCGTGGGCTCGGAG | |
| Morphoseq_index_F12 | TCGGTCTGCGCCCTCTAGCNNNNTGCTAAGGTCTTCGTCTCGTGGGCTCGGAG | |
| Morphoseq_index_G1 | TCGGTCTGCGCCCTCTAGCNNNNAGGACCAAGGCTCGTCTCGTGGGCTCGGAG | |
| Morphoseq_index_G2 | TCGGTCTGCGCCCTCTAGCNNNNTCAACGTCATGCTGTCTCGTGGGCTCGGAG | |
| Morphoseq_index_G3 | TCGGTCTGCGCCCTCTAGCNNNNTTCAAGGATCAAGGTCTCGTGGGCTCGGAG | |

| Primer | Sequence[a] | Protocol step[b,c] |
|---|---|---|
| Morphoseq_index_G4 | TCGGTCTGCGCCTCTAGCNNN**ACGGTACTGCTTA**GTCTCGTGGGCTCGGAG | |
| Morphoseq_index_G5 | TCGGTCTGCGCCTCTAGCNNN**TTCGAACCATCCG**GTCTCGTGGGCTCGGAG | |
| Morphoseq_index_G6 | TCGGTCTGCGCCTCTAGCNNN**TGGATGCATGAAC**GTCTCGTGGGCTCGGAG | |
| Morphoseq_index_G7 | TCGGTCTGCGCCTCTAGCNNN**CTCAGAAGGTACT**GTCTCGTGGGCTCGGAG | |
| Morphoseq_index_G8 | TCGGTCTGCGCCTCTAGCNNN**TGGACGGCCTTGC**GTCTCGTGGGCTCGGAG | |
| Morphoseq_index_G9 | TCGGTCTGCGCCTCTAGCNNN**AATCGTATAGCAA**GTCTCGTGGGCTCGGAG | |
| Morphoseq_index_G10 | TCGGTCTGCGCCTCTAGCNNN**TACGGCAAGCTA**TGTCTCGTGGGCTCGGAG | |
| Morphoseq_index_G11 | TCGGTCTGCGCCTCTAGCNNN**CAACCAAGGAAGC**GTCTCGTGGGCTCGGAG | |
| Morphoseq_index_G12 | TCGGTCTGCGCCTCTAGCNNN**TGCGAATAATGCG**GTCTCGTGGGCTCGGAG | |
| Morphoseq_index_H1 | TCGGTCTGCGCCTCTAGCNNN**ATCTCTTAAGAA**TGTCTCGTGGGCTCGGAG | |
| Morphoseq_index_H2 | TCGGTCTGCGCCTCTAGCNNN**AAGATATGATTAA**GTCTCGTGGGCTCGGAG | |
| Morphoseq_index_H3 | TCGGTCTGCGCCTCTAGCNNN**ATCTCAATAATAA**GTCTCGTGGGCTCGGAG | |
| Morphoseq_index_H4 | TCGGTCTGCGCCTCTAGCNNN**CTGCATCTATGGA**GTCTCGTGGGCTCGGAG | |
| Morphoseq_index_H5 | TCGGTCTGCGCCTCTAGCNNN**AGGAGTCTTAGCA**GTCTCGTGGGCTCGGAG | |
| Morphoseq_index_H6 | TCGGTCTGCGCCTCTAGCNNN**AATAGGACTCTGC**GTCTCGTGGGCTCGGAG | |
| Morphoseq_index_H7 | TCGGTCTGCGCCTCTAGCNNN**TCTTACGTTGCCG**GTCTCGTGGGCTCGGAG | |
| Morphoseq_index_H8 | TCGGTCTGCGCCTCTAGCNNN**TGGCATGAAGTA**TGTCTCGTGGGCTCGGAG | |
| Morphoseq_index_H9 | TCGGTCTGCGCCTCTAGCNNN**CAATATGCCAGG**TGTCTCGTGGGCTCGGAG | |
| Morphoseq_index_H10 | TCGGTCTGCGCCTCTAGCNNN**CATAAGGAGGTAA**GTCTCGTGGGCTCGGAG | |
| Morphoseq_index_H11 | TCGGTCTGCGCCTCTAGCNNN**ACGGTAAGCAAGC**GTCTCGTGGGCTCGGAG | |
| Morphoseq_index_H12 | TCGGTCTGCGCCTCTAGCNNN**AACTGCTTCGA**TCGTCTCGTGGGCTCGGAG | |
| Recovery | CAAGCAGAAGACGGCATACGAGATTCGGTCTGCGCCTCTAGC | Recovery |
| Enrichment | CAAGCAGAAGACGGCATACGA | Enrichment |
| Custom_i5_index_end | AATGATACGGCGACCACCGAGATCTACAC**AAGTTC**NNNNNNTCGTCGGCAGCGTC | End library preparation |

EP 3 950 958 B1

(continued)

| Primer | Sequence[a] | Protocol step[b,c] |
|---|---|---|
| Custom_i7_index_int | CAAGCAGAAGACGGCATACGAGAT**NNNNNN**TTAGGAGTCTCGTGGGCTCGG | Internal library preparation |
| Custom_i5_index_int | AATGATACGGCGACCACCGAGATCTACAC**TAACCG**NNNNNNTCGTCGGCAGCGTC | |
| Custom_i7_index_1 | CAAGCAGAAGACGGCATACGAGAT**NNNNNNCTACC**TGTCTCGTGGGCTCGG | Unmutated reference library preparation |
| Custom_i7_index_2 | CAAGCAGAAGACGGCATACGAGAT**NNNNNNTCTGAA**GTCTCGTGGGCTCGG | |
| Custom_i7_index_3 | CAAGCAGAAGACGGCATACGAGAT**NNNNNNAATACG**GTCTCGTGGGCTCGG | |
| Custom_i7_index_4 | CAAGCAGAAGACGGCATACGAGAT**NNNNNNATACTC**GTCTCGTGGGCTCGG | |
| Custom_i7_index_5 | CAAGCAGAAGACGGCATACGAGAT**NNNNNNAGGAGC**GTCTCGTGGGCTCGG | |
| Custom_i7_index_6 | CAAGCAGAAGACGGCATACGAGAT**NNNNNNAAGTTC**GTCTCGTGGGCTCGG | |
| Custom_i7_index_7 | CAAGCAGAAGACGGCATACGAGAT**NNNNNNTATAG**TGTCTCGTGGGCTCGG | |
| Custom_i7_index_8 | CAAGCAGAAGACGGCATACGAGAT**NNNNNNCGGAA**TGTCTCGTGGGCTCGG | |
| Custom_i7_index_9 | CAAGCAGAAGACGGCATACGAGAT**NNNNNNGGAACG**GTCTCGTGGGCTCGG | |
| Custom_i7_index_10 | CAAGCAGAAGACGGCATACGAGAT**NNNNNNGGC**TTGGTCTCGTGGGCTCGG | |
| Custom_i7_index_11 | CAAGCAGAAGACGGCATACGAGAT**NNNNNNAGGCC**TGTCTCGTGGGCTCGG | |
| Custom_i7_index_12 | CAAGCAGAAGACGGCATACGAGAT**NNNNNNCTTGCC**GTCTCGTGGGCTCGG | |
| Custom_i7_index_13 | CAAGCAGAAGACGGCATACGAGAT**NNNNNNTAGCGCG**TCTCGTGGGCTCGG | |
| Custom_i7_index_14 | CAAGCAGAAGACGGCATACGAGAT**NNNNNNGACCGG**GTCTCGTGGGCTCGG | |
| Custom_i7_index_15 | CAAGCAGAAGACGGCATACGAGAT**NNNNNNCCATGA**GTCTCGTGGGCTCGG | |
| Custom_i7_index_16 | CAAGCAGAAGACGGCATACGAGAT**NNNNNNTTGGAG**GTCTCGTGGGCTCGG | |
| Custom_i7_index_17 | CAAGCAGAAGACGGCATACGAGAT**NNNNNNGCCTGC**GTCTCGTGGGCTCGG | |
| Custom_i7_index_18 | CAAGCAGAAGACGGCATACGAGAT**NNNNNNGGCAAC**GTCTCGTGGGCTCGG | |
| Custom_i7_index_19 | CAAGCAGAAGACGGCATACGAGAT**NNNNNNTAACCG**GTCTCGTGGGCTCGG | |
| Custom_i7_index_20 | CAAGCAGAAGACGGCATACGAGAT**NNNNNNCGCGAG**GTCTCGTGGGCTCGG | |
| Custom_i7_index_21 | CAAGCAGAAGACGGCATACGAGAT**NNNNNNAACCA**TGTCTCGTGGGCTCGG | |
| Custom_i7_index_22 | CAAGCAGAAGACGGCATACGAGAT**NNNNNNTCATAC**GTCTCGTGGGCTCGG | |

| Primer | Sequence[a] | Protocol step[b,c] |
|---|---|---|
| Custom_i7_index_23 | CAAGCAGAAGACGGCATACGAGAT**NNNNNNACGG**TTGTCTCGTGGGCTCGG | |
| Custom_i7_index_24 | CAAGCAGAAGACGGCATACGAGAT**NNNNNNGGTTCT**GTCTCGTGGGCTCGG | |
| Custom_i5_index_1 | AATGATACGGCGACCACCGAGATCTACAC**TTAGGA**NNNNNNTCGTCGGCAGCGTC | |
| Custom_i5_index_2 | AATGATACGGCGACCACCGAGATCTACAC**AGGAGC**NNNNNNTCGTCGGCAGCGTC | |
| Custom_i5_index_3 | AATGATACGGCGACCACCGAGATCTACAC**AGGAGC**NNNNNNTCGTCGGCAGCGTC<br>AATGATACGGCGACCACCGAGATCTACAC**ACGGTT**NNNNNNTCGTCGGCAGCGTC | |
| Custom_i5_index_4 | AATGATACGGCGACCACCGAGATCTACAC**GCCTGC**NNNNNNTCGTCGGCAGCGTC | |
| Custom_i5_index_5 | AATGATACGGCGACCACCGAGATCTACAC**TAGCGC**NNNNNNTCGTCGGCAGCGTC | |
| Custom_i5_index_6 | AATGATACGGCGACCACCGAGATCTACAC**GGTTCT**NNNNNNTCGTCGGCAGCGTC | |
| Custom_i5_index_7 | AATGATACGGCGACCACCGAGATCTACAC**AGGCCT**NNNNNNTCGTCGGCAGCGTC | |
| Custom_i5_index_8 | AATGATACGGCGACCACCGAGATCTACAC**CTTGCC**NNNNNNTCGTCGGCAGCGTC | |
| Custom_i5_index_9 | AATGATACGGCGACCACCGAGATCTACAC**CTACCT**NNNNNNTCGTCGGCAGCGTC | |
| Custom_i5_index_10 | AATGATACGGCGACCACCGAGATCTACAC**TCATAC**NNNNNNTCGTCGGCAGCGTC | |
| Custom_i5_index_11 | AATGATACGGCGACCACCGAGATCTACAC**GTCGCG**NNNNNNTCGTCGGCAGCGTC | |
| Custom_i5_index_12 | AATGATACGGCGACCACCGAGATCTACAC**AACCAT**NNNNNNTCGTCGGCAGCGTC | |
| Custom_i5_index_13 | AATGATACGGCGACCACCGAGATCTACAC**CTGGTA**NNNNNNTCGTCGGCAGCGTC | |

EP 3 950 958 B1

| Primer | Sequence[a] | Protocol step[b,c] |
|---|---|---|
| Custom_i5_index_14 | AATGATACGGCGACCACCGAGATCTACAC**GACCGG**NNNNNNTCGTCGGCAGCGTC | |
| Custom_i5_index_15 | AATGATACGGCGACCACCGAGATCTACAC**CGGAAT**NNNNNNTCGTCGGCAGCGTC | |
| Custom_i5_index_16 | AATGATACGGCGACCACCGAGATCTACAC**TATAGT**NNNNNNTCGTCGGCAGCGTC | |
| Custom_i5_index_17 | AATGATACGGCGACCACCGAGATCTACAC**CAATAT**NNNNNNTCGTCGGCAGCGTC | |
| Custom_i5_index_18 | AATGATACGGCGACCACCGAGATCTACAC**GGCTTG**NNNNNNTCGTCGGCAGCGTC | |
| Custom_i5_index_19 | AATGATACGGCGACCACCGAGATCTACAC**AATACG**NNNNNNTCGTCGGCAGCGTC | |
| Custom_i5_index_20 | AATGATACGGCGACCACCGAGATCTACAC**CCATGA**NNNNNNTCGTCGGCAGCGTC | |
| Custom_i5_index_21 | AATGATACGGCGACCACCGAGATCTACAC**TCTGAA**NNNNNNTCGTCGGCAGCGTC | |
| Custom_i5_index_22 | AATGATACGGCGACCACCGAGATCTACAC**GGCAAC**NNNNNNTCGTCGGCAGCGTC | |
| Custom_i5_index_23 | AATGATACGGCGACCACCGAGATCTACAC**ATACTC**NNNNNNTCGTCGGCAGCGTC | |
| Custom_i5_index_24 | AATGATACGGCGACCACCGAGATCTACAC**TTGGAG**NNNNNNTCGTCGGCAGCGTC | |

Table S2: Primers used in this study.

a. Sample tag sequences are shown in bold.
b. A unique Morphoseq index primer was used for each sample during mutagenesis PCR
c. A unique combination of custom i7 index and custom i5 index primers was used for each unmutated reference library.

**Claims**

1. A method for determining a sequence of at least one target template nucleic acid molecule comprising:

(a) providing a pair of samples, each sample comprising at least one target template nucleic acid molecule;
(b) sequencing regions of the at least one target template nucleic acid molecule in a first of the pair of samples to provide non-mutated sequence reads;
(c) introducing mutations into the at least one target template nucleic acid molecule in a second of the pair of samples to provide at least one mutated target template nucleic acid molecule;
(d) sequencing regions of the at least one mutated target template nucleic acid molecule to provide mutated sequence reads;
(e) analysing the mutated sequence reads, and using information obtained from analysing the mutated sequence reads to assemble a sequence for at least a portion of at least one target template nucleic acid molecule from the non-mutated sequence reads,

wherein the first of the pair of samples is provided by pooling two or more sub-samples and/or the second of the pair of samples is provided by pooling two or more sub-samples, and wherein the method further comprises controlling the number of target template nucleic acid molecules in the first of the pair of samples and/or in the second of the pair of samples.

2. The method of claim 1, further comprising a step of normalising the number of target template nucleic acid molecules in each of the sub-samples that are pooled to provide the first of the pair of samples and/or the second of the pair of samples.

3. The method of any one preceding claim, wherein controlling or normalising the number of target template nucleic acid molecules comprises measuring the number of target template nucleic acid molecules in the at least one sample.

4. The method of claim 3, wherein measuring the number of target template nucleic acid molecules comprises preparing a dilution series of the at least one sample to provide a dilution series comprising diluted samples.

5. The method of any preceding claim, wherein controlling the number of target template nucleic acid molecules in a first of the pair of samples and/or the second of the pair of samples comprises measuring the number of target template nucleic acid molecules and diluting the first of the pair of samples and/or the second of the pair of samples such that the first of the pair of samples and/or the second of the pair of samples comprises a desired number of target template nucleic acid molecules.

6. The method of any preceding claim, wherein the first and/or the second of the pair of samples is provided by re-pooling the sub-samples such that the number of target template nucleic acid molecules in each of the sub-samples is in a desired ratio.

**Patentansprüche**

1. Verfahren zum Bestimmen einer Sequenz von mindestens einem Zielmatrizen-Nukleinsäuremolekül, Folgendes umfassend:

(a) Bereitstellen eines Probenpaares, wobei jede Probe mindestens ein Zielmatrizen-Nukleinsäuremolekül umfasst;
(b) Sequenzieren von Regionen des mindestens einen Zielmatrizen-Nukleinsäuremoleküls in einer ersten Probe des Probenpaares, um nicht-mutierte Sequenzreads bereitzustellen;

(c) Einführen von Mutationen in das mindestens eine Zielmatrizen-Nukleinsäuremolekül in einer zweiten Probe des Probenpaares, um mindestens ein mutiertes Zielmatrizen-Nukleinsäuremolekül bereitzustellen;

(d) Sequenzieren von Regionen des mindestens einen mutierten Zielmatrizen-Nukleinsäuremoleküls, um mutierte Sequenzreads bereitzustellen;

(e) Analysieren der mutierten Sequenzreads, und Verwenden von Informationen, die aus dem Analysieren der mutierten Sequenzreads erhalten wurden, um eine Sequenz für mindestens einen Abschnitt von mindestens einem Zielmatrizen-Nukleinsäuremolekül aus den nicht-mutierten Sequenzreads zusammenzubauen,

wobei die erste Probe des Probenpaares durch Poolen von zwei oder mehr Teilproben bereitgestellt ist und/oder die zweite Probe des Probenpaares durch Poolen von zwei oder mehr Teilproben bereitgestellt ist, und wobei das Verfahren ferner das Steuern der Anzahl von Zielmatrizen-Nukleinsäuremolekülen in der ersten Probe des Probenpaares und/oder in der zweiten Probe des Probenpaares umfasst.

2. Verfahren nach Anspruch 1, das ferner einen Schritt des Normalisierens der Anzahl von Zielmatrizen-Nukleinsäuremolekülen in jeder der Teilproben umfasst, die gepoolt werden, um die erste Probe des Probenpaares und/oder die zweite Probe des Probenpaares bereitzustellen.

3. Verfahren nach einem vorhergehenden Anspruch, wobei das Steuern oder Normalisieren der Anzahl von Zielmatrizen-Nukleinsäuremolekülen das Messen der Anzahl von Zielmatrizen-Nukleinsäuremolekülen in der mindestens einen Probe umfasst.

4. Verfahren nach Anspruch 3, wobei das Messen der Anzahl von Zielmatrizen-Nukleinsäuremolekülen das Herstellen einer Verdünnungsreihe der mindestens einen Probe umfasst, um eine Verdünnungsreihe, die verdünnte Proben umfasst, bereitzustellen

5. Verfahren nach einem vorhergehenden Anspruch, wobei das Steuern der Anzahl von Zielmatrizen-Nukleinsäuremolekülen in einer ersten Probe des Probenpaares und/oder der zweiten Probe des Probenpaares das Messen der Anzahl von Zielmatrizen-Nukleinsäuremolekülen und das Verdünnen der ersten Probe des Probenpaares und/oder der zweiten Probe des Probenpaares umfasst, sodass die erste Probe des Probenpaares und/oder die zweite Probe des Probenpaares eine gewünschte Anzahl von Zielmatrizen-Nukleinsäuremolekülen umfasst(umfassen).

6. Verfahren nach einem vorhergehenden Anspruch, wobei die erste Probe und/oder die zweite Probe des Probenpaares durch erneutes Poolen der Teilproben derart bereitgestellt wird(werden), dass die Anzahl von Zielmatrizen-Nukleinsäuremolekülen in jeder der Teilproben in einem gewünschten Verhältnis ist.

**Revendications**

1. Procédé de détermination d'une séquence d'au moins une molécule d'acide nucléique de matrice cible comprenant :

(a) la fourniture d'une paire d'échantillons, chaque échantillon comprenant au moins une molécule d'acide nucléique de matrice cible ;

(b) le séquençage de régions de l'au moins une molécule d'acide nucléique de matrice cible dans un premier échantillon de la paire d'échantillons pour fournir des lectures de séquence non mutée ;

(c) l'introduction de mutations dans l'au moins une molécule d'acide nucléique de matrice cible dans un second échantillon de la paire d'échantillons pour fournir au moins une molécule d'acide nucléique de matrice cible mutée ;

(d) le séquençage de régions de l'au moins une molécule d'acide nucléique de matrice cible mutée pour fournir des lectures de séquence mutée ;

(e) l'analyse des lectures de séquence mutée et l'utilisation d'informations obtenues à partir de l'analyse des lectures de séquence mutée pour assembler une séquence pour au moins une partie d'au moins une molécule d'acide nucléique de matrice cible à partir des lectures de séquence non mutée ;

dans lequel le premier échantillon de la paire d'échantillons est fourni en regroupant deux sous-échantillons ou plus et/ou le second échantillon de la paire d'échantillons est fourni en regroupant deux sous-échantillons ou plus, et dans lequel le procédé comprend en outre le contrôle du nombre de molécules d'acide nucléique de matrice cible dans le premier échantillon de la paire d'échantillons et/ou dans le second échantillon de la paire d'échantillons.

**2.** Procédé selon la revendication 1, comprenant en outre une étape de normalisation du nombre de molécules d'acide nucléique de matrice cible dans chacun des sous-échantillons qui sont regroupés pour fournir le premier échantillon de la paire d'échantillons et/ou le second échantillon de la paire d'échantillons.

**3.** Procédé selon une quelconque revendication précédente, dans lequel le contrôle ou la normalisation du nombre de molécules d'acide nucléique de matrice cible comprend la mesure du nombre de molécules d'acide nucléique de matrice cible dans l'au moins un échantillon.

**4.** Procédé selon la revendication 3, dans lequel la mesure du nombre de molécules d'acide nucléique de matrice cible comprend la préparation d'une série de dilutions de l'au moins un échantillon pour fournir une série de dilutions comprenant des échantillons dilués.

**5.** Procédé selon une quelconque revendication précédente, dans lequel le contrôle du nombre de molécules d'acide nucléique de matrice cible dans un premier échantillon de la paire d'échantillons et/ou le second échantillon de la paire d'échantillons comprend la mesure du nombre de molécules d'acide nucléique de matrice cible et la dilution du premier échantillon de la paire d'échantillons et/ou du second échantillon de la paire d'échantillons de telle sorte que le premier échantillon de la paire d'échantillons et/ou le second échantillon de la paire d'échantillons comprenne(nt) un nombre souhaité de molécules d'acide nucléique de matrice cible.

**6.** Procédé selon une quelconque revendication précédente, dans lequel le premier échantillon et/ou le second échantillon de la paire d'échantillons est(sont) fourni(s) en regroupant à nouveau les sous-échantillons de telle sorte que le nombre de molécules d'acide nucléique de matrice cible dans chacun des sous-échantillons soit dans un rapport souhaité.

Panel A:

percent identity of reads to reference

Panel B:

percent identity of reads to reference

Panel C:

FIGURE 2

FIGURE 3

SEQ ID NO: 1 – nucleotide sequence of DNA polymerase from Thermococcus sp. KS-1

```
atgatcctcg acactgacta cataactgag aatggaaaac ccgtcataag gattttcaag
aaggagaacg gcgagtttaa gattgagtac gataggactt ttgaacccta catttacgcc
ctcctgaagg acgattctgc cattgaggag gtcaagaaga taaccgccga gaggcacgga
acggttgtaa cggttaagcg ggctgaaaag gttcagaaga agttcctcgg gagaccagtt
gaggtctgga aactctactt tactcaccct caggacgtcc cagcgataag ggacaagata
cgagagcatc cagcagttat tgacatctac gagtacgaca tacccttcgc caagcgctac
ctcatagaca agggattagt gccaatggaa ggcgacgagg agctgaaaat gcttgccttt
gatatcgaga cgctctacca tgagggcgag gagttcgccg aggggccaat ccttatgata
agctacgccg acgaggaagg ggccagggtg ataacgtgga agaacgcgga tctgccctac
gttgacgtcg tctcgacgga gagggagatg ataaagcgct tcctaaaggt ggtcaaagag
aaagatcctg acgtcctaat aacctacaac ggcgacaact tcgacttcgc ctacctaaaa
aaacgctgtg aaaagcttgg aataaacttc acgctcggaa gggacggaag cgagccgaag
attcagagga tgggcgacag gtttgccgtc gaagtgaagg gacggataca cttcgatctc
tatcctgtga taagacggac gataaacctg cccacataca cgcttgaggc cgtttatgaa
gccgtcttcg gtcagccgaa ggagaaggtc tacgctgagg agatagctac agcttgggag
agcggtgaag gccttgagag agtagccaga tactcgatgg aagatgcgaa ggtcacatac
gagcttggga aggagttttt ccctatggag gcccagcttt ctcgcttaat cggccagtcc
ctctgggacg tctcccgctc cagcactggc aacctcgttg agtggttcct cctcaggaag
gcctacgaga ggaatgagct ggccccgaac aagcccgatg aaaaggagct ggccagaaga
cgacagagct atgaaggagg ctatgtaaaa gagcccgaga gagggttgtg ggagaacata
gtgtacctag attttagatc tctgtacccc tcaatcatca tcacccacaa cgtctcgccg
gatactctca cagggaagg atgcaaggaa tatgacgttg ccccccaggt cggtcaccgc
ttctgcaagg acttcccagg atttatcccg agcctgcttg gagacctcct agaggagagg
cagaagataa agaagaagat gaaggccacg attgacccga tcgagaggaa gctcctcgat
tacaggcaga gggccatcaa gatcctggcc aacagctact acggttacta cggctatgca
agggcgcgct ggtactgcaa ggagtgtgca gagagcgtaa cggcctgggg aagggagtac
ataacgatga ccatcagaga gatagaggaa aagtacggct ttaaggtaat ctacagcgac
accgacggat tttttgccac aatacctgga gccgatgctg aaaccgtcaa aaagaaggcg
atggagttcc tcaagtatat caacgccaaa ctcccggggcg cgcttgagct cgagtacgag
ggcttctaca acgcggctt cttcgtcacg aagaagaagt acgcggtgat agacgaggaa
ggcaagataa caacgcgcgg acttgagatt gtgaggcgcg actggagcga gatagcgaaa
gagacgcagg cgagggttct tgaagctttg ctaaaggacg gtgacgtcga aaggccgtg
aggatagtca aagaagttac cgaaaagctg agcaagtacg aggttccgcc ggagaagctg
gtgatccacg agcagataac gagggattta aaggactaca aggcaaccgg tccccacgtt
gccgttgcca agaggttggc cgcgagagga gtcaaaatac gccctggaac ggtgataagc
tacatcgtgc tcaagggctc tgggaggata ggcgacaggg cgataccgtt cgacgagttc
gacccgacga agcacaagta cgacgccgag tactacattg agaaccaggt tctcccagcc
gttgagagaa ttctgagagc cttcggttac cgcaaggaag acctgcgcta ccagaagacg
agacaggttg gtctgggagc ctggctgaag ccgaagggaa cttga
```

SEQ ID NO: 2 – polypeptide sequence of DNA polymerase from Thermococcus sp. KS-1

```
Met Ile Leu Asp Thr Asp Tyr Ile Thr Glu Asn Gly Lys Pro Val Ile
Arg Ile Phe Lys Lys Glu Asn Gly Glu Phe Lys Ile Glu Tyr Asp Arg
Thr Phe Glu Pro Tyr Ile Tyr Ala Leu Leu Lys Asp Asp Ser Ala Ile
Glu Glu Val Lys Lys Ile Thr Ala Glu Arg His Gly Thr Val Val Thr
Val Lys Arg Ala Glu Lys Val Gln Lys Lys Phe Leu Gly Arg Pro Val
Glu Val Trp Lys Leu Tyr Phe Thr His Pro Gln Asp Val Pro Ala Ile
Arg Asp Lys Ile Arg Glu His Pro Ala Val Ile Asp Ile Tyr Glu Tyr
Asp Ile Pro Phe Ala Lys Arg Tyr Leu Ile Asp Lys Gly Leu Val Pro
Met Glu Gly Asp Glu Glu Leu Lys Met Leu Ala Phe Asp Ile Glu Thr
Leu Tyr His Glu Gly Glu Glu Phe Ala Glu Gly Pro Ile Leu Met Ile
Ser Tyr Ala Asp Glu Glu Gly Ala Arg Val Ile Thr Trp Lys Asn Ala
Asp Leu Pro Tyr Val Asp Val Val Ser Thr Glu Arg Glu Met Ile Lys
Arg Phe Leu Lys Val Val Lys Glu Lys Asp Pro Asp Val Leu Ile Thr
Tyr Asn Gly Asp Asn Phe Asp Phe Ala Tyr Leu Lys Lys Arg Cys Glu
Lys Leu Gly Ile Asn Phe Thr Leu Gly Arg Asp Gly Ser Glu Pro Lys
Ile Gln Arg Met Gly Asp Arg Phe Ala Val Glu Val Lys Gly Arg Ile
His Phe Asp Leu Tyr Pro Val Ile Arg Arg Thr Ile Asn Leu Pro Thr
Tyr Thr Leu Glu Ala Val Tyr Glu Ala Val Phe Gly Gln Pro Lys Glu
Lys Val Tyr Ala Glu Glu Ile Ala Thr Ala Trp Glu Ser Gly Glu Gly
Leu Glu Arg Val Ala Arg Tyr Ser Met Glu Asp Ala Lys Val Thr Tyr
Glu Leu Gly Lys Glu Phe Phe Pro Met Glu Ala Gln Leu Ser Arg Leu
Ile Gly Gln Ser Leu Trp Asp Val Ser Arg Ser Ser Thr Gly Asn Leu
Val Glu Trp Phe Leu Leu Arg Lys Ala Tyr Glu Arg Asn Glu Leu Ala
Pro Asn Lys Pro Asp Glu Lys Glu Leu Ala Arg Arg Arg Gln Ser Tyr
Glu Gly Gly Tyr Val Lys Glu Pro Glu Arg Gly Leu Trp Glu Asn Ile
Val Tyr Leu Asp Phe Arg Ser Leu Tyr Pro Ser Ile Ile Ile Thr His
Asn Val Ser Pro Asp Thr Leu Asn Arg Glu Gly Cys Lys Glu Tyr Asp
Val Ala Pro Gln Val Gly His Arg Phe Cys Lys Asp Phe Pro Gly Phe
Ile Pro Ser Leu Leu Gly Asp Leu Leu Glu Glu Arg Gln Lys Ile Lys
Lys Lys Met Lys Ala Thr Ile Asp Pro Ile Glu Arg Lys Leu Leu Asp
Tyr Arg Gln Arg Ala Ile Lys Ile Leu Ala Asn Ser Tyr Tyr Gly Tyr
Tyr Gly Tyr Ala Arg Ala Arg Trp Tyr Cys Lys Glu Cys Ala Glu Ser
Val Thr Ala Trp Gly Arg Glu Tyr Ile Thr Met Thr Ile Arg Glu Ile
Glu Glu Lys Tyr Gly Phe Lys Val Ile Tyr Ser Asp Thr Asp Gly Phe
Phe Ala Thr Ile Pro Gly Ala Asp Ala Glu Thr Val Lys Lys Lys Ala
Met Glu Phe Leu Lys Tyr Ile Asn Ala Lys Leu Pro Gly Ala Leu Glu
Leu Glu Tyr Glu Gly Phe Tyr Lys Arg Gly Phe Phe Val Thr Lys Lys
Lys Tyr Ala Val Ile Asp Glu Glu Gly Lys Ile Thr Thr Arg Gly Leu
Glu Ile Val Arg Arg Asp Trp Ser Glu Ile Ala Lys Glu Thr Gln Ala
Arg Val Leu Glu Ala Leu Leu Lys Asp Gly Asp Val Glu Lys Ala Val
Arg Ile Val Lys Glu Val Thr Glu Lys Leu Ser Lys Tyr Glu Val Pro
Pro Glu Lys Leu Val Ile His Glu Gln Ile Thr Arg Asp Leu Lys Asp
Tyr Lys Ala Thr Gly Pro His Val Ala Val Ala Lys Arg Leu Ala Ala
Arg Gly Val Lys Ile Arg Pro Gly Thr Val Ile Ser Tyr Ile Val Leu
Lys Gly Ser Gly Arg Ile Gly Asp Arg Ala Ile Pro Phe Asp Glu Phe
Asp Pro Thr Lys His Lys Tyr Asp Ala Glu Tyr Tyr Ile Glu Asn Gln
Val Leu Pro Ala Val Glu Arg Ile Leu Arg Ala Phe Gly Tyr Arg Lys
Glu Asp Leu Arg Tyr Gln Lys Thr Arg Gln Val Gly Leu Gly Ala Trp
Leu Lys Pro Lys Gly Thr
```

SEQ ID NO: 3 – nucleic acid sequence of DNA polymerase from Thermococcus celer

```
atgatcctcg   acgctgacta   catcaccgaa   gatgggaagc   ccgtcgtgag   gatattcagg
aaggagaagg   gcgagttcag   aatcgactac   gacagggact   tcgagcccta   catctacgcc
ctcctgaagg   acgattcggc   catcgaggag   gtgaagagga   taaccgttga   gcgccacggg
aaggccgtca   gggttaagcg   ggtggagaag   gtcgaaaaga   agttcctcaa   caggccgata
gaggtctgga   agctctactt   caatcacccg   caggacgttc   cggcgataag   ggacgagata
aggaagcatc   cggccgtcgt   tgatatctac   gagtacgaca   tcccccttcgc   caagcgctac
ctcatcgata   aggggctcgt   cccgatggag   ggggaggagg   agctcaaact   gatggccttc
gacatcgaga   ccctctacca   cgagggagac   gagttcgggg   aggggccgat   cctgatgata
agctacgccg   acggggacgg   ggcgagggtc   ataacctgga   agaagatcga   cctcccctac
gtcgacgtcg   tctcgaccga   gaaggagatg   ataaagcgct   tcctccaggt   ggtgaaggag
aaggacccgg   acgtgctcgt   aacttacaac   ggcgacaact   tcgacttcgc   ctacctgaag
agacgctccg   aggagcttgg   attgaagttc   atcctcggga   gggacgggag   cgagcccaag
atccagcgca   tgggcgaccg   cttcgccgtc   gaggtgaagg   ggaggataca   cttcgacctc
tacccggtga   taaggcgcac   cgtgaacctg   ccgacctaca   cgctcgaggc   ggtctacgag
gccatcttcg   ggaggccaaa   ggagaaggtc   tacgccgggg   agatagtgga   ggcctgggaa
accggcgagg   gtcttgagag   ggttgcccgc   tactccatgg   aggacgcaaa   ggttaccttc
gagctcggga   gggagttctt   cccgatggag   gcccagctct   cgaggctcat   cggccagggt
ctctgggacg   tctcccgctc   gagcaccggc   aacctggtcg   agtggttcct   cctgaggaag
gcctacgaga   ggaacgaact   ggccccgaac   aagccgagcg   gccgggaagt   ggagatcagg
aggcgtggct   acgccggtgg   ttacgttaag   gagccggaga   ggggtttatg   ggagaacatc
gtgtacctcg   actttcgctc   tctttacccc   tccatcatca   taacccacaa   cgtctcgccc
gatacccctaa   acagggaggg   ctgtgagaac   tacgacgtcg   ccccccaggt   ggggcataag
ttctgcaaag   attttccggg   cttcatcccg   agcctgctcg   gaggcctgct   tgaggagagg
cagaagataa   agcggaggat   gaaggcctct   gtggatcccg   ttgagcggaa   gctcctcgat
tacaggcaga   gggccatcaa   gatactggcc   aacagcttct   acggatacta   cggctacgcg
agggcgaggt   ggtactgcag   ggagtgcgcg   gagagcgtta   ccgcctgggg   cagggagtac
atcgataggg   tcatcaggga   gctcgaggag   aagttcggct   tcaaggtgct   ctacgcggac
acggacggac   tgcacgccac   gatccccggg   gcggacgccg   ggaccgtcaa   ggagagggcg
aggggggttcc   tgagatacat   caaccccaag   ctccccggcc   tcctggagct   cgagtacgag
gggttctacc   tgaggggttt   cttcgtgacg   aagaagaagt   acgcggtcat   agacgaggag
ggcaagataa   ccacgcgcgg   cctcgagata   gtcaggcggg   actggagcga   ggtggccaag
gagacgcagg   cgagggtcct   ggaggcgata   ctgaggcacg   gtgacgtcga   ggaggccgtt
agaatcgtca   gggaggtaac   cgaaaagctg   agcaagtacg   aggttccgcc   ggagaaactg
gtgatccacg   agcagataac   gagggatttg   agggactaca   aagccacggg   accgcacgtg
gcggtggcga   agcgcctggc   cgggagggggg   gtaaggatac   gccccgggac   ggtgataagc
tacatcgtcc   tcaagggctc   cggaaggata   ggggacaggg   cgattccctt   cgacgagttc
gacccgacta   agcacaggta   cgacgccgac   tactacatcg   agaaccaggt   tctgccagcc
gtcgagagga   tcctgaaggc   cttcggctac   cgcaaggagg   acctgaaata   ccagaagacg
aggcaggtgg   gcctgggtgc   gtggctcaac   gcggggaagg   ggtga
```

SEQ ID NO: 4 – polypeptide sequence of DNA polymerase from Thermococcus celer

```
Met Ile Leu Asp Ala Asp Tyr Ile Thr Glu Asp Gly Lys Pro Val Val
Arg Ile Phe Arg Lys Glu Lys Gly Glu Phe Arg Ile Asp Tyr Asp Arg
Asp Phe Glu Pro Tyr Ile Tyr Ala Leu Leu Lys Asp Asp Ser Ala Ile
Glu Glu Val Lys Arg Ile Thr Val Glu Arg His Gly Lys Ala Val Arg
Val Lys Arg Val Glu Lys Val Glu Lys Lys Phe Leu Asn Arg Pro Ile
Glu Val Trp Lys Leu Tyr Phe Asn His Pro Gln Asp Val Pro Ala Ile
Arg Asp Glu Ile Arg Lys His Pro Ala Val Val Asp Ile Tyr Glu Tyr
Asp Ile Pro Phe Ala Lys Arg Tyr Leu Ile Asp Lys Gly Leu Val Pro
Met Glu Gly Glu Glu Glu Leu Lys Leu Met Ala Phe Asp Ile Glu Thr
Leu Tyr His Glu Gly Asp Glu Phe Gly Glu Gly Pro Ile Leu Met Ile
Ser Tyr Ala Asp Gly Asp Gly Ala Arg Val Ile Thr Trp Lys Lys Ile
Asp Leu Pro Tyr Val Asp Val Val Ser Thr Glu Lys Glu Met Ile Lys
Arg Phe Leu Gln Val Val Lys Glu Lys Asp Pro Asp Val Leu Val Thr
Tyr Asn Gly Asp Asn Phe Asp Phe Ala Tyr Leu Lys Arg Arg Ser Glu
Glu Leu Gly Leu Lys Phe Ile Leu Gly Arg Asp Gly Ser Glu Pro Lys
Ile Gln Arg Met Gly Asp Arg Phe Ala Val Glu Val Lys Gly Arg Ile
His Phe Asp Leu Tyr Pro Val Ile Arg Arg Thr Val Asn Leu Pro Thr
Tyr Thr Leu Glu Ala Val Tyr Glu Ala Ile Phe Gly Arg Pro Lys Glu
Lys Val Tyr Ala Gly Glu Ile Val Glu Ala Trp Glu Thr Gly Glu Gly
Leu Glu Arg Val Ala Arg Tyr Ser Met Glu Asp Ala Lys Val Thr Phe
Glu Leu Gly Arg Glu Phe Phe Pro Met Glu Ala Gln Leu Ser Arg Leu
Ile Gly Gln Gly Leu Trp Asp Val Ser Arg Ser Ser Thr Gly Asn Leu
Val Glu Trp Phe Leu Leu Arg Lys Ala Tyr Glu Arg Asn Glu Leu Ala
Pro Asn Lys Pro Ser Gly Arg Glu Val Glu Ile Arg Arg Arg Gly Tyr
Ala Gly Gly Tyr Val Lys Glu Pro Glu Arg Gly Leu Trp Glu Asn Ile
Val Tyr Leu Asp Phe Arg Ser Leu Tyr Pro Ser Ile Ile Ile Thr His
Asn Val Ser Pro Asp Thr Leu Asn Arg Glu Gly Cys Glu Asn Tyr Asp
Val Ala Pro Gln Val Gly His Lys Phe Cys Lys Asp Phe Pro Gly Phe
Ile Pro Ser Leu Leu Gly Gly Leu Leu Glu Glu Arg Gln Lys Ile Lys
Arg Arg Met Lys Ala Ser Val Asp Pro Val Glu Arg Lys Leu Leu Asp
Tyr Arg Gln Arg Ala Ile Lys Ile Leu Ala Asn Ser Phe Tyr Gly Tyr
Tyr Gly Tyr Ala Arg Ala Arg Trp Tyr Cys Arg Glu Cys Ala Glu Ser
Val Thr Ala Trp Gly Arg Glu Tyr Ile Asp Arg Val Ile Arg Glu Leu
Glu Glu Lys Phe Gly Phe Lys Val Leu Tyr Ala Asp Thr Asp Gly Leu
His Ala Thr Ile Pro Gly Ala Asp Ala Gly Thr Val Lys Glu Arg Ala
Arg Gly Phe Leu Arg Tyr Ile Asn Pro Lys Leu Pro Gly Leu Leu Glu
Leu Glu Tyr Glu Gly Phe Tyr Leu Arg Gly Phe Phe Val Thr Lys Lys
Lys Tyr Ala Val Ile Asp Glu Glu Gly Lys Ile Thr Thr Arg Gly Leu
Glu Ile Val Arg Arg Asp Trp Ser Glu Val Ala Lys Glu Thr Gln Ala
Arg Val Leu Glu Ala Ile Leu Arg His Gly Asp Val Glu Glu Ala Val
Arg Ile Val Arg Glu Val Thr Glu Lys Leu Ser Lys Tyr Glu Val Pro
Pro Glu Lys Leu Val Ile His Glu Gln Ile Thr Arg Asp Leu Arg Asp
Tyr Lys Ala Thr Gly Pro His Val Ala Val Ala Lys Arg Leu Ala Gly
Arg Gly Val Arg Ile Arg Pro Gly Thr Val Ile Ser Tyr Ile Val Leu
Lys Gly Ser Gly Arg Ile Gly Asp Arg Ala Ile Pro Phe Asp Glu Phe
Asp Pro Thr Lys His Arg Tyr Asp Ala Asp Tyr Tyr Ile Glu Asn Gln
Val Leu Pro Ala Val Glu Arg Ile Leu Lys Ala Phe Gly Tyr Arg Lys
Glu Asp Leu Lys Tyr Gln Lys Thr Arg Gln Val Gly Leu Gly Ala Trp
Leu Asn Ala Gly Lys Gly
```

SEQ ID NO: 5 – nucleic acid sequence of DNA polymerase from Thermococcus siculi

```
atgatcctcg  acacggacta  catcacggaa  gatgggaaac  ccgtcataag  gatattcaag
aaagagaacg  gcgagttcaa  gatcgagtac  gacaggactt  ttgaaccccta catctacgcc
ctcctgaagg  acgactccgc  gattgaggat  gttaaaaaga  taaccgccga  gaggcacgga
acggtggtga  aggtcaagcg  cgccgaaaag  gtgcagaaga  agttcctagg  caggccggtt
gaagtctgga  agctctactt  cacccacccc  caagatgtcc  cggcgataag  ggacaagatt
aggaagcatc  cagctgtaat  tgacatctac  gagtacgaca  taccattcgc  caagcgctac
ctcatcgaca  agggcctgat  tccgatggag  ggtgaagaag  agcttaagat  gctcgccttc
gacattgaga  cgctctacca  tgagggtgag  gagttcgccg  aggggcctat  tctgatgata
agctacgccg  acgagagcga  ggcacgcgtc  atcacctgga  agaaaatcga  cctcccctac
gttgacgtcg  tctcaacgga  gaaggagatg  ataaagcgct  tcctccgcgt  tgtgaaggag
aaagatcccg  atgtcctcat  aacctacaac  ggcgacaact  tcgacttcgc  ctacctgaag
aagcgctgtg  aaaagcttgg  aataaacttc  ctccttggaa  gggacgggag  cgagccgaag
atccagagaa  tgggtgaccg  cttcgccgtt  gaggtgaagg  ggaggataca  cttcgacctc
tatcctgtaa  taaggcgcac  gataaacctg  ccgacctaca  tgcttgaggc  agtctacgag
gccatctttg  ggaagccaaa  ggagaaggtt  tacgccgagg  agatagccac  cgcttgggaa
accggagagg  gccttgagag  ggtggctcgc  tactctatgg  aggacgcgaa  ggtcacgttt
gagcttggaa  aggagttctt  cccgatggag  gcccaacttt  cgaggttggt  cggccagagc
ttctgggatg  tcgcgcgctc  aagcacgggc  aatctggtcg  agtggttcct  cctcaggaag
gcctacgaga  ggaacgagct  ggctccaaac  aagccctctg  aagggaata  tgacgagagg
cgcggtggat  acgccggcgg  ctacgtcaag  gaaccggaaa  agggcctgtg  ggagaacata
gtctacctcg  actataaatc  tctctacccc  tcaatcatca  tcacccacaa  cgtctcgccc
gataccctca  accgcgaggg  ctgtaaggag  tatgacgtag  ctccacaggt  cggccaccgc
ttctgcaagg  actttccagg  cttcatcccg  agcctgctcg  gggatctcct  ggaggagagg
cagaagataa  agaggaagat  gaaggcaaca  attgacccga  tcgagagaaa  gctccttgat
tacaggcaac  gggccatcaa  gatccttcta  aatagttttt  acggctacta  cggctacgca
agggctcgct  ggtactgcaa  ggagtgtgcc  gagagcgtta  cggcatgggg  aagggaatat
atcaccatga  caatcaggga  aatagaagag  aagtatggct  ttaaagtact  ttatgcggac
actgacggct  tcttcgcgac  gattcccggg  gaagatgccg  agaccatcaa  aaagagggcg
atggagttcc  tcaagtacat  aaacgccaaa  ctccccggtg  cgctcgaact  tgagtacgag
gacttctaca  ggcgcggctt  cttcgtcacc  aagaagaaat  acgcggttat  cgacgaggag
ggcaagataa  caacgcgcgg  gctggagatc  gtcaggcgcg  actggagcga  gatagccaag
gagacgcagg  cgcgggttct  ggaggccctt  ctgaaggacg  gtgacgtcga  agaggccgtg
agcatagtca  aagaagtgac  cgagaagctg  agcaagtacg  aggttccgcc  ggagaagctc
gttatccacg  agcagataac  gcgcgagctg  aaggactaca  aggcaacggg  accacacgtg
gcgatagcga  agaggttagc  cgcgagaggc  gtcaaaatcc  gccccgggac  agtcatcagc
tacatcgtgc  tcaagggctc  cgggaggata  ggcgacaggg  cgattccctt  cgacgagttc
gaccccacga  agcacaagta  cgatgcagag  tactacatcg  agaaccaggt  tctacctgcc
gtcgagagga  ttctgaaggc  cttcggctat  cgcggtgagg  agctcagata  ccagaagacg
aggcaggttg  gacttggggc  gtggctgaag  ccgaaggga  aggggtga
```

SEQ ID NO: 6 – polypeptide sequence of DNA polymerase from Thermococcus siculi

```
Met Ile Leu Asp Thr Asp Tyr Ile Thr Glu Asp Gly Lys Pro Val Ile
Arg Ile Phe Lys Lys Glu Asn Gly Glu Phe Lys Ile Glu Tyr Asp Arg
Thr Phe Glu Pro Tyr Ile Tyr Ala Leu Leu Lys Asp Asp Ser Ala Ile
Glu Asp Val Lys Lys Ile Thr Ala Glu Arg His Gly Thr Val Val Lys
Val Lys Arg Ala Glu Lys Val Gln Lys Lys Phe Leu Gly Arg Pro Val
Glu Val Trp Lys Leu Tyr Phe Thr His Pro Gln Asp Val Pro Ala Ile
Arg Asp Lys Ile Arg Lys His Pro Ala Val Ile Asp Ile Tyr Glu Tyr
Asp Ile Pro Phe Ala Lys Arg Tyr Leu Ile Asp Lys Gly Leu Ile Pro
Met Glu Gly Glu Glu Glu Leu Lys Met Leu Ala Phe Asp Ile Glu Thr
Leu Tyr His Glu Gly Glu Glu Phe Ala Glu Gly Pro Ile Leu Met Ile
Ser Tyr Ala Asp Glu Ser Glu Ala Arg Val Ile Thr Trp Lys Lys Ile
Asp Leu Pro Tyr Val Asp Val Val Ser Thr Glu Lys Glu Met Ile Lys
Arg Phe Leu Arg Val Val Lys Glu Lys Asp Pro Asp Val Leu Ile Thr
Tyr Asn Gly Asp Asn Phe Asp Phe Ala Tyr Leu Lys Lys Arg Cys Glu
Lys Leu Gly Ile Asn Phe Leu Leu Gly Arg Asp Gly Ser Glu Pro Lys
Ile Gln Arg Met Gly Asp Arg Phe Ala Val Glu Val Lys Gly Arg Ile
His Phe Asp Leu Tyr Pro Val Ile Arg Arg Thr Ile Asn Leu Pro Thr
Tyr Met Leu Glu Ala Val Tyr Glu Ala Ile Phe Gly Lys Pro Lys Glu
Lys Val Tyr Ala Glu Glu Ile Ala Thr Ala Trp Glu Thr Gly Glu Gly
Leu Glu Arg Val Ala Arg Tyr Ser Met Glu Asp Ala Lys Val Thr Phe
Glu Leu Gly Lys Glu Phe Phe Pro Met Glu Ala Gln Leu Ser Arg Leu
Val Gly Gln Ser Phe Trp Asp Val Ala Arg Ser Ser Thr Gly Asn Leu
Val Glu Trp Phe Leu Leu Arg Lys Ala Tyr Glu Arg Asn Glu Leu Ala
Pro Asn Lys Pro Ser Gly Arg Glu Tyr Asp Glu Arg Arg Gly Gly Tyr
Ala Gly Gly Tyr Val Lys Glu Pro Glu Lys Gly Leu Trp Glu Asn Ile
Val Tyr Leu Asp Tyr Lys Ser Leu Tyr Pro Ser Ile Ile Ile Thr His
Asn Val Ser Pro Asp Thr Leu Asn Arg Glu Gly Cys Lys Glu Tyr Asp
Val Ala Pro Gln Val Gly His Arg Phe Cys Lys Asp Phe Pro Gly Phe
Ile Pro Ser Leu Leu Gly Asp Leu Leu Glu Glu Arg Gln Lys Ile Lys
Arg Lys Met Lys Ala Thr Ile Asp Pro Ile Glu Arg Lys Leu Leu Asp
Tyr Arg Gln Arg Ala Ile Lys Ile Leu Leu Asn Ser Phe Tyr Gly Tyr
Tyr Gly Tyr Ala Arg Ala Arg Trp Tyr Cys Lys Glu Cys Ala Glu Ser
Val Thr Ala Trp Gly Arg Glu Tyr Ile Thr Met Thr Ile Arg Glu Ile
Glu Glu Lys Tyr Gly Phe Lys Val Leu Tyr Ala Asp Thr Asp Gly Phe
Phe Ala Thr Ile Pro Gly Glu Asp Ala Glu Thr Ile Lys Lys Arg Ala
Met Glu Phe Leu Lys Tyr Ile Asn Ala Lys Leu Pro Gly Ala Leu Glu
Leu Glu Tyr Glu Asp Phe Tyr Arg Arg Gly Phe Phe Val Thr Lys Lys
Lys Tyr Ala Val Ile Asp Glu Glu Gly Lys Ile Thr Thr Arg Gly Leu
Glu Ile Val Arg Arg Asp Trp Ser Glu Ile Ala Lys Glu Thr Gln Ala
Arg Val Leu Glu Ala Leu Leu Lys Asp Gly Asp Val Glu Glu Ala Val
Ser Ile Val Lys Glu Val Thr Glu Lys Leu Ser Lys Tyr Glu Val Pro
Pro Glu Lys Leu Val Ile His Glu Gln Ile Thr Arg Glu Leu Lys Asp
Tyr Lys Ala Thr Gly Pro His Val Ala Ile Ala Lys Arg Leu Ala Ala
Arg Gly Val Lys Ile Arg Pro Gly Thr Val Ile Ser Tyr Ile Val Leu
Lys Gly Ser Gly Arg Ile Gly Asp Arg Ala Ile Pro Phe Asp Glu Phe
Asp Pro Thr Lys His Lys Tyr Asp Ala Glu Tyr Tyr Ile Glu Asn Gln
Val Leu Pro Ala Val Glu Arg Ile Leu Lys Ala Phe Gly Tyr Arg Gly
Glu Glu Leu Arg Tyr Gln Lys Thr Arg Gln Val Gly Leu Gly Ala Trp
Leu Lys Pro Lys Gly Lys Gly
```

SEQ ID NO: 7 - polypeptide sequence of DNA polymerase from Thermococcus kodakarensis

```
Met Ile Leu Asp Thr Asp Tyr Ile Thr Glu Asp Gly Lys Pro Val Ile
Arg Ile Phe Lys Lys Glu Asn Gly Glu Phe Lys Ile Glu Tyr Asp Arg
Thr Phe Glu Pro Tyr Phe Tyr Ala Leu Leu Lys Asp Asp Ser Ala Ile
Glu Glu Val Lys Lys Ile Thr Ala Glu Arg His Gly Thr Val Val Thr
Val Lys Arg Val Glu Lys Val Gln Lys Lys Phe Leu Gly Arg Pro Val
Glu Val Trp Lys Leu Tyr Phe Thr His Pro Gln Asp Val Pro Ala Ile
Arg Asp Lys Ile Arg Glu His Pro Ala Val Ile Asp Ile Tyr Glu Tyr
Asp Ile Pro Phe Ala Lys Arg Tyr Leu Ile Asp Lys Gly Leu Val Pro
Met Glu Gly Asp Glu Glu Leu Lys Met Leu Ala Phe Asp Ile Glu Thr
Leu Tyr Glu Glu Gly Glu Glu Phe Ala Glu Gly Pro Ile Leu Met Ile
Ser Tyr Ala Asp Glu Glu Gly Ala Arg Val Ile Thr Trp Lys Asn Val
Asp Leu Pro Tyr Val Asp Val Val Ser Thr Glu Arg Glu Met Ile Lys
Arg Phe Leu Arg Val Val Lys Glu Lys Asp Pro Asp Val Leu Ile Thr
Tyr Asn Gly Asp Asn Phe Asp Phe Ala Tyr Leu Lys Lys Arg Cys Glu
Lys Leu Gly Ile Asn Phe Ala Leu Gly Arg Asp Gly Ser Glu Pro Lys
Ile Gln Arg Met Gly Asp Arg Phe Ala Val Glu Val Lys Gly Arg Ile
His Phe Asp Leu Tyr Pro Val Ile Arg Arg Thr Ile Asn Leu Pro Thr
Tyr Thr Leu Glu Ala Val Tyr Glu Ala Val Phe Gly Gln Pro Lys Glu
Lys Val Tyr Ala Glu Glu Ile Thr Thr Ala Trp Glu Thr Gly Glu Asn
Leu Glu Arg Val Ala Arg Tyr Ser Met Glu Asp Ala Lys Val Thr Tyr
Glu Leu Gly Lys Glu Phe Leu Pro Met Glu Ala Gln Leu Ser Arg Leu
Ile Gly Gln Ser Leu Trp Asp Val Ser Arg Ser Ser Thr Gly Asn Leu
Val Glu Trp Phe Leu Leu Arg Lys Ala Tyr Glu Arg Asn Glu Leu Ala
Pro Asn Lys Pro Asp Glu Lys Glu Leu Ala Arg Arg Arg Gln Ser Tyr
Glu Gly Gly Tyr Val Lys Glu Pro Glu Arg Gly Leu Trp Glu Asn Ile
Val Tyr Leu Asp Phe Arg Ser Leu Tyr Pro Ser Ile Ile Ile Thr His
Asn Val Ser Pro Asp Thr Leu Asn Arg Glu Gly Cys Lys Glu Tyr Asp
Val Ala Pro Gln Val Gly His Arg Phe Cys Lys Asp Phe Pro Gly Phe
Ile Pro Ser Leu Leu Gly Asp Leu Leu Glu Glu Arg Gln Lys Ile Lys
Lys Lys Met Lys Ala Thr Ile Asp Pro Ile Glu Arg Lys Leu Leu Asp
Tyr Arg Gln Arg Ala Ile Lys Ile Leu Ala Asn Ser Tyr Tyr Gly Tyr
Tyr Gly Tyr Ala Arg Ala Arg Trp Tyr Cys Lys Glu Cys Ala Glu Ser
Val Thr Ala Trp Gly Arg Glu Tyr Ile Thr Met Thr Ile Lys Glu Ile
Glu Glu Lys Tyr Gly Phe Lys Val Ile Tyr Ser Asp Thr Asp Gly Phe
Phe Ala Thr Ile Pro Gly Ala Asp Ala Glu Thr Val Lys Lys Lys Ala
Met Glu Phe Leu Lys Tyr Ile Asn Ala Lys Leu Pro Gly Ala Leu Glu
Leu Glu Tyr Glu Gly Phe Tyr Glu Arg Gly Phe Phe Val Thr Lys Lys
Lys Tyr Ala Val Ile Asp Glu Glu Gly Lys Ile Thr Thr Arg Gly Leu
Glu Ile Val Arg Arg Asp Trp Ser Glu Ile Ala Lys Glu Thr Gln Ala
Arg Val Leu Glu Ala Leu Leu Lys Asp Gly Asp Val Glu Lys Ala Val
Arg Ile Val Lys Glu Val Thr Glu Lys Leu Ser Lys Tyr Glu Val Pro
Pro Glu Lys Leu Val Ile His Glu Gln Ile Thr Arg Asp Leu Lys Asp
Tyr Lys Ala Thr Gly Pro His Val Ala Val Ala Lys Arg Leu Ala Ala
Arg Gly Val Lys Ile Arg Pro Gly Thr Val Ile Ser Tyr Ile Val Leu
Lys Gly Ser Gly Arg Ile Gly Asp Arg Ala Ile Pro Phe Asp Glu Phe
Asp Pro Thr Lys His Lys Tyr Asp Ala Glu Tyr Tyr Ile Glu Asn Gln
Val Leu Pro Ala Val Glu Arg Ile Leu Arg Ala Phe Gly Tyr Arg Lys
Glu Asp Leu Arg Tyr Gln Lys Thr Arg Gln Val Gly Leu Ser Ala Trp
Leu Lys Pro Lys Gly Thr
```

SAMPLE TAG SEQUENCES

| SEQ ID Number | Sample tag sequence |
|---|---|
| 8 | TAGAATTGAAGAA |
| 9 | TGGCCATAGCTAC |
| 10 | GTCATCTGCGACC |
| 11 | TTCGCGCTTGGAC |
| 12 | CGCGAACCGTTAG |
| 13 | TTGCAGCCTCTAA |
| 14 | TCTACTAGTACGA |
| 15 | GTAGGTTCTACTG |
| 16 | GCCAATATCAAGT |
| 17 | CTATCTTGCTGGT |
| 18 | GTTCTCATAGGTA |
| 19 | GTCTATGAACCAA |
| 20 | CGGAGCGCTTATT |
| 21 | TATGCCATGAGGA |
| 22 | ATACGACTCGGAG |
| 23 | GATGGAACTCAGC |
| 24 | GGACCTGCATGAA |
| 25 | TAGACTGGAACTT |
| 26 | GAATTACCTCGTT |
| 27 | AGGATCAGGCTAC |
| 28 | ACGCGTAGAAGAG |
| 29 | CTTCGAGACTTAC |
| 30 | GACGGCTAACTCC |
| 31 | TTAGCATTCTCTT |

| 32 | GCAAGGCATAGTA |
|----|---------------|
| 33 | ACCTAGATATGGA |
| 34 | ACGCCAAGGCGTA |
| 35 | TATGACGGATCCG |
| 36 | CCTCCATTAGAGA |
| 37 | ATTGAATACTCTG |
| 38 | GAGATGAGAAGAA |
| 39 | TCTGAGTAGCCGG |
| 40 | AATAGGTAGTACG |
| 41 | GTCGAAGAAGTCC |
| 42 | TACTGCATCTCGT |
| 43 | GACGTATTAGAGC |
| 44 | CCTGCATTATTCG |
| 45 | ACGAATGATGCTC |
| 46 | TACTAGCAGAGAT |
| 47 | CTCCTCATCTTCC |
| 48 | TCCTCTGCGCTGC |
| 49 | CCTTCTCAGTCCG |
| 50 | CAGCTTCATAGCG |
| 51 | TTGACTCTCGCGC |
| 52 | TATCCTGAGCGAT |
| 53 | AACGCCTAGCCGA |
| 54 | CCGAAGACGTCAT |
| 55 | GAGTTCTCCAGAT |
| 56 | TGCATCCGCGCTT |
| 57 | CCTGAACTCAAGT |
| 58 | GGTCGTATGCGTA |

| 59 | AGGCCTCTCTACC |
| 60 | GTACTCCATCCAA |
| 61 | CAGCGGACGCGCT |
| 62 | ATCTCTCTTAGCA |
| 63 | AAGCAATAATAAT |
| 64 | AAGGCGACTCCGA |
| 65 | ACGTCTCTAGGAG |
| 66 | CCATCAGACCTCT |
| 67 | ACTTAATCGTACT |
| 68 | TGGAATTCTCCAA |
| 69 | CCATACGATCAGG |
| 70 | TTATGGAGCAATA |
| 71 | GCTCGGCGTTCGA |
| 72 | TTGGCCAGTCGCT |
| 73 | CAGATACGTAGAG |
| 74 | AATGCTATTATCC |
| 75 | GCAGCATGCCGAT |
| 76 | GGAGAGTTACCTC |
| 77 | GAGAGTCCATGAT |
| 78 | CAATCTATTCTGA |
| 79 | GCTCTTAGTATCC |
| 80 | CCATAGTTATGGT |
| 81 | TGCGAGATCGAAG |
| 82 | AGAGAAGTCGAGT |
| 83 | GGTAACTCCATAT |
| 84 | TGCTATTCCAGGC |
| 85 | AACCGCGAGGCTC |

| 86 | TTCTAGAGATACC |
| 87 | TTCGCTCAAGTAT |
| 88 | CAGAGAAGGCGCA |
| 89 | TAGAATTGGCCTC |
| 90 | GGCCATTCTCCAG |
| 91 | TCCAACGCGCGTT |
| 92 | GCCGCAGATTACG |
| 93 | GCAGTTCGAACGC |
| 94 | TTCTCTCTGCAGG |
| 95 | TAAGCTACCAGCG |
| 96 | CTGCATGAGGTTG |
| 97 | TTGCCTAGCGAGG |
| 98 | CAACTGAATTAGG |
| 99 | AAGCGGTCCTCTT |
| 100 | AATGGAAGGACCG |
| 101 | GAGTTAGTAAGTT |
| 102 | TTCCTAATTCCAA |
| 103 | GTTCTGGTTCGCT |
| 104 | GTTCATCTCTTCC |
| 105 | ATTCCGAGGAAGA |
| 106 | CTTAGCCGAGAGA |
| 107 | GTCTGCTACGCTT |
| 108 | ATGGCGCCGCGCA |
| 109 | TAATGGTTATCT |
| 110 | TCGGTTATAAGTC |
| 111 | TGCCTGAGAACGT |
| 112 | AGATGCGGTTAAC |

| 113 | ATGGAATAGGCGA |
| 114 | AGAGATGCGATCG |
| 115 | CTCCAACTAACGT |
| 116 | GCCTTGCTACTGG |
| 117 | CTTCGTCTCTACG |
| 118 | ACGCTCATAGCCT |
| 119 | GTCGAAGATAAGG |
| 120 | GCCGGAGTCCTCG |
| 121 | TATACGGCGACCT |
| 122 | AGGTAGATATTCG |
| 123 | TTAAGGTACTGCT |
| 124 | CGGATCTGGTATA |
| 125 | GAGGTCTCGGAGG |
| 126 | GGCATCGATGGAC |
| 127 | GATCTCCGATATA |
| 128 | GATTCGGAATACT |
| 129 | CTGCGATCCGGCC |
| 130 | GATCCGGTTGCAA |
| 131 | CGTCAGGCTTGAC |
| 132 | TCGGCAAGGCGAG |
| 133 | GAACGGCGAACGC |
| 134 | CCTCAAGCGGACT |
| 135 | GAAGCCAGATGGT |
| 136 | TGCTCATACCAAT |

FIGURE 4

## morphoseq 1-2kbp test

reconstructed fragment length in nucleotides

FIGURE 5

A

B

Figure 6

## Figure 7

Replicates of at least one target
template nucleic acid molecule

Sample 1

At least one
target template
nucleic acid
molecule

Sequence
(S1)

Non-mutated
sequence reads

Prepare assembly graph
comprising nodes computed from
non-mutated sequence reads

Sample 2

At least one
target template
nucleic acid
molecule

Mutate
(S2)

A   B   C   F   ·
E   G   F   I
I   J   K   L

Sequence
(S3)

A B      B C D
    C D          E F G
G H      I J K      L M
E F      I J      J K

Mutated
sequence
reads

Map mutated reads to
the assembly graph (S5)

Optional pre-clustering
step (S5A)

Identify routes through
the assembly graph (S6)

Assemble a
sequence (S7)

## Figure 8

Prepare assembly graph comprising nodes computed from non-mutated sequence reads (S4)

Optionally map ends of the at least one target template nucleic acid molecule to the assembly graph (S4A)

Map the mutated sequence reads to the assembly graph (S5)

Optionally pre-cluster the mutated sequence reads (S5A)

Identify mutated sequence reads that are likely to have originated from the same at least one mutated target template nucleic acid molecule (S5B)

Identify nodes that are linked by mutated sequence reads that are likely to have originated from the same at least one mutated target template nucleic acid molecule (S6)

Assemble portions of at least one target template nucleic acid molecule by concatenating the identified nodes (S7)

## Figure 9

A

Putative route Ⓒ

Node

Node

Node

Putative route Ⓓ

Putative route Ⓐ

Putative route Ⓑ

B

Putative route

Ⓓ

Mutated reads indicate route
Ⓓ is a true route

A

Figure 10

1 Dual primer binding sites          2 Single primer binding site

Figure 11

Figure 12

A

B

Figure 13

Figure 14

Figure 15

C03: *Arcobacter butzleri*, Strain JV22

| | shovill | 20190304 |
|---|---|---|

Bandage

| | | |
|---|---|---|
| num_contigs | 78 | 1 |
| num_scaffolds | 78 | 1 |
| total_size | 2271342 | 2297844 |
| max_scaffold | 342386 | 2297844 |
| max_scaffold_%_of_total | 15.0741720093231 | 100 |
| scaffold_n50 | 126670 | 2297844 |
| scaffold_n90 | 26514 | 2297844 |
| avg_contigs_per_scaffold | 1 | 1 |

Figure 16

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 02079502 A
- WO 2014013218 A
- WO 2016057947 A

**Non-patent literature cited in the description**

- **MAXAM AM ; GILBERT W.** A new method for sequencing DNA. *Proc. Natl. Acad. Sci. U. S. A.,* February 1977, vol. 74 (2), 560-4
- **SANGER F ; COULSON AR.** A rapid method for determining sequences in DNA by primed synthesis with DNA polymerase. *J. Mol. Biol.,* May 1975, vol. 94 (3), 441-8
- **BENTLEY DR ; BALASUBRAMANIAN S et al.** Accurate whole human genome sequencing using reversible terminator chemistry. *Nature,* 2008, vol. 456 (7218), 53-59